(19) Europäisches Patentamt / European Patent Office / Office européen des brevets

(11) EP 4 778 980 A1

(12) EUROPEAN PATENT APPLICATION
published in accordance with Art. 153(4) EPC

(43) Date of publication:
22.07.2026 Bulletin 2026/30

(21) Application number: 24864754.7

(22) Date of filing: 13.09.2024

(51) International Patent Classification (IPC):
C08L 83/07 (2006.01) C08L 83/05 (2006.01)
A61L 31/06 (2006.01) A61L 31/16 (2006.01)
A61K 47/34 (2017.01) A61K 9/00 (2006.01)
A61K 31/00 (2006.01) A61J 3/00 (2006.01)

(52) Cooperative Patent Classification (CPC):
A61J 3/00; A61K 9/00; A61K 31/00; A61K 47/34; A61L 31/06; A61L 31/16; C08L 83/04

(86) International application number:
PCT/CN2024/118882

(87) International publication number:
WO 2025/056039 (20.03.2025 Gazette 2025/12)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 13.09.2023 CN 202311180556
13.09.2023 CN 202311180557

(71) Applicant: SHENYANG XINGHUA PHARMACEUTICAL TECHNOLOGY CO., LTD
Shenyang, Liaoning 110000 (CN)

(72) Inventors:
- YANG, Xinggang
Shenyang, Liaoning 110000 (CN)
- LIU, Baolin
Shenyang, Liaoning 110000 (CN)
- SHENG, Huali
Shenyang, Liaoning 110000 (CN)
- WANG, Tianyuan
Shenyang, Liaoning 110000 (CN)
- SANG, Guantong
Shenyang, Liaoning 110000 (CN)
- WANG, Tianyu
Shenyang, Liaoning 110000 (CN)
- DIAO, Rui
Shenyang, Liaoning 110000 (CN)

(74) Representative: Michalski Hüttermann & Partner mbB
Kaistraße 16A
40221 Düsseldorf (DE)

# (54) SILICONE MATERIAL, SILICONE TUBE, IMPLANT AND PREPARATION METHOD THEREFOR, ROD CORE, PERFORATED IMPLANT AND PREPARATION METHOD THEREFOR AND USE THEREOF

(57) Disclosed in the present invention are a silicone material, a silicone tube, an implant and a preparation method therefor, a rod core, a perforated implant and a preparation method therefor and the use thereof. The silicone material comprises the following components: 100 parts of R-vinyl polysiloxane, 10-80 parts of a reinforcing agent and 0.000002-1 part of a catalyst. The rod core comprises a drug-containing layer, wherein the drug-containing layer contains an active drug and an auxiliary material; the auxiliary material comprises a sustained- and controlled-release auxiliary material; the sustained- and controlled-release auxiliary material has a molecular weight of 1000 to 10 million; and the drug-auxiliary material ratio is 1:(0.01-100). With regard to the silicone material, while meeting mechanical properties, the permeability of water to the silicone tube is increased, so that the release amount of drugs is increased and the defect of low in-vitro release in the prior art is ameliorated. The implant can be suitable for most drugs and can achieve long-acting release of a drug.

EP 4 778 980 A1

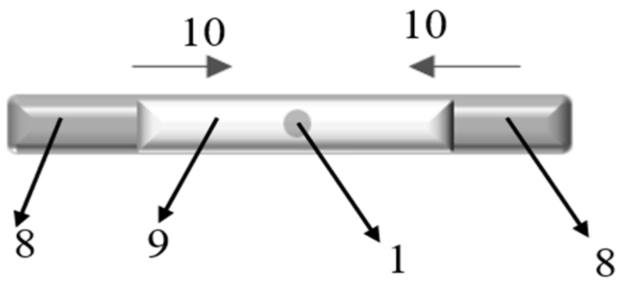


FIG. 9

## Description

The present application claims the priority of Chinese patent applications 2023111805573 and 2023111805569, filed on 2023/9/13. The contents of the Chinese patent applications are incorporated herein by reference in their entireties.

TECHNICAL FIELD

The present disclosure relates to silicone material, silicone tube and implant and preparation method therefor, rod core, perforated implant and preparation method therefor and use thereof.

BACKGROUND

Silicone subdermal implants involve filling a silicone tube with medication and implanting it under the skin. The medication solution is formed by water seeping into the silicone tube and is then released stably through the silicone tube to achieve a long-term effect. Currently available on the market is levonorgestrel implant, which can provide long-term contraception. This formulation avoids the first-pass effect in the gastrointestinal tract, thereby greatly improving the bioavailability of the drug.

In the prior art, the silicone subcutaneous implant mainly adopts methyl vinyl polysiloxane and hydrophobic silica as base materials. Hydrophobic silica exhibits better reinforcing properties, and the silicone tubes prepared from it can better meet the mechanical requirements for implantation. However, as a hydrophobic substance, its low water permeability prevents water from effectively penetrating the silicone tube to dissolve the drug into a solution. This results in a low drug release from the implant, which cannot meet the needs of drugs that require large doses to take effect, thus limiting its clinical application.

Subcutaneous implants mainly involve placing active drugs in a carrier and embedding them under the skin. The implant controls the release of the drug through the carrier, and the drug is absorbed into the bloodstream through local capillaries, achieving stable release for several years and a long-term effect. This formulation avoids the first-pass effect in the gastrointestinal tract, thereby greatly improving the bioavailability of the drug. Since it needs to be implanted in the body, the choice of material for its carrier is particularly important.

Implants typically consist of a drug core and a silicone tube. The drug cores of traditional implants are generally divided into two types: one is rod core filled with powder drugs. There are some problems in powder drug filling, such as large drug consumption, difficulty in controlling drug filling uniformity, and obvious drug release burst. Another type is the silicone matrix rod core, which requires high-temperature sulfidation and shaping during its preparation, obviously, this type of rod core is not suitable for heat-sensitive drugs that are prone to high-temperature degradation.

The silicone tube of the traditional silicone implant is not perforated, and the silicone tube forms pores through self-crosslinking network structure, and external moisture enters the silicone tube through these pores, dissolving drugs into a solution, and the drug solution first diffuses to the silicone tube wall and is adsorbed with the silicone tube wall. Since the polarity of silicone is very low, only drugs with very low polarity can complete this adsorption process and then diffuse, while drugs with high polarity cannot diffuse to the outside of the silicone tube. Secondly, due to the inherent properties of silicone and the size of the mesh-like channels of the silicone tube, only small molecule drugs with low polarity and molecular weight below 800 and water molecules can pass through, while drugs with molecular weight greater than 800 are significantly retained. Therefore, it is not suitable for large molecule drugs.

Therefore, in order to solve the above problems, it is urgent to develop an implant that is suitable for various types of drugs.

CONTENT OF THE PRESENT INVENTION

The technical problem to be solved in the present disclosure is for overcoming the shortcomings of silicone materials of the prior art that mechanical properties and water permeability are difficult to achieve simultaneously, and provides silicone materials, silicone tube and implant and preparation method therefor, rod core, perforated implant and preparation method therefor and use thereof. The silicone material provided by the disclosure not only satisfies mechanical properties, but also increases the permeability of water to the silicone tube, thereby increasing the release of drugs and overcoming the deficiency of low in vitro release in the prior art.

The present disclosure solves the above-mentioned technical problems through the following technical solutions.

The present disclosure provides a raw material composition of silicone material, comprising the following components in parts by weight:

R-vinyl polysiloxane: 100 parts;

reinforcing agent: 10-80 parts;

catalyst: 0.000002-1 part;

wherein, R in R-vinyl polysiloxane is a substituted or unsubstituted $C_1$-$C_5$ straight-chain alkane or branched-chain alkane, a substituted or unsubstituted $C_6$-$C_{20}$ aromatic hydrocarbon;

the vinyl content in the R-vinyl polysiloxane is 0.1 mol%-0.5 mol%;

the components in the reinforcing agent contains hydrophilic groups;

the Si-H group content in the hydrogen-containing silicone oil is 0.18 mol% -1.6 mol%;

the molar ratio of Si-H groups in the hydrogen-containing silicone oil to the vinyl groups in R-vinyl polysiloxane is (0.5-10):1;

optionally, further comprising an inhibitor, which is a substance that can inhibit the addition reaction between the R-vinyl polysiloxane and the hydrogen-containing silicone oil.

In the present disclosure, R in R-vinyl polysiloxane is a substituted or unsubstituted $C_1$-$C_5$ straight-chain alkane, for example, methyl.

When R is methyl, the R-vinyl polysiloxane is methyl vinyl polysiloxane.

In the present disclosure, the structural formula of the R-vinyl polysiloxane can be as follows:

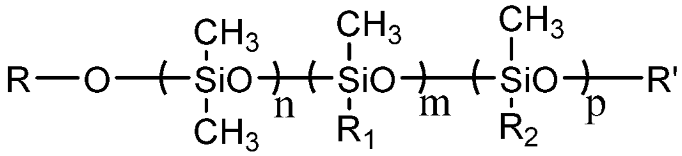

wherein, m, n, and p are natural numbers, $R_1$ and $R_2$ can be conventional substituents in the art, and R and R' can be conventional end-capping groups in the art.

For example, when $R_1$=$R_2$=$CH_3$, it corresponds to dimethylpolysiloxane;

for example, when $R_1$=$CH_3$, $R_2$=$CH$=$CH_2$, it corresponds to methyl vinyl polysiloxane;

for example, when $R_1$=Ph, $R_2$=$CH$=$CH_2$, it corresponds to methylphenylvinyl polysiloxane;

for example, when $R_1$=$CH_3CH_2CF_3$, $R_2$=$CH$=$CH_2$, it corresponds to fluorosilicone polysiloxane;

for example, when $R_1$=$CH_3CH_2CN$, $R_2$=$CH$=$CH_2$, it corresponds to nitrile polysiloxane.

In the present disclosure, the vinyl content in the R-vinyl polysiloxane is preferably 0.15 mol%-0.3 mol%, for example, 0.18 mol% or 0.23 mol%.

In the present disclosure, the vinyl content in the R-vinyl polysiloxane refers to the molar percentage of vinyl in the R-vinyl polysiloxane.

In the present disclosure, the polysiloxane can be any one of silicone rubber, silicone oil, and silicone resin, preferably silicone rubber.

In the present disclosure, the reinforcing agent is 20-70 parts by weight, for example, 30 parts, 40 parts, 45 parts or 50 parts.

In the present disclosure, the water contact angle of the reinforcing agent can be ≤90°, for example, 14-90°, 16-35° or ≤25°. The water contact angle in the present disclosure is the static water contact angle of the material, and its measurement is conducted based on the principle of optical appearance projection. During the process, the water droplet volume is set using the testing software, and the syringe needle drops the water droplet of the set volume onto the surface of the sample to be tested. Against a background of LED light source, the image of the droplet is projected into the automatic image analysis system by a screen micrometer for measurement. In the present disclosure, the hydrophilic group can be a conventional atomic group in the art that is soluble in water or readily affinity for water. Examples are one or more selected from the group consisting of hydroxyl group, mercapto group, aldehyde group, ketone group, ether bond, block polyether group, carboxyl group, carboxyl ester group, sulfate group, sulfone group, sulfinate group, sulfonate group, sulfonamide group, sulfonate ester group, thioether group, phosphate group, phosphoramide group, phosphinimide group, phosphate ester group, phosphite ester group, phosphino group, amino group, imino group, tertiary amino group, quaternary ammonium group, cyano group, cyanate ester group, thiocyanate group, amide group, and other oxygen-containing groups capable of forming hydrogen bonds with water molecules.

Wherein, the hydroxyl group can be an alcoholic hydroxyl group or a phenolic hydroxyl group.

In present disclosure, the reinforcing agent may be one or more selected from the group consisting of silica, hydroxyapatite, diatomaceous earth, quartz powder, silica micropowder, aluminum hydroxide, magnesium hydroxide, aluminum oxide, magnesium oxide, titanium dioxide, magnesium silicate, carbon black, zinc oxide, titanium dioxide, clay, lignin, carbonates, and kaolin. The reinforcing agent serves to increase the water permeability of the silicone material while

maintaining its mechanical properties.

Wherein, the silica is preferably hydrophilic silica or a mixture of "hydrophilic silica and hydrophobic silica".

The hydrophilic silica is preferably hydrophilic fumed silica, for example, one or more selected from the group consisting of hydrophilic fumed silica N20, hydrophilic silica TH-a200, hydrophilic fumed silica A380, hydrophilic fumed silica A300, T30 hydrophilic fumed silica, T40 hydrophilic fumed silica, and V15 hydrophilic fumed silica.

As a reinforcing agent for addition-type polysiloxanes, hydrophilic fumed silica is a white, fluffy powder with active hydroxyl groups on its surface, these hydroxyl groups readily connect and adsorb to polysiloxanes through hydrogen bonds and van der Waals forces, indirectly increasing the cross-linking density among reactant molecules and thus achieving a reinforcing effect. However, compared with hydrophobic silica, hydrophilic silica is detrimental to the mechanical properties of silicone tube. Since hydrophilic silica has not undergone silanization, the silanol groups on its surface tend to introduce moisture during the preparation process, which can cause bubbles in the resulting silicone tubes, thus affecting their mechanical properties and release characteristics. By adjusting the formulation of silicone material and optimizing the preparation process, the present disclosure significantly improves the drug release capacity of silicone material on the premise of satisfying the mechanical properties of silicone tubes.

The hydrophobic silica may be HB-615 hydrophobic silica, hydrophobic silica R106, hydrophobic silica R202, hydrophobic silica R812, hydrophobic silica R974, or hydrophobic silica AEROSIL R972.

In present disclosure, the hydrogen-containing silicone oil can be a conventional hydrogen-containing silicone oil in the art, for example. The hydrogen-containing silicone oil in the present disclosure can be used as a crosslinking agent to undergo a crosslinking reaction with R-vinyl polysiloxane. Through the addition reaction between Si-H in the crosslinking agent and $-CH=CH_2-$ of R-vinyl polysiloxane, the linear R-vinyl polysiloxane macromolecules are bonded and crosslinked with each other to form a three-dimensional network structure.

In the present disclosure, the mass of the hydrogen-containing silicone oil is calculated using the following formula (1):

$$W = \frac{A \times \omega\,(Vi)}{\omega\,(H) \times 27} \times W_1 \quad (1)$$

In the formula (1):

W: mass of hydrogen-containing silicone oil;
A: the molar ratio of Si-H in hydrogen-containing silicone oil to vinyl content in R-vinyl polysiloxane;
ω(Vi): the mass percentage of vinyl in R-vinyl polysiloxane;
ω(H): the content of Si-H group in hydrogen-containing silicone oil;
27: molar mass of vinyl group;
W1: mass of R-vinyl polysiloxane.

In the present disclosure, the amount of the hydrogen-containing silicone oil is 0.3-5 parts, preferably 0.3-3 parts, for example, 1.01 parts, 1.07 parts, 1.33 parts, 1.60 parts or 1.70 parts.

In the present disclosure, the Si-H group content in the hydrogen-containing silicone oil is 0.18-1.2 mol%, for example, 0.75mol%.

In the present disclosure, the molar ratio of Si-H groups in the hydrogen-containing silicone oil to vinyl groups in R-vinyl polysiloxane can be (0.5-8):1, preferably (0.8-5):1, for example, 0.8:1, 1:1, 1.2:1, 1.5:1, 1.8:1, 2:1 or 3:1.

In the present disclosure, the catalyst can be a conventional catalyst in the art that can catalyze the addition reaction between methyl vinyl polysiloxane and hydrogen-containing silicone oil, for example, a platinum catalyst.

Wherein, the concentration of platinum in the platinum catalyst is preferably $1\times10^3$-$1\times10^5$ ppm, for example, $1\times10^3$ ppm, $1\times10^4$ ppm, wherein ppm means that the mass concentration of platinum in the platinum catalyst is one part per million.

In the present disclosure, the amount of the catalyst used is 0.00001-0.5 parts, for example, 0.00001 parts, 0.0098 parts or 0.21 parts.

In the present disclosure, the raw material composition of the silicone material may further include an inhibitor, the function of the inhibitor is to inhibit the addition reaction between R-vinyl polysiloxane and hydrogen-containing silicone oil at room temperature.

In the present disclosure, the inhibitor is an alkyne alcohol compound, a nitrogen-containing compound, or an organic peroxide, for example, methylbutynol, and for another example, 2-methyl-3-butyn-2-ol.

In the present disclosure, the weight fraction of the inhibitor can be 0.03-2.0 parts, for example, 0.69 parts or 0.7 parts.

In the present disclosure, PHR refers to the parts by mass of each specific component corresponding to every

100 parts by mass of R-vinyl polysiloxane (e.g., methyl vinyl polysiloxane).

In some specific embodiments, the raw material composition of the silicone material may include the following components: wherein, the Si-H group content of the hydrogen-containing silicone oil is 0.75%;

| serial number | methyl vinyl polysiloxane | vinyl content in methyl vinyl polysiloxane | hydrophilic silica TH-a200 | the molar ratio of Si-H in hydrogen-containing silicone oil to vinyl groups in methyl vinyl polysiloxane | platinum catalyst (3000ppm) | 2-Methyl-3-butyn-2-ol |
|---|---|---|---|---|---|---|
| 1-1 | 100PHR | 0.18% | 40PHR | 1.5:1 | 0.0098PHR | 0.69PHR |
| 1-2 | 100PHR | 0.23% | 40PHR | 1.5:1 | 0.0098PHR | 0.69PHR |
| 2-1 | 100PHR | 0.18% | 30PHR | 1.2:1 | 0.0098PHR | 0.69PHR |
| 2-2 | 100PHR | 0.18% | 40PHR | 1.2:1 | 0.0098PHR | 0.69PHR |
| 2-3 | 100PHR | 0.18% | 45PHR | 1.2:1 | 0.0098PHR | 0.69PHR |
| 2-4 | 100PHR | 0.18% | 50PHR | 1.2:1 | 0.0098PHR | 0.69PHR |
| 3-1 | 100PHR | 0.18% | 30PHR | 1.5:1 | 0.0098PHR | 0.69PHR |
| 3-2 | 100PHR | 0.18% | 30PHR | 1.8:1 | 0.0098PHR | 0.69PHR |

In some specific embodiments, the raw material composition of the silicone material may include the following components: wherein, the Si-H group content of the hydrogen-containing silicone oil is 0.75%;

| serial number | methyl vinyl polysiloxane | vinyl content in methyl vinyl polysiloxane | hydrophilic silica A380 | the molar ratio of Si-H in hydrogen-containing silicone oil to vinyl groups in methyl vinyl polysiloxane | platinum catalyst (10,000ppm) | 2-Methyl-3-butyn-2-ol |
|---|---|---|---|---|---|---|
| 8-1 | 100PHR | 0.18% | 30PHR | 1.5:1 | 0.01PHR | 0.69PHR |
| 8-2 | 100PHR | 0.18% | 30PHR | 1.5:1 | 0.01PHR | 0.69PHR |
| 8-3 | 100PHR | 0.18% | 30PHR | 1.5:1 | 0.01PHR | 0.69PHR |
| 9-1 | 100PHR | 0.18% | 20PHR | 1.5:1 | 0.01PHR | 0.69PHR |
| 9-2 | 100PHR | 0.18% | 40PHR | 1.5:1 | 0.01PHR | 0.69PHR |
| 10 | 100PHR | 0.23% | 30PHR | 1.5:1 | 0.01PHR | 0.69PHR |
| 11-1 | 100PHR | 0.18% | 30PHR | 1.2:1 | 0.01PHR | 0.69PHR |
| 11-2 | 100PHR | 0.18% | 30PHR | 1.8:1 | 0.01PHR | 0.69PHR |

In some specific embodiments, the raw material composition of the silicone material may include the following components: wherein, the Si-H group content of the hydrogen-containing silicone oil is 0.75%;

| serial number | methyl vinyl polysiloxane | vinyl content in methyl vinyl polysiloxane | hydroxylapatite | HB-615 Hydrophobic Silica | the molar ratio of Si-H in hydrogen-containing silicone oil to vinyl groups in methyl vinyl polysiloxane | platinum catalyst (10000ppm) | 2-Methyl-3-butyn-2-ol |
|---|---|---|---|---|---|---|---|
| 12-1 | 100PHR | 0.18% | 30PHR | 20PHR | 1.5:1 | 0.21PHR | 0.98PHR |

(continued)

| serial number | methyl vinyl polysiloxane | vinyl content in methyl vinyl polysiloxane | hydroxylapatite | HB-615 Hydrophobic Silica | the molar ratio of Si-H in hydrogen-containing silicone oil to vinyl groups in methyl vinyl polysiloxane | platinum catalyst (10000ppm) | 2-Methyl-3-butyn-2-ol |
|---|---|---|---|---|---|---|---|
| 12-2 | 100PHR | 0.18% | 30PHR | 20PHR | 1.5:1 | 0.21PHR | 0.98PHR |
| 12-3 | 100PHR | 0.18% | 30PHR | 20PHR | 1.5:1 | 0.21PHR | 0.98PHR |
| 13 | 100PHR | 0.23% | 20PHR | 20PHR | 1.5:1 | 0.21PHR | 0.98PHR |
| 14-1 | 100PHR | 0.18% | 40PHR | 20PHR | 1.5:1 | 0.21PHR | 0.98PHR |
| 14-2 | 100PHR | 0.18% | 30PHR | 20PHR | 1.5:1 | 0.21PHR | 0.98PHR |

In some preferred embodiments, the raw material composition of the silicone material may include the following components:

methyl vinyl polysiloxane: 100 parts; the vinyl content in methyl vinyl polysiloxane is 0.18 mol%;
hydrophilic silica: 30 parts;
hydrogen-containing silicone oil: 1.33 parts; the molar ratio of Si-H groups in the hydrogen-containing silicone oil to vinyl groups in methyl vinyl polysiloxane is 1.5:1;
platinum catalyst (concentration 3000 ppm): 0.0098 parts;
2-Methyl-3-butyn-2-ol: 0.69 parts.

In some preferred embodiments, the raw material composition of the silicone material may include the following components:

methyl vinyl polysiloxane: 100 parts; the vinyl content in methyl vinyl polysiloxane is 0.18 mol%;
hydrophilic silica: 30 parts;
hydrogen-containing silicone oil: 1.33 parts; the molar ratio of Si-H groups in the hydrogen-containing silicone oil to vinyl groups in methyl vinyl polysiloxane is 1.5:1;
platinum catalyst (concentration 3000 ppm): 0.01 parts;
2-Methyl-3-butyn-2-ol: 0.69 parts.

In some preferred embodiments, the raw material composition of the silicone material may include the following components:

methyl vinyl polysiloxane: 100 parts; the vinyl content in methyl vinyl polysiloxane is 0.18 mol%;
hydroxyapatite: 20 parts;
HB-615 hydrophobic fumed silica: 20 parts;
hydrogen-containing silicone oil: 1.33 parts; the molar ratio of Si-H groups in the hydrogen-containing silicone oil to vinyl groups in methyl vinyl polysiloxane is 1.5:1;
platinum catalyst (concentration 3000 ppm): 0.21 parts;
2-Methyl-3-butyn-2-ol: 0.98 parts.

In some specific embodiments, the raw material composition of the silicone material may include the following components:

methyl vinyl polysiloxane: 100 parts; the vinyl content in methyl vinyl polysiloxane is 0.17 mol%;
hydrophilic fumed silica N20: 20 parts;
hydrophobic fumed silica R106: 20 parts;
the content of Si-H group in hydrogen-containing silicone oil is 0.75%; the molar ratio of Si-H groups in the hydrogen-containing silicone oil to vinyl groups in methyl vinyl polysiloxane is 1.2:1;

platinum catalyst: 0.00001 parts;
2-Methyl-3-butyn-2-ol: 0.7 parts.

The present disclosure also provides a method for preparing a silicone material, which employs any of the following methods:

Method 1:

S1. mixing R-vinyl polysiloxane and the reinforcing agent to obtain mixture A;
S2. dividing the mixture A into a component A1 and a component A2, the component A1 is mixed with the hydrogen-containing silicone oil to obtain a component B1, and the component A2 is mixed with a catalyst to obtain a component B2; optionally, the component B1 further comprises an inhibitor;
S3. mixing the component B1 and the component B2 to obtain a mixture B, and carrying out catalytic addition to obtain the silicone material.

Method 2:
the silicone material is obtained by catalytic addition of the raw material composition; the raw material composition includes: R-vinyl polysiloxane, reinforcing agent, hydrogen-containing silicone oil and catalyst; optionally, it may also include inhibitor.

In the present disclosure, the reinforcing agent can be pretreated using conventional methods in the art, the pretreatment method preferably involves drying at 90-220°C (e.g., 130°C) for 1-24 hours (e.g., 24 hours) for later use; for example, the pretreatment method involves drying at 130°C for 24 hours.
In the present disclosure, the R-vinyl polysiloxane can be pretreated using conventional methods in the art, the preferred pretreatment method involves drying at 20-90°C for 1-24 hours for later use; for example, the pretreatment method involves drying at 40°C for 12 hours.
In step S1, the mixing step can be achieved by adding the reinforcing agent to the R-vinyl polysiloxane in small portions multiple times for kneading, then subjecting to extrusion and thin-pass processing.
In step S2, the preparation method of component B1 can be as follows: refining component A1 in an open mill with a roller distance parameter of 1 unit, and the hydrogen-containing silicone oil and the inhibitor are added dropwise, then setting different roller distances after addition, performing triangular packing, and thin-pass processing, then taking out and allowing to stand for 24 hours for later use.
In step S2, the preparation method of component B2 can be as follows: refining component A2 in an open mill with a roller distance parameter of 1 unit, adding the catalyst dropwise, continuously shortening the roller distance, performing thin-pass processing, taking out and allowing to stand for 24 hours for later use.
In step S3, the mass ratio of component B1 to component B2 in mixture B can be 1:1.
In step S3, the preparation method of the mixture B involves placing component B1 and component B2 in an open mill for mixing and extrusion, and performing thin-pass processing for 6 times to obtain the mixture.
In step S3, the catalytic addition step can be performed by subjecting the mixture B to a first sulfidation treatment and a second sulfidation treatment in sequence, and preferably also includes a third sulfidation treatment.
Wherein, the temperature of the first sulfidation treatment can be 200°C-340°C, for example 250°C. The temperature of the first sulfidation treatment is also known as the pre-drying tunnel temperature. The silicone material passes through a short pre-drying tunnel (a 0.8m long vertical hot air sulfidation channel) after being extruded from the extruder die, which is the first sulfidation treatment. The inhibitors in the silicone material decompose into gas and volatilize when exposed to high temperature, and the catalyst then takes effect and the catalytic addition reaction proceeds, so that the silicone material undergoes preliminary sulfidation and shaping. The length of the pre-drying tunnel is short, which is mainly used to make the silicone material vulcanize and shape quickly in a short time, so as to prevent the silicone material from being flattened and deformed due to the gravity of the tube during conveyance on the conveyor belt, which would cause tube flattening and dimensional errors.
Wherein, the time of the first sulfidation treatment can be 2s-30s.
Wherein, the first sulfidation treatment can be carried out in a vertical hot air sulfidation channel. The first sulfidation treatment can perform preliminary high-temperature shaping of the rubber compound.
Wherein, the temperature of the second sulfidation treatment can be 180°C-280°C, for example 220°C, 240°C, 260°C or 280°C. The temperature of the second sulfidation treatment is also known as the post-drying tunnel temperature. After the preliminary sulfidation and shaping in the pre-drying tunnel, the silicone material is not fully crosslinked due to the short reaction time. Subsequently, under the traction of the conveyor belt, it passes through a long post-drying tunnel (a 2.5m long horizontal hot air sulfidation channel) for the second sulfidation treatment, so that the addition reaction proceeds more completely and the silicone material is further vulcanized to achieve good mechanical properties.

Wherein, the time of the second sulfidation treatment can be 0.1min- 5min.

Wherein, the second sulfidation treatment can be carried out in a horizontal hot air sulfidation channel. The second sulfidation treatment can fully vulcanize the rubber compound.

Wherein, the temperature of the third sulfidation treatment can be 150°C-240°C, for example 180°C. The sulfidation temperature has a significant impact on the mechanical properties and appearance of silicone materials, if the temperature is too low, the sulfidation will be insufficient, and the silicone material will be too soft and easily deformed, resulting in poor mechanical properties. If the temperature is too high, the silicone material is prone to over-sulfidation, becoming brittle, easily broken, and lacking elasticity.

In the present disclosure, the sulfidation principle of the two-component addition-type polysiloxane is as follows: the two-component addition-type polysiloxane vulcanized at room temperature, for example, uses vinyl polydimethylsiloxane as the base polymer and hydrogen-containing silicone oil as the crosslinking agent. Under the catalytic action of a catalyst (e.g., a platinum catalyst), the vinyl group and the hydrogen group undergo a hydrosilylation reaction to form a crosslinked network structure. The crosslinked structure can control the release of drugs. The reaction scheme is shown below.

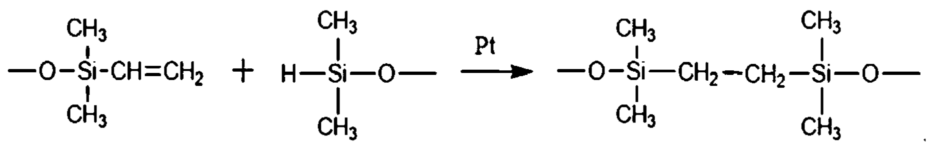


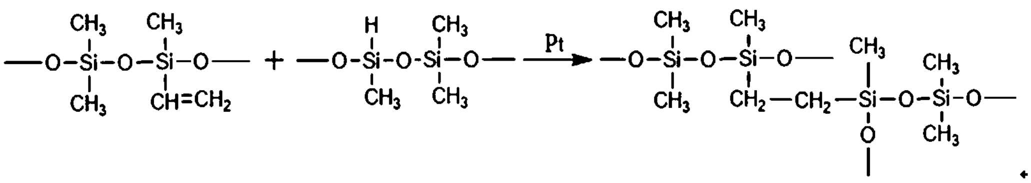


Raw rubber can react at room temperature after being mixed with crosslinking agents and catalysts, but the mixing and processing of rubber compounds require a certain period of time. If the reactants are cured prematurely during operation, the desired shape and properties cannot be obtained. This is especially true for addition-type polysiloxanes. Therefore, it is generally required that the catalytic reaction has almost no effect before sulfidation (mixing at room temperature), and reacts rapidly when the sulfidation temperature is reached. The method to inhibit the reaction is usually to add an inhibitor. Inhibitors can form certain complexes with platinum catalysts, effective inhibitors can be left with rubber compounds for a considerable period of time, and can only be vulcanized when heated to a certain sulfidation temperature. Commonly used compounds include alkynyl alcohols compounds, nitrogen-containing compounds, and organic peroxides, which have good compatibility.

The present disclosure also provides a silicone material, which is prepared by the preparation method described above.

The present disclosure also provides a method for preparing a silicone tube, wherein, the mixture B described above is formed into a tube by extrusion and then subjected to catalytic addition to obtain the silicone tube;
or, the catalytic addition process described above is carried out in a tubular mold to obtain the silicone tube.

The present disclosure also provides a silicone tube, which is prepared by the preparation method described above.

The present disclosure also provides an implant, wherein, it comprises a drug core and the silicone tube as described above, the drug core contains the pharmaceutically active ingredient.

In the present disclosure, the pharmaceutical active ingredient can be a conventional sustained-release pharmaceutical ingredient that needs long-term drug release in the field, preferably an active drug with a solubility of ≤100mg/mL (using water as a solvent).

In the present disclosure, the pharmaceutically active ingredient may comprise a pharmaceutically active ingredient acting on the reproductive system, or a pharmaceutically active ingredient acting on the urinary system, or a pharmaceutically active ingredient for chronic diseases related to metabolism and nutrition, or a pharmaceutically active ingredient for treating chronic diseases related to connective tissue and rheumatism, or a pharmaceutically active ingredient for treating hyperlipidemia, tumors, neuropsychiatric disorders, chronic dental diseases (dental caries, periodontal diseases), simple obesity, chronic low back pain, or leukemia, or a pharmaceutically active ingredient acting on the circulatory system, or a pharmaceutically active ingredient acting on the respiratory system, or a pharmaceutically active ingredient acting on the digestive system, or a pharmaceutically active ingredient acting on the hematological system, or a pharmaceutically active ingredient acting on the endocrine system.

Wherein, the pharmaceutically active ingredient acting on the reproductive system may comprise a pharmaceutically active ingredient for contraception, or a steroidal estrogen.

The active pharmaceutical ingredient used for contraception may be conventional in the art, and preferably includes levonorgestrel, pregnadienone, or pregnatrinone.

The steroidal estrogen is preferably estradiol.

Wherein, the active pharmaceutical ingredient acting on the urinary system preferably includes an active pharmaceutical ingredient for treating chronic nephritis, chronic renal failure, or chronic prostatitis.

The active ingredient of the drug for treating chronic prostatitis is preferably a nonsteroidal anti-inflammatory drug, and more preferably ibuprofen. Generally, the medications used to treat chronic prostatitis may be: antibiotics (e.g., fluoroquinolones such as norfloxacin, enoxacin, ofloxacin, ciprofloxacin, etc.), macrolides (e.g., erythromycin, roxithromycin, azithromycin, acetylspiramycin, etc.), tetracyclines (tetracycline), alpha-blockers (e.g., doxazosin, terazosin, etc.), nonsteroidal anti-inflammatory drugs (e.g., ibuprofen, diclofenac, loxoprofen, flurbiprofen ester, ketorolac, celecoxib, etc.), analgesics (e.g., acetaminophen, etc.), opioids (e.g., ibuprofen, fentanyl, nalbuphine, pentazocine, etc.), anti-neuropathic pain medications (e.g., serotonin, duloxetine, venfalacin), antiepileptics (e.g., pregabalin, etc.), M-blockers (e.g., solifenacin, tolterodine, etc.).

The medications used to treat chronic nephritis may generally be: angiotensin-converting enzyme inhibitors (such as benazepril, ramipril, fosinopril, perindopril, cilazapril, enalapril, etc.), angiotensin II receptor blockers (such as irbesartan, valsartan, losartan, etc.), calcium channel blockers (such as amlodipine, felodipine, nifedipine, etc.), beta-blockers (such as atenolol, bisoprolol, carvedalol, propranolol, etc.), diuretics (such as furosemide, spironolactone, hydrochlorothiazide, benzfluthiazide, bumetanide, etc.), immunosuppressants (such as prednisone, enrofloxacin, methylprednisolone, cyclophosphamide, dexamethasone, etc.), statins (such as fluvastatin, simvastatin, pravastatin).

The medications used to treat chronic renal failure may generally be: angiotensin-converting enzyme inhibitors (such as benazepril, ramipril, fosinopril, perindopril, cilazapril, enalapril, etc.), angiotensin II receptor blockers (such as irbesartan, valsartan, losartan, etc.), diuretics (such as furosemide, spironolactone, hydrochlorothiazide, benzfluthiazide, bumetanide, etc.), iron supplements (such as ferrous fumarate), compound amino acids, $\alpha$-keto acids, recombinant human erythropoietin.

Wherein, the active pharmaceutical ingredients that act on chronic diseases related to metabolism and nutrition preferably include active pharmaceutical ingredients for treating diabetes, nutritional deficiencies, gout, or osteoporosis.

The active pharmaceutical ingredient for treating gout is preferably an anti-gout drug, such as colchicine, indomethacin, diclofenac, ibuprofen, rofecoxib, prednisone, hydrocortisone, prednisolone, aspirin, diflunisal, para-aminosalicylic acid, disalicylate, benorilate, and more preferably ibuprofen. Generally, the aforementioned anti-gout drugs may also be uric acid excretion agents (such as benzbromarone, probenecid), uric acid synthesis blocking agents (such as allopurinol).

The antidiabetic drugs mentioned can generally be anti-type 1 diabetes drugs (such as insulin), anti-type 2 diabetes drugs. The anti-type 2 diabetes drugs may be sulfonylureas (e.g., glimepiride, glibenclamide), meglitinides (e.g., repaglinide, nateglinide), metformin (e.g., metformin), $\alpha$-glucosidase inhibitors (e.g., acarbose, voglibose), DPP-4 inhibitors (e.g., sitagliptin), GLP-1 receptor agonists (e.g., liraglutide, exenatide), or SGLT-2 inhibitors (e.g., dapagliflozin, empagliflozin).

The drugs used to treat nutritional deficiencies can generally be drugs for gastrointestinal diseases (such as omeprazole and mosapride), nutritional supplements (such as vitamin E).

The anti-osteoporosis drugs can generally be bisphosphonates (e.g., alendronate sodium, ibandronate sodium, pamidronate sodium, aminobisphosphonates, disodium chlorophosphate, zoledronic acid sodium, risedronate sodium), calcitonin (e.g., salmon calcitonin), estrogen (e.g., estradiol, estradiol benzoate, estradiol acetate, estradiol valerate), selective estrogen receptor modulators (SERMs) (e.g., raloxifene, benzothiophene), RANKL inhibitors, parathyroid hormone, monofluorophosphate glutamine, strontium salts (e.g., strontium ranelate), active vitamin D and analogues (e.g., calcitriol, $\alpha$-calciferol), vitamin K (e.g., tetraene-menaquinone).

Wherein, the active pharmaceutical ingredient used to treat chronic diseases involving connective tissue and rheumatism preferably includes active pharmaceutical ingredients for treating rheumatoid arthritis, systemic lupus erythematosus, ankylosing spondylitis, Sjögren's syndrome, vasculitis, idiopathic inflammatory myopathy, systemic sclerosis, or osteoarthritis.

The active pharmaceutical ingredient for treating rheumatoid arthritis is preferably a nonsteroidal anti-inflammatory drug, such as aspirin, meloxicam, celecoxib, ibuprofen, nimesulide, nabumetone, and more preferably meloxicam or ibuprofen. Generally, the drugs used to treat rheumatoid arthritis may be glucocorticoids (such as hydrocortisone, dexamethasone, prednisone), slow-acting antirheumatic drugs (such as methotrexate, cyclophosphamide, azathioprine, cyclosporine, leflunomide).

The active pharmaceutical ingredient for treating osteoarthritis is preferably an analgesic, such as ibuprofen, diclofenac, meloxicam, celecoxib, loxoprofen sodium, nimesulide, and more preferably ibuprofen or meloxicam. Generally, the drugs used to treat osteoarthritis may be cartilage-nourishing drugs, opioids (such as oxycodone, tamsulosin).

The drugs used to treat systemic lupus erythematosus may generally be nonsteroidal anti-inflammatory drugs (such as naproxen, ibuprofen), antimalarial drugs, glucocorticoids (such as prednisolone acetate, methylprednisolone), immunosuppressants (such as methotrexate, cyclophosphamide, azathioprine, methotrexate, cyclosporine, mycophenolate mofetil, tacrolimus), biologics (such as rituximab, belimumab).

The drugs for treating ankylosing spondylosis may generally be non-steroidal anti-inflammatory drugs (such as diclofenac sodium, etoricoxib, celecoxib, nabumetone, imrecoxib), disease-modifying anti-rheumatic drugs (such as sulfasalazine, methotrexate, hydroxychloroquine), glucocorticoids (such as prednisone, prednisolone, betamethasone dipropionate), biological TNF-$\alpha$ antagonist agents (such as etanercept).

The drugs for treating Sjögren's syndrome may generally be systemic therapeutic agents (such as levamisole, transfer factor coenzyme Q10, thymosin), glucocorticoid drugs (such as prednisone), immunosuppressive drugs (such as hydroxychloroquine, azathioprine, iguratimod).

The drugs for the treatment of vasculitis as mentioned may generally be: glucocorticoids (e.g., prednisone, methylprednisolone, dexamethasone), immunosuppressive agents (e.g., cyclophosphamide, cyclosporine, azathioprine).

The drugs for the treatment of idiopathic inflammatory myopathies as mentioned may generally be: glucocorticoids (e.g., dexamethasone, prednisone, hydrocortisone, methylprednisolone), immunosuppressive agents (e.g., cyclophosphamide, azathioprine, methotrexate, cyclosporine, tacrolimus, mycophenolate mofetil).

The drugs for treating systemic sclerosis may generally be antifibrotic drugs (e.g., colchicine), vasoactive agents (e.g., Nifedipine, Nifedipine GITS), and several other commonly used drugs (e.g., naproxen, nifedipine, bosentan, sildenafil, epoprostenol, nintedanib, tocilizumab).

Wherein, the active pharmaceutical ingredient for treating neuropsychiatric diseases may generally be an antipsychotic for schizophrenia, an antidepressant, a therapeutic agent for opioid abuse, or an anxiolytic; preferably an antipsychotic for schizophrenia, more preferably a phenothiazine (such as chlorpromazine), a thioxanthene (such as chlorprothixene), a butyrophenone (such as haloperidol), a benzodiazepine (such as olanzapine, clozapine), a benzisoxazole (such as risperidone, paliperidone), a benzisothiazole (such as ziprasidone), a dibenzothiazepine (such as quetiapine), a quinolone (such as aripiprazole), and even more preferably a benzisoxazole (such as paliperidone).

The aforementioned antidepressant drugs may generally be tricyclic antidepressants (such as imipramine, clomipramine, amitriptyline), monoamine oxidase inhibitors (such as moclobemide), selective serotonin reuptake inhibitors (such as sertraline), serotonin and norepinephrine reuptake inhibitors (such as venlafaxine), serotonin blocking and reuptake inhibitors (such as trazodone), norepinephrine and dopamine reuptake inhibitors (such as bupropion), norepinephrine reuptake inhibitors (such as reboxetine),$\alpha$2-adrenergic receptor blockers (such as mirtazapine).

The drugs for treating opioid abuse may generally be buprenorphine or methadone.

The anti-anxiety drugs may generally be benzodiazepines (such as diazepam), 5-HT1A receptor partial agonists (such as buspirone), $\beta$-adrenergic receptor blockers (such as propranolol), valproate.

Wherein, the lipid-lowering drugs may generally be statins (such as simvastatin, atorvastatin, pravastatin), fibrates (such as fenofibrate, bezafibrate, gemfibrozil), nicotinic acid agents (such as nicotinic acid).

Wherein, the antitumor drugs may generally be anti-breast cancer drugs (such as azacitidine, docetaxel, buserelin, tamoxifen, mitoxantrone, doxorubicin, paclitaxel, capecitabine, goserelin, cyclophosphamide, megestrol acetate, cetuximab or leuprorelin), anti-prostate cancer drugs (such as degarelix, leuprorelin, histrelin, flutamide, estramustine, cyproterone acetate), anti-ovarian cancer drugs (such as carboplatin, topotecan, methotrexate), anti-rectal cancer drugs (such as panitumumab), anti-colon cancer drugs (such as bevacizumab, oxaliplatin), anti-liver cancer drugs (such as sorafenib), anti-lung cancer drugs (such as erlotinib, gefitinib, gemcitabine), anti-renal cancer drugs (such as pazopanib, everolimus, temsirolimus), anti-gastric cancer drugs (such as fluorouracil, mitomycin, cisplatin, doxorubicin, etoposide), anti-pancreatic cancer drugs (such as nimotuzumab), anti-esophageal cancer drugs (such as docetaxel, paclitaxel, cisplatin, gemcitabine, tegafur/gimeracil/oteracil, irinotecan, oxaliplatin, gefitinib, trastuzumab, anlotinib), anti-skin cancer drugs (such as fluorouracil), anti-lymphoma drugs (such asvincristine, bexarotene, dacarbazine, etoposide), anti-myeloma drugs (such as bortezomib), anti-cervical cancer drugs (such as bleomycin) or anti-bladder cancer drugs (such as epirubicin, bacillus Calmette-Guérin).

Wherein, the drugs for treating chronic dental diseases (such as dental caries, periodontal disease) may generally be divided into nitroimidazoles (such as metronidazole, tinidazole, ornidazole), penicillins, and other commonly used drugs (minocycline, chlorhexidine acetate).

Wherein, the drugs for treating simple obesity may generally be orlistat.

Wherein, the pharmaceutically active ingredient for treating chronic low back pain is preferably a non-steroidal analgesic drug, such as ibuprofen, celecoxib, tramadol, oxycodone, meloxicam, loxoprofen, paracetamol/codeine, diclofenac sodium; more preferably ibuprofen or meloxicam.

The drugs for treating leukemia are generally drugs that interfere with nucleic acid biosynthesis (such as cytarabine, methotrexate and 6-mercaptopurine), drugs that directly affect the DNA structure and function of cancer cells (such as busulfan, mitomycin, chlorambucil, phenylalanine nitrogen mustard and cyclophosphamide), drugs that interfere

with transcription process and prevent RNA synthesis (such as daunorubicin, doxorubicin, aclacinomycin), drugs that inhibit the synthesis and function of protein (such as vincristine, vincristine, L-aspartase, homoharringtonine) and other commonly used drugs (such as interferon, fludarabine, arsenite, etoposide, carmustine).

Wherein, the active pharmaceutical ingredient acting on the circulatory system preferably includes active pharmaceutical ingredients for treating chronic heart failure, coronary heart disease, congenital heart disease, or chronic infective endocarditis, or chronic pericarditis.

The active pharmaceutical ingredient for treating coronary heart disease can generally be a drug that improves angina symptoms (such as puerarin, isosorbide mononitrate), an antiplatelet aggregation drug (such as aspirin, clopidogrel bisulfate, ticagrelor), a lipid-lowering and plaque-stabilizing drug (such as atorvastatin, rosuvastatin, pravastatin), a drug that inhibits sympathetic nerve activity (such as metoprolol, bisoprolol fumarate), or a drug that improves myocardial remodeling (such as angiotensin-converting enzyme inhibitors and angiotensin II receptor antagonists); preferably a drug that improves angina symptoms, more preferably puerarin; the active pharmaceutical ingredient for treating chronic heart failure can generally be a cardiotonic drug (such as digitalis, digoxin, deslanoside), a vasodilator (such as sodium nitroprusside, nitroglycerin, an angiotensin-converting enzyme inhibitor (such as enalapril, lenopril, etc.), a diuretic (such as furosemide, hydrochlorothiazide, spironolactone).

The active pharmaceutical ingredients of the drugs used to treat congenital heart disease may generally be digitalis, furosemide, spironolactone, phentolamine, quinidine, digoxin, hydrochlorothiazide, or coenzyme Q10.

The active pharmaceutical ingredient of the drugs used to treat chronic infective endocarditis may generally be an antibiotic (such as vancomycin, cephalosporins, penicillin, aminoglycosides).

The active pharmaceutical ingredient of the drugs used to treat chronic pericarditis may generally be digitalis.

Wherein, the active pharmaceutical ingredients acting on the respiratory system can generally include pharmaceutical active ingredients for treating chronic obstructive emphysema, asthma, chronic cor pulmonale, chronic respiratory failure, silicosis or pulmonary fibrosis.

The active pharmaceutical ingredients of the medicine for treating chronic obstructive emphysema can generally be bronchodilators (including beta agonists and anticholinergic drugs), inhaled sex hormones (such as budesonide, fluticasone), theophylline antiasthmatic drugs (such as theophylline), expectorants (such as carbomestane, fudosteine), and glucocorticoids, antibiotics (such as penicillins, glycosides and cephalosporins) can be appropriately selected when the condition requires.

The active pharmaceutical ingredients of the medicine for treating asthma can generally be commonly used inhaled drugs (such as beclomethasone, budesonide, fluticasone, mometasone), β2 agonists (such as salbutamol), sustained-release theophylline, leukotriene modulators (which can be used in combination), anticholinergic drugs (such as isopropanolamine) and antihistamines (such as astemizole, ketotifen).

The active pharmaceutical ingredients of the medicine for treating chronic cor pulmonale can generally be antibiotics (such as amoxicillin, ceftizoxime, cefuroxime, levofloxacin), corticosteroid anti-inflammatory bronchodilators (such as selective β2 receptor agonists, theophylline drugs), airway nonspecific inflammation elimination (such as prednisone), inhaled drugs (such as buprenone), respiratory stimulants (such as lobeline, doxapram, Duxil, etc.).

The active pharmaceutical ingredients of the drugs used to treat chronic respiratory failure may generally be bronchospasm-relieving and expectorant drugs (such as salbutamol, acetylcysteine, etc.).

The active pharmaceutical ingredients of the drugs for treating silicosis can generally be Siping, acetylcysteine, aluminum preparation, kesipin, salvia miltiorrhiza.

The active pharmaceutical ingredients of the drugs for treating pulmonary fibrosis can generally be pirfenidone, nindanib, glucocorticoids (such as methylprednisolone, prednisone), immunosuppressants (such as azathioprine, methotrexate), colchicine, interferon, ACEI or statins, etc.

Wherein, the active pharmaceutical ingredients that act on the digestive system may generally include active pharmaceutical ingredients for treating chronic gastritis, peptic ulcers, intestinal tuberculosis, chronic enteritis, chronic diarrhea, chronic hepatitis, cirrhosis, chronic pancreatitis, chronic cholecystitis.

The active pharmaceutical ingredients of the drugs for treating chronic gastritis can generally be analgesics (atropine, propofol, etc.), PPI proton pump inhibitors (such as lansoprazole, omeprazole, etc.) for elevated gastric acid, H2 receptor blockers (such as cimetidine, ranitidine, aluminum hydroxide amine, etc.) for mild symptoms, digestive AIDS (pancreatic enzyme), agents for bile reflux (metoclopramide, domperidone, cholestyramine, sucralfate, which can bind to bile acids).

The active pharmaceutical ingredients of the drugs for treating peptic ulcer can generally be levofloxacin, tinidazole, omeprazole.

The active pharmaceutical ingredient of the drugs for treating intestinal tuberculosis can generally be rifampicin.

The active ingredients of the medicine for treating chronic enteritis can generally be anti-inflammatory painkillers, probiotics, antispasmodic painkillers (for example, atropine and propofol).

The active pharmaceutical ingredients of the drugs for treating chronic diarrhea can generally be antidiarrheal drugs (such as montmorillonite powder, diphenoxylate, loperamide), intestinal microbial agents (such as lactobacillus,

bifidobacteria), spasmodic painkillers (such as pinaverium bromide).

The active pharmaceutical ingredients of the drugs for treating chronic hepatitis can generally be liver-protecting drugs (such as silymarin preparations, Schisandra preparations, etc.), anti-fibrosis drugs (such as Chinese patent medicine oral preparations), anti-virus drugs (such as common interferon and pegylated interferon), oral nucleoside drugs anti-virus drugs (such as lamivudine, adefovir dipivoxil, telbivudine, entecavir) and immunosuppressants (azathioprine).

The active pharmaceutical ingredients of the drugs for treating liver cirrhosis can generally be drugs for treating hepatitis B (such as nucleoside analogues), drugs for treating autoimmune hepatitis (such as glucocorticoids), anti-inflammatory drugs, liver-protecting drugs, anti-hepatic fibrosis drugs (such as reduced glutathione, polyene phosphatidylcholine, magnesium isoglycyrrhizinate, etc.), drugs for treating spontaneous bacterial peritonitis (such as antibiotics), drugs for treating portal hypertension (such as carvedilol).

The active pharmaceutical ingredients of the drugs for treating chronic pancreatitis can generally be analgesic drugs (such as buprenorphine and fentanyl), pancreatin therapy drugs (such as pancreatin).

The active pharmaceutical ingredients of the drugs for treating chronic cholecystitis can generally be antibacterial and anti-inflammatory drugs (such as levofloxacin, ciprofloxacin, amoxicillin), antispasmodic painkillers, cholagogic drugs (such as ursodeoxycholic acid).

Wherein, the active pharmaceutical ingredients acting on the blood system can generally include pharmaceutical active ingredients for treating chronic anemia, chronic myeloid leukemia, chronic lymphocytic leukemia.

The drugs for treating chronic anemia can generally be: trace element drugs (such as folic acid, vitamin B12, etc.), bone marrow stimulants (such as strychnine nitrate, securinine, hyoscyamine drugs (hyoscyamine), etc.), cobamamide, glucocorticoids (prednisone, methylprednisone, betamethasone, beclomethasone dipropionate, prednisolone, hydrocortisone, dexamethasone, prednisone), iron preparations (such as ferrous fumarate, ferrous gluconate, ferrous succinate, ferrous lactate, iron sucrose, iron dextran, iron carboxymaltose, iron isomaltoside, iron glucuronate, nano-iron oxide, iron sorbitol, etc.), erythropoiesis-stimulating drugs (recombinant human erythropoietin $\alpha$, darbepoetin $\alpha$, etc.).

The drugs used to treat chronic myeloid leukemia may generally be: tyrosine kinase inhibitors (such as imatinib, nilotinib, bosutinib, ponatinib, etc.), homoharringtonine.

The drugs used to treat chronic lymphocytic leukemia may generally be: chemotherapy drugs (such as nimustine, fludarabine, chlorambucil, bendamustine, etc.), targeted drugs (such as edarab, venetumab, ibrutinib, imatinib, dasatinib, etc.), monoclonal antibody drugs (such as oflamb, rituximab, atoruzumab, alenzusmab, etc.).

Wherein, the pharmaceutical active ingredients acting on endocrine system can generally include pharmaceutical active ingredients for treating chronic lymphocytic thyroiditis, hyperthyroidism, hypothyroidism.

The drugs for the treatment of chronic lymphocytic thyroiditis as mentioned may generally be: thyroid hormones (e.g., levothyroxine, thyroxine), glucocorticoids (e.g., prednisone, methylprednisolone, betamethasone, beclomethasone dipropionate, prednisolone, hydrocortisone, dexamethasone).

The drugs for the treatment of hyperthyroidism as mentioned may generally be: thiouracils (e.g., propylthiouracil, methylthiouracil, etc.), imidazoles (e.g., methimazole, carbimazole, etc.), iodine and iodides (e.g., Lugol's solution, etc.), radioactive iodine (e.g., iodine-131, etc.), and $\beta$-blockers (e.g., metoprolol, atenolol, bisoprolol, carvedilol, propranolol, etc.).

The drugs for the treatment of hypothyroidism as mentioned may generally be: thyroid hormones (e.g., levothyroxine, levothyroxine sodium, thyroxine, etc.).

In the present disclosure, the preparation method of the drug core can be: subjecting t the active pharmaceutical ingredients and polysiloxane to mixing, extrusion, and sulfidation treatment in sequence, to obtain the drug core.

Wherein, the polysiloxane includes R-vinyl polysiloxane, hydrogen-containing silicone oil, catalyst, and inhibitor.

Wherein, the temperature of the sulfidation treatment can be 70°C-160°C.

Wherein, the time of the sulfidation treatment can be 0.5-10h.

Wherein, the raw material composition of the drug core may be as follows: in parts by weight, 100 parts of active pharmaceutical ingredient, 100 parts of R-vinyl polysiloxane, 0.21 parts of catalyst, 0.98 parts of inhibitor, and the molar ratio of Si-H in the hydrogen-containing silicone oil to vinyl in the R-vinyl polysiloxane is 4.5:1.

The present disclosure provides a method for preparing the implants as described above, which includes the following steps: filling the drug core material into the silicone tube and then sealing the end to obtain the implants.

In the present disclosure, the drug core can be a skeleton-type rod core or a powder-type drug core.

When the drug core is a skeleton-type rod core, the length of the silicone tube is 0.4 cm longer than the length of the skeleton-type rod core. A length of 0.2cm is reserved at each end of the silicone tube for sealing the implant.

When the drug core is a skeleton-type rod core, the filling method is preferably to quickly insert the skeleton-type rod core into the silicone tube when it is in a swollen state, which can be achieved by immersing the silicone tube in petroleum ether for 1-2 min. After filling, it is preferably further air-dried naturally for 12-24 hours; the purpose of natural air drying is to allow the residual petroleum ether to evaporate completely.

When the drug core is a powder-type drug core, the preferred filling method is to first seal one end of the silicone

tube and then fill it through a filling funnel.

In the present disclosure, the end-sealing can be carried out using a sealing adhesive; the sealing adhesive is preferably silicone.

In the present disclosure, after end-sealing, it is preferably placed at room temperature for 12-24 h to allow the sealing adhesive to cure.

In the present disclosure, the silicone tube is preferably further subjected to pretreatment, which comprises soaking in 75% ethanol for 15 min, then rinsing repeatedly with 75% ethanol several times, followed by natural air-drying.

The present disclosure also provides a use of the aforementioned silica material or the aforementioned silicone tube as a release rate adjusting medium in a sustained and controlled release formulation.

The technical problem to be solved by the present disclosure is to overcome the defects of the narrow application scope of existing implants, especially the scarcity of implants suitable for drugs with high solubility, heat-sensitive drugs or macromolecular drugs, the present disclosure provides a rod core, a perforated implant, a preparation method and use thereof. The implant of the present disclosure is applicable to most drugs and can achieve long-term drug release. Through a large number of experimental studies, the inventors in the present disclosure adopts a solid compression molding method to compress active drugs and excipients into rod cores, which not only solves the problems of drug waste or unstable drug release in powder-type drug cores, but also solves the problem of drug instability in the preparation process of silicone skeleton-type rod cores. The perforation of the silicone tube increases the amount of drug released and expands the application scope of the implant.

The present disclosure solves the above-mentioned technical problems through the following technical solutions.

The present disclosure provides a rod core for a perforated implant, which comprises a drug-containing layer, the drug-containing layer contains an active drug and excipients;

> wherein, the excipients comprise sustained and controlled release excipients; the molecular weight of the sustained and controlled release excipients is 1000-10,000,000;
> the drug-to-excipient ratio is 1:(0.01-100); the drug-to-excipient ratio represents the mass ratio of the active drug to the excipients in the drug-containing layer;
> optionally, the rod core of the perforated implant further includes a booster layer composited on the drug-containing layer.

In the present disclosure, the active drug may include conventional drugs in the field, such as one or more selected from the group consisting of macromolecular drugs with good solubility, small molecule drugs with good solubility, insoluble macromolecular drugs, and insoluble small molecule drugs.

Wherein, the macromolecular drug with good solubility may be a conventional active drug in the field with a solubility of >10 mg/mL and a molecular weight of more than 800 Da, preferably comprising an active drug with a drug solubility > 30 mg/mL and a drug molecular weight greater than 800 Da, for example, one or more selected from the group consisting of exenatide, semaglutide, total asiaticoside and human growth hormone.

Wherein, the small-molecule drug with good solubility may be a conventional active drug in the field with a solubility of >10 mg/mL and a molecular weight of less than or equal to 800 Da, preferably including an active drug with a drug solubility > 30 mg/mL and a drug molecular weight less than or equal to 800 Da, for example, metformin and/or doxorubicin hydrochloride.

Wherein, the insoluble macromolecular drug may be a conventional active drug in the field with a solubility of ≤10 mg/mL and a molecular weight of more than 800 Da, preferably including an active drug with a drug solubility ≤5 mg/mL and a drug molecular weight greater than 800 Da, for example, one or more selected from the group consisting of bovine insulin, paclitaxel and levothyroxine sodium.

Wherein, the insoluble small molecule drug may be a conventional active drug in the field with a solubility of≤10 mg/mL and a molecular weight of less than or equal to 800 Da, preferably comprising an active drug with a drug solubility ≤5 mg/mL and a molecular weight of less than or equal to 800 Da, for example, one or more selected from the group consisting of estradiol, pregnardene, levonorgestrel, meloxicam, ganciclovir, puerarin and paliperidone.

In the present disclosure, the weight-average molecular weight of the sustained and controlled release excipient is preferably 1,100 - 8,000,000, for example, 1,171, 10,000, 44,000, 200,000, 300,000, 400,000 or 7,000,000.

In the present disclosure, the sustained and controlled release excipient preferably comprises a insoluble matrix material and/or a hydrophilic gel material.

Wherein, the weight-average molecular weight of the insoluble matrix material is preferably 2,000 to 8,000,000, more preferably 3,000 to 7,000,000;

Wherein, the insoluble matrix material preferably comprises one or more selected from the group consisting of calcium alginate, methyl cellulose, polyethylene, polyvinyl chloride, methacrylic acid-methyl acrylate copolymer and ethyl cellulose, such as ethyl cellulose. The methacrylic acid-methyl acrylate copolymer is preferably one or more selected from

the group consisting of Eudragit S100, Eudragit L100, RL, RS, NE and FS, such as Eudragit S100.

Wherein, the weight-average molecular weight of the hydrophilic gel material may be 1,000 to 8,000,000, preferably 10,000 to 7,000,000.

Wherein, the hydrophilic gel material preferably comprises one or more selected from the group consisting of hydroxypropyl methyl cellulose, polyoxyethylene, sodium alginate, chitosan, agar, xanthan gum, carbomer, polyvinyl alcohol, N-vinyl amide polymer, polylactic-co-glycolic acid (PLGA) and polyethylene oxide. The hydroxypropyl methyl cellulose is preferably one or more selected from the group consisting of HPMC K100M, HPMC K15M and HPMC K4M. The N-vinyl amide polymer is preferably PVP K30 and/or PVP K90. The polyethylene oxide is preferably one or more selected from the group consisting of PEO N80, PEO N-750, PEO WSR N-12K, PEO WSR N-60K, PEO WSR 303 and PEO WSR 1105.

Wherein, when the sustained and controlled release excipient is a insoluble matrix material and a hydrophilic hydrogel material, the mass ratio of the insoluble matrix material to the hydrophilic hydrogel material is preferably 1:(0.01-100), such as 1:0.01, 1:0.05, 1:0.1, 1:0.2, 1:0.5, 1:1, 1:1.5, 1:3, 1:4.33, 1:5, 1:7, 1:10, 1:20, 1:40, 1:60, 1:80 or 1:100.

In a preferred embodiment, the active drug is insulin, and the excipients are the sustained and controlled release excipients and the drug stabilizers, such as HPMC K4M and trehalose. The mass ratio of the sustained and controlled release excipients to the excipients can be 1:4.33.

In a preferred embodiment, the active drug is semaglutide, and the excipients are the sustained and controlled release excipients and the fillers, such as HPMC K4M and MCC. The mass ratio of the sustained and controlled release excipients to the excipients can be 1:4.33.

In a preferred embodiment, the active drug is metformin, and the excipients are the sustained and controlled release excipients and the fillers, such as HPMC K4M and MCC. The mass ratio of the sustained and controlled release excipients to the excipients can be 1:4.33.

In a preferred embodiment, the active drug is paliperidone, and the excipients are the sustained and controlled release excipients and the fillers, such as HPMC K4M and MCC. The mass ratio of the sustained and controlled release excipients to the excipients can be 1:4.33.

In a preferred embodiment, the active drug is exenatide, the excipients are the sustained and controlled release excipients, and the sustained and controlled release excipients are preferably one or more selected from the group consisting of Eudragit S100, calcium alginate, ethyl cellulose, PLGA, HPMC K15M and PEO N80, such as ethyl cellulose, or ethyl cellulose and HPMC K15M.

In a preferred embodiment, the active drug is metformin, and the excipients is the sustained and controlled release excipients, which preferably include one or more selected from the group consisting of ethyl cellulose, HPMCK 15M, HPMCK 4M, and PEO N80, such as HPMCK 15M, HPMCK 4M, or ethyl cellulose and HPMCK 15M.

In a preferred embodiment, the active drug is semaglutide, and the sustained and controlled release excipients preferably include one or more selected from the group consisting of Eutrapeptide S100, calcium alginate, ethyl cellulose, PLGA, HPMCK 15M, and PEO N80, such as ethyl cellulose, or ethyl cellulose and HPMCK 15M.

In a preferred embodiment, the active drug is levothyroxine sodium, and the excipients preferably include the sustained and controlled release excipients and the fillers, such as PEO N-750 and MCC.

In the present disclosure, the drug-to-excipient ratio is preferably 1:(0.01-99), for example 1:0.01, 1:0.05, 1:0.1, 1:0.2, 1:0.5, 1:1, 1:1.5, 1:3, 1:4.33, 1:5, 1:7, 1:10, 1:20, 1:40, 1:60 or 1:80.

In the present disclosure, the excipients further include one or more selected from the group consisting of drug stabilizers, fillers and suspending agents.

Wherein, the drug stabilizer is preferably trehalose.

Wherein, the filler is preferably one or more selected from the group consisting of starch, dextrin, microcrystalline cellulose (MCC), and lactose.

Wherein, the suspending agent is preferably hydroxypropyl methylcellulose and/or polyethylene oxide.

Wherein, the mass ratio of the sustained and controlled release excipients to the excipients is preferably 1:(0.01-100), for example, 1:0.01, 1:0.05, 1:0.1, 1:0.2, 1:0.5, 1:1, 1:1.5, 1:3, 1:4.33, 1:5, 1:7, 1:10, 1:20, 1:40, 1:60, 1:80 or 1:100.

In the present disclosure, the concentration of the active drug in the drug-containing layer can be set as a gradient concentration. The gradient is preferably 1-50 segments, for example, 2 segments, 3 segments, 5 segments, 10 segments, 20 segments, or 40 segments; the gradient concentration is generally expressed such that the concentration of each gradient is independent.

When the gradient has three segments, the gradient is preferably set as a first gradient drug release module, a second gradient drug release module and a third gradient drug release module in sequence. The concentration of the active drug in the first gradient drug release module is preferably 0.1 %-50%, for example 2%, 5% or 10%. The concentration of the active drug in the second gradient drug release module is preferably 1%-80%, for example 10% or 20%. The concentration of the active drug in the third gradient drug release module is preferably 1%-100%, for example

20% or 30%. The concentration of the active drug generally represents the percentage of the mass of the active drug relative to the mass of the gradient drug release module.

In a preferred embodiment, the concentration of the active drug in the drug-containing layer is set in a three-segments manner, with the concentration of the active drug in the first gradient drug release module being 2% and the concentration of the active drug in the second gradient drug release module being 10%. The concentration of the active drug in the third gradient drug release module is preferably 20%.

In a preferred embodiment, the concentration of the active drug in the drug-containing layer is set in a three-segments manner, with the concentration of the active drug in the first gradient drug release module being 2% and the concentration of the active drug in the second gradient drug release module being 20%. The concentration of the active drug in the third gradient drug release module is preferably 30%.

In a preferred embodiment, the concentration of the active drug in the drug-containing layer is set in a three-segments manner, with the concentration of the active drug in the first gradient drug release module being 10% and the concentration of the active drug in the second gradient drug release module being 20%. The concentration of the active drug in the third gradient drug release module is preferably 30%.

In a preferred embodiment, the concentration of the active drug in the drug-containing layer is set in a three-segments manner, with the concentration of the active drug in the first gradient drug release module being 5% and the concentration of the active drug in the second gradient drug release module being 20%. The concentration of the active drug in the third gradient drug release module is preferably 30%.

In the present disclosure, when the rod core further comprises a booster layer, the raw materials of the booster layer preferably include expandable materials and penetration enhancer.

Wherein, the expandable materials in the booster layer can be polyethylene oxide, preferably polyethylene oxide with a weight-average molecular of 10-7,000,000, for example, one or more selected from the group consisting of PEO N-80, PEO N-750, PEO WSR 1105, PEO WSR 303, PEO N-12K and PEO N-60K.

The penetration enhancer in the booster layer can be a conventional substance in the field that can increase osmotic pressure, is preferably one or more of selected from the group consisting mannitol, lactose, fructose, glucose, and NaCl, for example, NaCl.

Wherein, the mass ratio of the expandable material and the permeation enhancer in the booster layer is preferably (0.01-100):1, for example, 0.01:1, 0.02:1, 0.05:1, 0.1:1, 0.2:1, 0.5:1, 1:1, 2:1, 3:1, 4:1, 5:1, 10:1, 20:1, 40:1, 60:1, 80:1 or 100:1.

In the present disclosure, when the rod core further comprises a booster layer, the mass ratio of the drug-containing layer to the booster layer can be (0.01-100):1, for example, 0.01:1, 0.02:1, 0.05:1, 0.1:1, 0.2:1, 0.5:1, 1:1, 2:1, 3:1, 5:1, 10:1, 20:1, 40:1, 60:1, 80:1 or 100:1.

In the present disclosure, when the rod core further comprises a booster layer, the preparation method of the booster layer can be conventional in the field, such as pressing the raw material of the booster layer.

Wherein, the pressing can be accomplished by solid column molding. The pressing pressure can be 0.01-10 MPa.

In the present disclosure, when the rod core further comprises a booster layer, the number of booster layers can be an integer, preferably an even number, for example, two. When the number of booster layers is an even number, the booster layers are preferably disposed at both ends of the drug-containing layer.

In the present disclosure, the single pressing length of the rod core is preferably 1-50mm, for example, 1mm or 2mm or 3mm or 4mm or 5mm or 7mm or 10mm or 20mm or 30mm or 40mm or 50mm. The total length of the rod core can be selected according to the length of the silicone tube.

In the present disclosure, the total length of the rod core is preferably 1-100mm, more preferably 5-60mm, for example 7mm or 10mm or 15mm or 18mm or 20mm or 30mm or 40mm or 50mm or 60mm.

In the present disclosure, the diameter of the rod core is preferably 1-10 mm, more preferably 1-5 mm, for example, 2 mm or 3 mm or 4 mm.

The present disclosure also provides a method for preparing a perforated implant rod core as described above, which comprises the following steps: pressing the active drug and the excipients to obtain the drug-containing layer; optionally, the booster layer is composited onto the drug-containing layer.

In the present disclosure, the pressing pressure is 0.01-10 MPa, for example, 0.1, 0.2, 0.3, 0.5, 1, 2, 5 or 10 MPa.

In the present disclosure, the pressing can be accomplished by solid column molding.

The present disclosure also provides a perforated implant, which comprises a rod core for a perforated implant as described above and a silicone tube with drug release holes covering the outside of the rod core.

In the present disclosure, when the silicone tube covers the outside of the rod core, the center point of the rod core and the center point of the silicone tube can coincide.

In the present disclosure, the plane where the rod core is located can be perpendicular to the radial plane of the silicone tube.

In the present disclosure, the size, position, and number of drug release holes of the silicone tube can be adjusted

according to the actual requirements of drug release amount.

In the present disclosure, in the silicone tube with the drug release holes, the ratio of the diameter of drug release holes to the outer diameter of the silicone tube can be (0.0001-0.5):1, preferably (0.002-0.4):1, for example, 0.06:1, 0.14:1 or 0.23:1, more preferably (0.005-0.2):1.

In the present disclosure, the diameter of the drug release holes is preferably 1$\mu$m-1000$\mu$m, more preferably 10-600$\mu$m, for example, 10, 20, 50, 100, 150, 250, 350, 450 or 550$\mu$m, further preferably 250-450$\mu$m.

In the present disclosure, in the silicone tube with drug release holes, the total area of the drug release holes in the silicone tube with drug release holes can be adjusted according to the actual requirements of drug release amount, for example, the ratio of the total area of the drug release holes to the outer surface area of the silicone tube can be (0.00001-0.4):1, preferably (0.0001-0.35):1, more preferably (0.0005-0.3):1, for example, 0.0005:1, 0.001:1, 0.005:1, 0.01:1, 0.05:1, 0.1:1 or 0.2:1.

In the present disclosure, the number of drug release holes is preferably >1, more preferably 1-1,000,000, for example, 1, 2, 5, 10, 20, 50, 100, 200, 500, 1,000, 5,000, 10,000, 50,000, 100,000, 500,000 or 1,000,000.

In the present disclosure, preferably, the position of the drug release holes satisfies the following conditions: the silicone tube includes a first end surface and a second end surface disposed opposite to each other, the distance between the first end surface and the second end surface is the total length of the silicone tube, and a plurality of drug release holes are provided in the silicone tube.

When the number of drug release holes is one, the distance from the center point of the drug release hole to the first end surface of the silicone tube with the drug release hole can be 0-100% of the length of the silicone tube, but not 0 and 100%, preferably the distance from the center point of any of the drug release holes to the first end surface of the silicone tube with the drug release hole is 0.1%-99.9% of the length of the silicone tube, more preferably 1%-99%, for example, 1%, 2%, 3%, 5%, 7%, 7.5%, 10%, 20%, 30%, 40%, 50%, 60%, 70%, 80%, 90%, 95%, 98% or 99%.

When the number of drug release holes is ≥2, the distance between the center points of the drug release holes can satisfy the following conditions:

when the planes of any two of the drug release holes are not on the same radial cross-section of the silicone tube, in the axial cross-section direction of the silicone tube, the distance from the center point of any of the drug release holes to the first end surface of the silicone tube with the drug release holes can be 0-100% of the length of the silicone tube, but not 0 and 100%, preferably the distance from the center point of any of the drug release holes to the first end surface of the silicone tube with the drug release holes is 0.1%-99.9% of the length of the silicone tube, more preferably 1%-99%, for example, 1%, 2%, 3%, 5%, 7%, 7.5%, 10%, 20%, 30%, 40%, 50%, 60%, 70%, 80%, 90%, 95%, 98% or 99%; the distance between the center points of adjacent drug release holes can be 0.001-80 mm.

When the number of drug release holes is ≥2, the distance between the center points of the drug release holes can also satisfy the following conditions:

when the planes of any two of the drug release holes are on the same radial cross-section of the silicone tube, the distance from the center point of any of the drug release holes to the first end surface of the silicone tube with the drug release holes can be 0-100% of the length of the silicone tube, but not 0 and 100%, preferably the distance from the center point of any of the drug release holes to the first end surface of the silicone tube with the drug release holes is 0.1%-99.9% of the length of the silicone tube, more preferably 1%-99%, for example, 1%, 2%, 3%, 5%, 7%, 7.5%, 10%, 20%, 30%, 40%, 50%, 60%, 70%, 80%, 90%, 95%, 98% or 99%; the circumferential distance between the center points of adjacent drug release holes can be (2-10)$\pi$ mm.

In a preferred embodiment, in a silicone tube with drug release holes, the pore diameter of the drug release holes is 350$\mu$m, the ratio of the pore diameter of the drug release holes to the outer diameter of the silicone tube is 0.35 mm: 2.4 mm, and the number of the drug release holes is one, the positions of the drug release holes meet the following conditions: the distance from the center of the drug release holes to the first end surface of the silicone tube with drug release holes is 50% of the length of the silicone tube.

In a preferred embodiment, in a silicone tube with drug release holes, the pore diameter of the drug release holes is 350$\mu$m, the ratio of the pore diameter of the drug release holes to the outer diameter of the silicone tube is 0.35 mm: 2.4 mm, and the number of the drug release holes is two, the positions of the drug release holes meet the following conditions: the planes of the two drug release holes are not on the same radial section of the silicone tube, in the direction of the axial section of the silicone tube, the distance between the center point of any drug release hole and the first end surface of the silicone tube with drug release holes is 50% of the length of the silicone tube, and the distance between the center points of two drug release holes is 0.3cm.

In a preferred embodiment, in a silicone tube with drug release holes, the pore diameter of the drug release holes is 350$\mu$m, the ratio of the pore diameter of the drug release holes to the outer diameter of the silicone tube is 0.35 mm: 2.4 mm, and the number of the drug release holes is one, the positions of the drug release holes meet the following conditions: the distance from the center of the drug release holes to the first end surface of the silicone tube with drug release holes is 7.5% of the length of the silicone tube.

In the present disclosure, the drug release holes can be obtained by laser drilling or mechanical drilling.

In the present disclosure, the raw material composition of the silicone tube preferably comprises the following components in parts by weight:

R-vinyl silicone rubber: 100 parts;
reinforcing agent: 20-80 parts;
hydrogen-containing silicone oil: 0.3-3.0 parts;
catalyst: 0.000002-1 part;
wherein, R in the R- vinyl silicone rubber is preferably a substituted or unsubstituted C1-C5 straight-chain alkane or branched-chain alkane, a substituted or unsubstituted C6-C20 aromatic hydrocarbon, and more preferably a substituted or unsubstituted C1-C3 straight-chain alkane, such as methyl.

When the R is methyl, the R- vinyl silicone rubber is methyl vinyl silicone rubber.
Wherein, the vinyl content in the R- vinyl silicone rubber can be 0.10%-0.50mol%, such as 0.18mol% or 0.23mol%.
Wherein, the reinforcing agent may be a conventional reinforcing agent in the art that can improve the hardness of the R-vinyl silicone rubber, such as one or more selected from the group consisting of silica, diatomaceous earth, quartz powder, silica powder, calcium carbonate, aluminum hydroxide, magnesium oxide, titanium dioxide, magnesium silicate, carbon black, zinc oxide, iron oxide, titanium dioxide, zirconium silicate and calcium carbonate, for example, silica.
Wherein, the reinforcing agent is preferably 30-50 parts by weight, such as 40 parts.
Wherein, the content of Si-H groups in the hydrogen-containing silicone oil can be 0.18-1.6mol%, preferably 0.5-1mol%, such as 0.75mol%.
Wherein, the molar ratio of Si-H groups in the hydrogen-containing silicone oil and vinyl groups in the R- vinyl silicone rubber can be (0.5-10):1, for example, 1.2: 1 or 2.5: 1.
Wherein, the catalyst can be a conventional catalyst in the field that can catalyze the addition reaction between R-vinyl silicone rubber and the hydrogen-containing silicone oil, such as a platinum catalyst.
Wherein, the catalyst is preferably 0.000005-0.5 parts by weight, for example 0.00001 parts, 0.0098 parts or 0.21 parts.
Wherein, the raw material composition of the silicone tube with drug release holes can further comprise an inhibitor, and the inhibitor is used for inhibiting the addition reaction of R-vinyl silicone rubber and hydrogen-containing silicone oil at normal temperature.
The inhibitor can be alkynol compounds, nitrogen-containing compounds or organic peroxides, such as 2-methyl-3-butyn-2-ol.
The inhibitor can be 0.03-2.0 parts by weight, for example, 0.7 parts.
In a preferred embodiment, the silicone tube can be prepared by the following method:

S1, mixing the R- vinyl silicone rubber and the reinforcing agent to obtain mixture A;
S2, dividing the mixture A into component A1 and component A2, mixing the component A1 and hydrogen-containing silicone oil to obtain component B1, and mixing the component A2 and a catalyst to obtain component B2;
S3. mixing the component B1 and the component B2 to obtain mixture B, and carrying out catalytic addition to obtain the silicone tube.

In present disclosure, the silica can be pretreated using conventional methods in the field, the pretreatment is preferably drying at 130°C for 24 hours for standby.
In the present disclosure, the R- vinyl silicone rubber and the reinforcing agent are preferably pretreated before mixing. The pretreatment may be drying. The drying operation can be conventional in this field, such as drying the R- vinyl silicone rubber in an oven at 40°C for 12h, and then drying the reinforcing agent in an oven at 110°C for 12h.
In step S1, the mixing operation can be conventional in the field, such as kneading R-vinyl silicone rubber and reinforcing agent in a vacuum kneader to obtain a rubber compound, and then refining the rubber compound on an open mill. The reinforcing agent is preferably added in portions of 10 parts each time. The roll spacing of the open mill can be adjusted to perform thin-pass processing for the rubber compound, so that the reinforcing agent is uniformly dispersed.
In step S2, when the silicone tube with drug release holes further comprises an inhibitor, the inhibitor is mixed with component A1 and the hydrogen-containing silicone oil to obtain component B1.
In step S2, the preparation method of the component B1 can be refining the component A1 in an open mill, and adding the hydrogen-containing silicone oil and the inhibitor.
In step S2, the preparation method of the component B2 can be adding the component A2 into the catalyst drop by drop, continuously shortening the roll gap, and carrying out thin-pass processing.
In step S3, the mass ratio of component B1 to component B2 in mixture B can be 1:1.
In step S3, the preparation method of the mixture B can be that mixing and extruding the component B1 and the

component B2 in an open mill.

In step S3, the catalytic addition step can be subjecting the mixture B to a first sulfidation treatment and a second sulfidation treatment in sequence.

Wherein, the first sulfidation treatment can be carried out in a pre-drying tunnel. The temperature of the first sulfidation treatment can be 300°C. The time of the first sulfidation treatment can be 5s. The first sulfidation treatment can perform preliminary high-temperature shaping of the rubber compound.

Wherein, the second sulfidation treatment is preferably carried out in a post-drying tunnel. The temperature of the second sulfidation treatment can be 240°C. The time of the second sulfidation treatment can be 2min. The second sulfidation treatment can fully vulcanize the rubber compound.

In the present disclosure, the silicone tube with drug release holes are obtained by drilling holes in the silicone tube using laser drilling or mechanical drilling.

In the present disclosure, when the rod core of the perforated implant comprising a drug-containing layer, the perforated implant is a single-layer implant.

Wherein, when the concentration of the active drug in the drug-containing layer is a single concentration, the perforated implant is a single-layer single-concentration implant.

Wherein, when the concentration of the active drug in the drug-containing layer is gradient concentration, the perforated implant is a single-layer and multi-gradient implant. The gradient is preferably 1-50 segments, for example, 2 segments, or 3 segments, or 5 segments, or 10 segments, or 20 segments, or 40 segments.

In a preferred embodiment, the perforated implant is a single-layer and multi-gradient implant, the pore diameter of the drug release holes in the silicone tube with drug release holes is 350 $\mu$m, and the ratio of the pore diameter of the drug release holes to the outer diameter of the silicone tube is 0.35 mm: 2.4 mm, the number of the drug release holes is one, and the position of the drug release holes satisfies the following conditions: the distance from the center point of the drug release holes to the first end surface of the silicone tube with drug release holes is 50% of the length of the silicone tube, the multi-gradient is a three-segment design, sequentially configured as a first gradient drug release module, a second gradient drug release module, and a third gradient drug release module, the center point of the first gradient drug release module coincides with the center point of the drug release hole.

In a preferred embodiment, the perforated implant is a single-layer and multi-gradient implant, the pore diameter of the drug release holes in the silicone tube with drug release holes is 350 $\mu$m, and the ratio of the pore diameter to the outer diameter of the silicone tube is 0.35 mm:2.4 mm, the number of the drug release holes is two, and the positions of the drug release holes meet the following conditions: the two drug release holes are not on the same radial section of the silicone tube; in the axial section direction of the silicone tube, the distance from the center point of any drug release hole to the first end surface of the silicone tube with drug release holes is 50% of the length of the silicone tube; and the distance between the center points of the two drug release holes is 0.3 cm, the multi-gradient can be three-segmented, sequentially configured as a first gradient drug release module, a second gradient drug release module, and a third gradient drug release module, the center points of the first gradient drug release module and the second gradient drug release module coincide with the center points of the two drug release holes, respectively.

In a preferred embodiment, the perforated implant is a single-layer and multi-gradient implant, the pore diameter of the drug release holes in the silicone tube with drug release holes is 350 $\mu$m, and the ratio of the pore diameter to the outer diameter of the silicone tube is 0.35 mm:2.4 mm, the number of the drug release holes is three, and the positions of the drug release holes meet the following conditions: the three drug release holes are not on the same radial section of the silicone tube; in the axial section direction of the silicone tube, the distance from the center point of any drug release hole to the first end surface of the silicone tube with drug release hole is 50% of the length of the silicone tube; and the distance between the center points of adjacent drug release holes is 0.3 cm, the multi-gradient can be three-segmented, sequentially configured as a first gradient drug release module, a second gradient drug release module, and a third gradient drug release module, the center points of the first gradient drug release module, the second gradient drug release module, and the third gradient drug release module coincide with the center points of the three drug release holes, respectively.

In the present disclosure, when the rod core of the perforated implant further comprises a booster layer compounded on the drug-containing layer, the perforated implant is a double-layer implant.

Wherein, preferably, the plane where the drug-containing layer and the booster layer are located is perpendicular to the radial plane of the silicone tube.

Wherein, preferably, when the number of the drug release holes is one, the center point of the drug-containing layer and the center point of the drug release holes coincide.

Wherein, preferably, when the number of the drug release holes is more than one, the positional relationship between the center point of the drug-containing layer and the center point of the drug release holes can be selected according to the requirement of the release amount of the active drug.

The present disclosure also provides a preparation method of the perforated implant as described above, which comprises the following steps: filling the pharmaceutical composition or the rod core into the silicone tube with drug release

holes.

The present disclosure also provides a use of the rod core or the perforated implant as a release rate regulating medium in a sustained and controlled release formulation.

Based on the common sense in the field, the preferred conditions of the preparation methods can be combined arbitrarily to obtain preferred examples of the present disclosure.

The reagents and raw materials used in the present disclosure are commercially available.

The positive progress of the present disclosure is as follows:

The silicone tube made of the silicone material in the present disclosure has excellent mechanical properties, and the permeability of water to the silicone tube is increased, so that during application, the drug can be more easily released from the implant, and the release amount of the drug is improved. The drug release can reach several times to tens of times that of the traditional silicone tube, thus broadening the optional range of drugs and greatly expanding the application range of implants.

In the present disclosure, the solid column compression molding method is adopted to compress and mold the active drug and excipients to prepare the rod core, which not only solves the phenomena of drug waste or unstable drug release of the powder-type drug core, but also solves the problem of unstable drug during the preparation process of the silicone matrix-type rod core. After the silicone tube is drilled, the applicable range of drugs is expanded, including conventional drugs in the field, such as macromolecular drugs with good solubility, small molecular drugs with good solubility, insoluble macromolecular drugs and insoluble small molecular drugs, thereby increasing the drug release amount.

## BRIEF DESCRIPTION OF THE DRAWINGS

FIG.1 shows the in vitro release of modified pregnadienone reservoir skeleton implants with different wall thicknesses prepared in Examples 8-1, 8-2 and 8-3.

FIG. 2 shows the in vitro release of the modified pregnadienone reservoir skeleton implants prepared with different amounts of silica in Examples 8-1, 9-1, and 9-2.

FIG. 3 shows the in vitro release of the modified pregnadienone reservoir skeleton implants prepared with methyl vinyl polysiloxanes with different vinyl contents in Examples 8-1 and 10.

FIG. 4 shows the in vitro release of the modified pregnadienone reservoir skeleton implants prepared in Examples 8-1, 11-1, and 11-2 using different molar ratios of Si-H in hydrogen-containing silicone oils to vinyl groups in methyl vinyl polysiloxanes.

FIG. 5 shows the in vitro release data of the modified paliperidone reservoir implants prepared with different contents of hydroxyapatite in Examples 12-1, 12-2 and 12-3.

FIG. 6 shows the in vitro release of the modified pregnadienone reservoir skeleton implants prepared with methyl vinyl polysiloxanes with different vinyl contents in Examples 12-1 and 13.

FIG. 7 shows the in vitro release of the modified pregnadienone reservoir skeleton implants prepared in Examples 12-1, 14-1, and 14-2 using different molar ratios of Si-H in hydrogen-containing silicone oils to vinyl groups in methyl vinyl polysiloxanes.

FIG. 8 is a flowchart of the silicone tube preparation process.

FIG. 9 is a schematic diagram of the perforated implants in Examples 30-33.

FIG. 10 is a schematic diagram of the perforated implants in Examples 20-23.

FIG. 11 is a schematic diagram of the perforated implants in Examples 34.

FIG. 12 is a schematic diagram of the perforated implants in Examples 24-1 and 25-1.

FIG. 13 is a schematic diagram of the perforated implants in Examples 24-2 and 25-2.

FIG. 14 is a schematic diagram of the perforated implants in Examples 24-3 and 25-3.

FIG. 15 is a schematic diagram of the perforated implants in Examples 26-2, 27-2, 28-2 and 29-2.

Reference numerals are explained as follows;
1-Drug release hole; 2-Silicone tube; 3-Length of silicone tube; 4-Outer diameter of silicone tube; 5-Diameter of drug release hole; 6-Distance from the center point of drug release hole to the end point of silicone tube; 7-Distance between the center points of adjacent drug release holes; 8-End sealing adhesive; 9-Drug-containing layer; 901-First drug release module; 902-Second drug release module; 903-Third drug release module; 10-Direction of drug release; 11-Boosting layer.

DETAILED DESCRIPTION OF THE PREFERRED EMBODIMENT

The present disclosure will be further illustrated by way of examples below, but the present disclosure is not limited to the scope of the examples described herein. For experimental methods in the following examples where specific conditions are not specified, follow conventional methods and conditions, or select according to the product instructions.

The sources of each component in the examples and comparative examples are shown in the table below.

| Components | Commercial source |
|---|---|
| Methyl vinyl polysiloxane, 100,000-800,000 g/mol | Dongjue Silicone Group Co., Limited |
| Hydrogen-containing silicone oil (Si-H group content is 0.75 mol%) | Guangdong Guiye New Material Technology Co., Ltd. |
| Hydrophilic fumed silica N20 (contact angle 14-90°) | Germany Wacker International Group Co., Ltd. |
| Hydrophilic silica TH-a200 (contact angle <90°) | Shandong Wanhua Tianhe New Materials Co., Ltd. |
| Hydrophilic fumed silica A380 (contact angle 16-35°) | Evonik Degussa GmbH |
| Hydrophobic fumed silica R106 (contact angle >90°) | Evonik Degussa GmbH |
| HB-615 hydrophobic fumed silica (contact angle >138°) | Dalian Shengsen Nanomaterials Co., Ltd. |
| Hydroxyapatite (contact angle 0-25°) | Shanghai Aladdin Biochemical Technology Co., Ltd. |
| Platinum catalyst (3000ppm) | Guangdong Guiye New Material Technology Co., Ltd. |
| 2-Methyl-3-butyn-2-ol | Jiuding Chemical Technology Co., Ltd. |
| Paliperidone | Shanghai Aladdin Biochemical Technology Co., Ltd. |
| Pregnadienone (purity 98%, batch number: 190327) | Hebei Mokai Technology Co., Ltd. |
| Estradiol (99% purity, batch number: 190327) | Hebei Mokai Technology Co., Ltd. |
| KN-300N Adhesive | Kanglibang Polymer New Materials Co., Ltd. |
| Anhydrous ethanol (analytical grade) | Shandong Yuwang Chemical Reagent Co., Ltd. |
| Chromatography Methanol | Shandong Yuwang Chemical Reagent Co., Ltd. |
| Petroleum ether (boiling range 60-90°C) | Shandong Yuwang Chemical Reagent Co., Ltd. |
| Semaglutide (purity 98%, batch number P 21090311384) Mw = 4113.58, solubility 70 mg/ml | Nanjing Peptide Valley Biotechnology Co., Ltd. |
| Exenatide (purity 98%, batch number P 22100814242), Mw = 3369.757, solubility 50 mg/ml | Nanjing Peptide Valley Biotechnology Co., Ltd. |
| Bovine insulin (purity 98%, batch number N2210120028), Mw = 5733, practically insoluble in water. | Nanjing Peptide Valley Biotechnology Co., Ltd. |
| Levothyroxine sodium (purity 98%, batch number 220301) Mw = 800.87, solubility 0.15 mg/ml | Shanghai Meilian Biotechnology Co., Ltd. |
| Paliperidone (98% purity) Mw = 426.48, 0.05 mg/ml | Hubei Weideli Chemical Reagent Co., Ltd. |
| Estradiol (98% purity) Mw = 272.382, practically insoluble in water. | Hebei Mokai Technology Co., Ltd. |
| Metformin hydrochloride (98% purity) Mw = 165.63, 50 mg/ml | Hebei Mokai Technology Co., Ltd. |
| Chromatography Acetonitrile | Shandong Yuwang Chemical Reagent Co., Ltd. |
| Anhydrous sodium sulfate | Tianjin Hengxing Chemical Reagent Manufacturing Co., Ltd. |
| Phosphoric acid | Tianjin Kemio Chemical Reagent Co., Ltd. |

(continued)

| Components | Commercial source |
|---|---|
| Ethanolamine | Tianjin Hengxing Chemical Reagent Manufacturing Co., Ltd. |
| HPMC K4M, Mw400,000 | Shanghai Colorcon Coating Technology Co., Ltd. |
| Trehalose | Macklin Reagents |
| MCC, Mw324.28 | Anhui Sunhere Pharmaceutical Excipients Co., Ltd. |
| PEO N-80, Mw 200,000 | Shanghai Colorcon Coating Technology Co., Ltd. |
| UTEC S100, Mw5000 | Shanghai Colorcon Coating Technology Co., Ltd. |
| Calcium alginate | Macklin Reagents |
| Ethyl fiber, Mw448.5 | Shanghai Colorcon Coating Technology Co., Ltd. |
| PLGA, Mw10,000 | Shandong Daigang Bioengineering Co., Ltd. |
| HPMC K15M, Mw10,000 | Shanghai Colorcon Coating Technology Co., Ltd. |
| PEO N-750, Mw300,000 | Shanghai Colorcon Coating Technology Co., Ltd. |
| PEOWSR 303, Mw7,000,000 | Shanghai Colorcon Coating Technology Co., Ltd. |
| PVP K30, MW 44,000 | BASF Corporation |
| PEOWSR 1105, Mw900,000 | Shanghai Colorcon Coating Technology Co., Ltd. |
| PEOWSR N-12K, Mw1,000,000 | Shanghai Colorcon Coating Technology Co., Ltd. |
| PEOWSR N-60K, Mw2,000,000 | Shanghai Colorcon Coating Technology Co., Ltd. |
| Methyl vinyl silicone rubber | Dongjue Silicone Group Co., Limited |

The equipment and instruments used in the preparation and testing process are shown in the table below:

| | |
|---|---|
| Vacuum kneader (small) | Laizhou Zhongtai Chemical Machinery Co., Ltd. |
| Precision open mill (small) | Dongguan Xilong Electrical Machinery Equipment Co., Ltd. |
| Diameter measuring and control instrument (3020N) | Shanghai Pinzhong Testing Equipment Co., Ltd. |
| Fourier transform infrared tablet press (FW-4A model) | Tianjin Tianguang Optical Instruments Co., Ltd. |
| Silicone extruder (Φ16) | Hebei Xulang Machinery Equipment Manufacturing Co., Ltd. |
| Electronic analytical balance (XS105 model) | Mettler-Toledo Instruments Co., Ltd. |
| Electronic analytical balance (SMD200-2) | OHAUS International Trading Co., Ltd. |
| Electric heating forced-air drying oven (Type 101-2AB) | Tianjin Test Instrument Co., Ltd. |
| Intelligent Measurement System Microscope (V3.0) | Shenzhen Zhongwei Kechuang Co., Ltd. |
| Electronic tensile testing machine (QL-5E) | Xiamen Qunlong Instruments Co., Ltd. |
| Hot air sulfidation channel (vertical) | Hebei Xulang Machinery Equipment Manufacturing Co., Ltd. |
| Hot air sulfidation channel (horizontal) | Hebei Xulang Machinery Equipment Manufacturing Co., Ltd. |
| Fourier transform infrared spectrometer (Bruker Vertex 70) | Bruker Optics Inc. |
| LC-10ATVP High Performance Liquid Chromatograph | Shimadzu Corporation |
| SMD200-2 Electronic Analytical Balance | OHAUS International Trading Co., Ltd. |

(continued)

| | |
|---|---|
| AG245 Ultra-micro Electronic Balance - Electronic Analytical Balance | Mettler Toledo, Switzerland |
| KQ-250DE CNC Ultrasonic Cleaner | Kunshan Ultrasonic Instruments Co., Ltd. |
| CHA-S Thermostatic Oscillator | Changzhou Guohua Electric Co., Ltd. |
| SDC350 Contact Angle Meter | Dongguan Shengding Precision Instruments Co., Ltd. |
| UV-9000 Ultraviolet Spectrophotometer | Shanghai Yuanxi Instruments Co., Ltd. |
| TS-100 Type B Thermostatic Oscillator | Shanghai Jiecheng Experimental Instruments Co., Ltd. |

1. Silicone tube

1.1 Preparation method of silicone tube

(1) The methyl vinyl polysiloxane raw rubber was dried at 40 °C for 12 hours for later use; the hydrophilic fumed silica was dried at 130°C for 24h;
(2) the methyl vinyl polysiloxane raw rubber and hydrophilic fumed silica was mixed according to the prescribed amount to obtain a mixture A;
(3) the mixture A was divided into a component A1 and a component A2, component A1 was refined in an open mill with a roller distance parameter of 1 unit; the prescribed amount of the hydrogen-containing silicone oil and the inhibitor 2-methyl-3-butyn-2-ol were added dropwise thereto, after addition, the roller distance was set to different values, and the mixture was subjected to triangular packing, thin-pass processing, taken out and allowed to stand for 24 h to obtain component B1 for later use; component A2 was refined in an open mill with a roller distance parameter of 1 unit; the prescribed amount of platinum catalyst was added dropwise thereto, the roller distance was continuously shortened, and the mixture was subjected to thin-pass processing, taken out and allowed to stand for 24 h to obtain component B2 for later use;
(4) B1 and B2 prepared in step (3) with the ratio of 1:1 were placed in an open mill, mixed and extruded, and subjected to thin-pass processing for 4-6 times to obtain a mixture B;
(5) the mixture B prepared in the step (4) in a thin sheet form was unloaded from the open mill and cut into strips; a mold with a suitable size was installed on the extruder, the screw speed of the extruder was set, the cut silicone rubber strips were put into the extruder, and the rubber material was continuously extruded into a silicone tube through the die opening under the push of the screw rotation. The outer diameter of the silicone tube was determined by the extruder screw speed, the post-drying tunnel conveyor belt speed, and the die size, while the wall thickness was determined by the die size.
(6) the first sulfidation treatment and second sulfidation treatment were carried out on the silicone tube prepared in the step (5); so as to obtain the finished product of the silicone tube;

wherein, the first sulfidation treatment: the silicone tube extruded from the die opening first passed through a pre-drying tunnel (vertical hot air sulfidation channel) for rapid high-temperature sulfidation, which was the first sulfidation treatment, so that the silicone tube quickly changed from a sticky state to an elastic state and was preliminarily shaped.
wherein, the second sulfidation treatment: after the first sulfidation, it enterd the post-drying tunnel (horizontal hot air sulfidation channel) to continue sulfidation, which was the second sulfidation treatment, so that the sulfidation reaction of the silicone tube was more sufficient and reached the best cross-linking state, and then the finished silicone tube was continuously transported out by the conveyor belt of the post-drying tunnel.

1.2 Investigation of the Formulation and Process of Silicone Tubes

(1) The effects of vinyl content in methyl vinyl polysiloxane, the amount of reinforcing agent and the molar ratio of Si-H in hydrogen-containing silicone oil to vinyl in methyl vinyl polysiloxane on the mechanical properties of silicone tube.
A. Formulation: The concentration of platinum catalyst was 3000 ppm; the amount of platinum catalyst was 0.0098 PHR; the amount of inhibitor 2-methyl-3-butyn-2-ol was 0.69 PHR; other formulation parameters are shown in Table 1.

Table 1

| Serial number | Vinyl content in methyl vinyl polysiloxane | Hydrophilic silica TH-a200 | the Molar ratio of Si-H in hydrogen-containing silicone oil to vinyl groups in methyl vinyl polysiloxane |
|---|---|---|---|
| Example 1-1 | **0.18%** | 40PHR | 1.5:1 |
| Example 1-2 | **0.23%** | 40PHR | 1.5:1 |
| Example 2-1 | 0.18% | **30PHR** | 1.2:1 |
| Example 2-2 | 0.18% | **40PHR** | 1.2:1 |
| Example 2-3 | 0.18% | **45PHR** | 1.2:1 |
| Example 2-4 | 0.18% | **50PHR** | 1.2:1 |
| Example 3-1 | 0.18% | 30PHR | **1.5:1** |
| Example 3-2 | 0.18% | 30PHR | **1.8:1** |

B. Preparation method: The preparation method described in 1.1 was adopted, and the specific process parameters were as follows: the temperature of the first sulfidation treatment was 250°C; the time of the first sulfidation treatment was 3s; the temperature of the second sulfidation treatment was 240°C; the time of the second sulfidation treatment was 4min; the diameter of the die was 3mm; the diameter of the core mold was 2.6mm; the wall thickness of the obtained silicone tube was 0.2mm; and the screw speed was 5 $r \cdot min^{-1}$.

(2) The effect of the temperature of the second sulfidation treatment on the mechanical properties of silicone tube

A. Formulation: The concentration of platinum catalyst was 3000 ppm; the amount of platinum catalyst was 0.0098 PHR; the amount of inhibitor 2-methyl-3-butyn-2-ol was 0.69 PHR; the vinyl content in methyl vinyl polysiloxane was 0.18%; the amount of hydrophilic silica TH-a200 was 30 PHR; the molar ratio of Si-H in the hydrogen-containing silicone oil to vinyl in methyl vinyl polysiloxane was 1.5:1.

B. Preparation method: The preparation method described in 1.1 was adopted, except that the sulfidation temperature was set as recorded in Table 2; the first sulfidation time was 3s, the second sulfidation time was 4min; the diameter of the die was 3mm; the diameter of the core mold was 2.6mm; the wall thickness of the obtained silicone tube was 0.2mm; and the screw speed was 5 $r \cdot min^{-1}$.

Table 2

| Serial number | First sulfidation temperature /°C | Second sulfidation temperature /°C |
|---|---|---|
| Example 4-1 | 250 | **220** |
| Example 4-2 | 250 | **240** |
| Example 4-3 | 250 | **260** |
| Example 4-4 | 250 | **280** |

(3) The effect of mold size and screw speed on the outer diameter of silicone tube

A. Formulation: The concentration of platinum catalyst was 3000 ppm; the amount of platinum catalyst was 0.0098 PHR; the amount of inhibitor 2-methyl-3-butyn-2-ol was 0.69 PHR; the vinyl content in methyl vinyl polysiloxane was 0.18%; the amount of hydrophilic silica TH-a200 was 30 PHR; the molar ratio of Si-H in the hydrogen-containing silicone oil to vinyl in methyl vinyl polysiloxane was 1.5:1.

B. Preparation process: The preparation method described in 1.1 was adopted, the temperature of the first sulfidation treatment was 250°C; the time of the first sulfidation treatment was 3s; the temperature of the second sulfidation treatment was 240°C; the time of the second sulfidation treatment was 4min; the mold size and screw speed are shown in Table 3. (Note: Ring width = (die inner diameter - core mold diameter) / 2)

Table 3

| Serial number | Die inner diameter / mm | Core mold diameter / mm | Ring width/mm | Screw speed / r/min | Silicone tube outer diameter/mm |
|---|---|---|---|---|---|
| Example 5-1 | 3.30 | 2.50 | 0.40 | 5.0 | 2.23 |
| Example 5-2 | 3.30 | 2.50 | 0.40 | 6.0 | 2.30 |

(continued)

| Serial number | Die inner diameter / mm | Core mold diameter / mm | Ring width/mm | Screw speed / r/min | Silicone tube outer diameter/mm |
|---|---|---|---|---|---|
| Example 5-3 | 3.30 | 2.50 | 0.40 | 7.0 | 2.35 |
| Example 5-4 | 3.30 | 2.50 | 0.40 | 8.0 | 2.45 |
| Example 5-5 | 3.30 | 2.50 | 0.40 | 9.0 | 2.61 |
| Example 5-6 | 3.30 | 2.50 | 0.40 | 10.0 | 2.74 |
| Example 6-1 | 3.90 | 3.00 | 0.45 | 5.0 | 2.32 |
| Example 6-2 | 3.90 | 3.00 | 0.45 | 6.0 | 2.37 |
| Example 6-3 | 3.90 | 3.00 | 0.45 | 7.0 | 2.40 |
| Example 6-4 | 3.90 | 3.00 | 0.45 | 8.0 | 2.52 |
| Example 6-5 | 3.90 | 3.00 | 0.45 | 9.0 | 2.66 |
| Example 6-6 | 3.90 | 3.00 | 0.45 | 10.0 | 2.79 |
| Example 7-1 | 3.50 | 2.50 | 0.50 | 5.0 | 2.47 |
| Example 7-2 | 3.50 | 2.50 | 0.50 | 6.0 | 2.55 |
| Example 7-3 | 3.50 | 2.50 | 0.50 | 7.0 | 2.62 |
| Example 7-4 | 3.50 | 2.50 | 0.50 | 8.0 | 2.67 |
| Example 7-5 | 3.50 | 2.50 | 0.50 | 9.0 | 2.71 |
| Example 7-6 | 3.50 | 2.50 | 0.50 | 10.0 | 2.78 |

The results in Table 3 show that when the screw speed reaches 5 $r \cdot min^{-1}$, the rubber compound can be continuously and uniformly conveyed. With a constant screw speed, the greater the ring width between the die inner diameter and core mold diameter, the greater the wall thickness of the silicone tube. The outer diameter of the silicone tube is not directly related to the ring width; it is influenced by the combined effects of the die and core mold dimensions, the ring width, and the screw speed.

When using the same mold, the higher the screw speed, the larger the outer diameter of the silicone tube, this is because when the screw speed is higher, the silicone tube is extruded at a higher speed, and the stretching effect of the conveyor belt on the silicone tube is smaller, so the outer diameter is larger. The lower the screw speed, the lower the extrusion speed of the silicone tube, the greater the stretching effect of the conveyor belt on the silicone tube, and the smaller the outer diameter of the silicone tube.

In summary, the outer diameter of silicone tubes is greatly affected by the screw speed, silicone tubes with different outer diameters can be prepared by using molds of different sizes and corresponding screw speeds, laying the foundation for subsequent research on the preparation of implants of different specifications and their drug release.

2. Improved Gestodene Depot Matrix Implant

2.1 Preparation method of implant

(1) The silicone tube is prepared according to steps (1)-(6) of the preparation method in 1.1;

(2) Basic formulation and preparation process of the drug core: gestodene, with a particle size of 2-3 μm, added in an amount of 100 PHR; methyl vinyl polysiloxane, with a vinyl content of 0.18%-0.23%; hydrogen-containing silicone oil, with a Si-H group content of 0.75%, added in an amount of 0.3-5.0 PHR; platinum catalyst, with a platinum concentration of 10000 ppm, added in an amount of 0.000002-0.5 PHR; 2-methyl-3-butyn-2-ol as an inhibitor, added in an amount of 0.03-2.0 PHR;

the sulfidation temperature was 90°C; the sulfidation time was 1 h; the diameter of the drug core was 0.2 cm and the length was 1.5 cm.

(3) The silicone tube was immersed in petroleum ether for 1-2 min, the residual petroleum ether on the surface of the silicone tube was quickly flicked off by hand. The precut drug core was immediately and rapidly inserted into the middle

of the silicone tube using tweezers, leaving equal lengths of space at both ends of the silicone tube for sealing. After assembly, the implant was allowed to air-dry naturally for 12-24 h to completely volatilize the residual petroleum ether, then both ends of the air-dried implant were sealed with sealing adhesive, and placed at room temperature for 12-24 h to cure the adhesive, thereby obtaining the improved gestodene depot matrix implant.

2.2 The effect of preparation process or formulation on the performance of silicone tube

(1) The effects of vinyl content in methyl vinyl polysiloxane, the amount of reinforcing agent, the molar ratio of Si-H in hydrogen-containing silicone oil to vinyl in methyl vinyl polysiloxane and the wall thickness of silicone tube on the release of silicone tube in vitro.

A. Formulation: The concentration of platinum catalyst was 10,000 ppm; the amount of platinum catalyst was 0.01 PHR; the amount of inhibitor 2-methyl-3-butyn-2-ol was 0.69 PHR; other formulation parameters are shown in Table 1.

Table 4

| Serial number | Vinyl content | Amount of hydrophilic fumed silica A380 | Molar ratio of hydrogen-containing silicone oil | Wall thickness of silicone tube /mm |
|---|---|---|---|---|
| Example 8-1 | 0.18% | 30PHR | 1.5:1 | 0.2 |
| Example 8-2 | 0.18% | 30PHR | 1.5:1 | 0.3 |
| Example 8-3 | 0.18% | 30PHR | 1.5:1 | 0.4 |
| Example 9-1 | 0.18% | 20PHR | 1.5:1 | 0.2 |
| Example 9-2 | 0.18% | 40PHR | 1.5:1 | 0.2 |
| Example 10 | 0.23% | 30PHR | 1.5:1 | 0.2 |
| Example 11-1 | 0.18% | 30PHR | 1.2:1 | 0.2 |
| Example 11-2 | 0.18% | 30PHR | 1.8:1 | 0.2 |

B. The preparation method described in 2.1 was adopted, and the specific process parameters were as follows: the temperature of the first sulfidation treatment was 250°C; the time of the first sulfidation treatment was 3s; the temperature of the second sulfidation treatment was 240°C; the time of the second sulfidation treatment was 4min; the diameter of the core was fixed at 0.2 cm and the length at 1.5 cm; the outer diameter of the silicone tube was fixed at 2.4 mm and the length at 1.9 cm.

3. Improved Paliperidone Depot Implant

3.1 Preparation method of implants

The silicone tube was prepared according to steps (1)-(6) of the preparation method in 1.1, and then paliperidone depot implant was prepared through the following steps (7);

(7) Preparation of paliperidone depot implant: One end was sealed with KN-300N adhesive and cured for 24 hours. The drug was then filled through the filling funnel. After filling, the other end was sealed with KN-300N adhesive and cured for 24 hours. After curing, the implant was inspected for drug leakage. After confirming that there was no leakage, it was washed repeatedly with anhydrous ethanol for 30 s. The preparation was thus completed, yielding the improved paliperidone depot implant.

3.2 The effects of amount of hydroxyapatite, vinyl content of methyl vinyl polysiloxane, and amount of hydrosilicone oil on in vitro drug release

A. Formulation: Paliperidone was added at 100 PHR; HB-615 hydrophobic fumed silica was added at 20 PHR; platinum catalyst concentration was 3000 ppm; platinum catalyst dosage was 0.21 PHR; inhibitor 2-methyl-3-butyn-2-ol dosage was 0.98 PHR; other formulation parameters are shown in Table 5.

Table 5

| Serial number | Vinyl content | Hydroxylapatite | Molar ratio of hydrogen-containing silicone oil |
|---|---|---|---|
| Example 12-1 | 0.18% | 20PHR | 1.2:1 |
| Example 12-2 | 0.18% | 30PHR | 1.2:1 |
| Example 12-3 | 0.18% | 40PHR | 1.2:1 |
| Example 13 | 0.23% | 20PHR | 1.2:1 |
| Example 14-1 | 0.18% | 20PHR | 1.5:1 |
| Example 14-2 | 0.18% | 20PHR | 1.8:1 |

B. Preparation method: The preparation method described in 2.1 was adopted, and the specific process parameters were as follows: the temperature of the first sulfidation treatment was 250°C; the time of the first sulfidation treatment was 5s; the temperature of the second sulfidation treatment was 240°C; the time of the second sulfidation treatment was 2min; the prepared implant had a wall thickness of 0.2 mm, an outer diameter of 2.2-2.3 mm, and a drug release area of 2.7 ± 0.5 $cm^2$.

4. Improved Estradiol Depot Implant

4.1 Preparation method of implants

Same as the method for preparing the implants described in 3.1

4.2 The effects of sulfidation temperature, silicone tube wall thickness, and drug release area on in vitro release of implants

A. Formulation: As shown in the table below:

Table 6

| Material name | Dosage |
|---|---|
| Methyl vinyl polysiloxane | 100 PHR |
| Vinyl content in methyl vinyl polysiloxane | 0.17% |
| Hydrophilic fumed silica N20 | 20PHR |
| Hydrophobic fumed silica R106 | 20PHR |
| Molar ratio of Si-H in hydrogen-containing silicone oil to vinyl groups in methyl vinyl polysiloxane | 1.2:1 |
| Content of Si-H group in hydrogen-containing silicone oil | 0.75% |
| 2-Methyl-3-butyn-2-ol | 0.7 PHR |
| Platinum catalyst (3000ppm) | 0.00001 PHR |

B. Preparation process: The time of the first sulfidation treatment was 5 s; the time of the second sulfidation treatment was 2 min; the time of the third sulfidation treatment was 48 h; other parameters are shown in the table below:

Table 7

| Serial number | First sulfidation temperature / °C | Second sulfidation temperature / °C | Third sulfidation temperature / °C |
|---|---|---|---|
| Example 17-1 | 250 | 220 | 180 |
| Example 17-2 | 250 | 240 | 180 |
| Example 17-3 | 250 | 260 | 180 |

Table 8

| Serial number | Outer diameter of silicone tube /mm | Wall thickness of silicone tube /mm | Drug-containing length/cm |
|---|---|---|---|
| Example 18-1 | 2.2 | 0.2 | 4 |
| Example 18-2 | 2.2 | 0.4 | 4 |
| Example 18-3 | 2.2 | 0.6 | 4 |

Table 9

| Serial number | Outer diameter of silicone tube /mm | Wall thickness of silicone tube /mm | Drug release area/$cm^2$ |
|---|---|---|---|
| Example 19-1 | 2.2 | 0.2 | 0.69 |
| Example 19-2 | 2.2 | 0.2 | 1.38 |
| Example 19-3 | 2.2 | 0.2 | 2.07 |
| Example 19-4 | 2.2 | 0.2 | 2.76 |
| Example 19-5 | 2.2 | 0.2 | 3.45 |

Comparative Example 1: Gestodene Depot Matrix Implant

A. Formulation: Compared with the formulation of the improved gestodene depot matrix implant, the difference lies in the fact that the reinforcing agent was only HB-615 hydrophobic fumed silica.
B. Preparation process: The preparation method is the same as described in 2.1.

Comparative Example 2: Paliperidone Depot Implant

A. Formulation: Compared with the formulation of the improved paliperidone depot implant, the difference lies in the fact that the reinforcing agent was only HB-615 hydrophobic fumed silica, and did not contain hydroxyapatite.
B. Preparation process: The preparation method is the same as described in 3.1.

Comparative Example 3: Estradiol Depot Implant

A. Formulation: Compared with the formulation of the modified estradiol depot implant, the difference lies in the fact that the reinforcing agent was only hydrophobic fumed silica R106, and did not contain N20 hydrophilic silica.
B. Preparation process: The preparation method is the same as described in 4.1.

Effect Example

The mechanical properties and in vitro release performance of the examples and comparative examples of the present disclosure were studied.

I. Mechanical Properties

The subcutaneous implant shall comply with the national standard GB/T 528 (ISO 37) "Rubber, vulcanized or thermoplastic - Determination of tensile stress-strain properties", so as to possess favorable mechanical properties and ensure that its shape remains unchanged during implantation and removal.

1. Elongation at break (%): The elongation of the sample at tensile fracture.
2. Tensile strength (MPa): The maximum tensile stress a sample endured by the sample until fracture during a tensile

test.
3. Tear strength (KN•m-1): The strength required for the sample to be torn.

Specific testing method: Samples of about 5 cm in length were prepared, with three parallel samples for each type. The samples were tested using a tensile tester with the test parameters as follows: grip distance 50 mm, tensile speed 200 mm•min$^{-1}$. After the sample test was completed, the data were recorded.

Table 10

| | Elongation at break /% | Tensile strength / MPa | Tear strength / kN•m$^{-1}$ |
|---|---|---|---|
| Example 1-1 | 131.0 | 2.54 | 16.90 |
| Example 1-2 | 163.7 | 3.06 | 23.17 |
| Example 2-1 | 151.5 | 1.79 | 10.98 |
| Example 2-2 | 185.6 | 2.25 | 13.74 |
| Example 2-3 | 194.8 | 2.68 | 13.80 |
| Example 2-4 | 167.9 | 2.03 | 9.96 |
| Example 3-1 | 211.7 | 3.87 | 26.55 |
| Example 3-2 | 210.8 | 3.79 | 26.07 |
| Example 4-1 | 187.4 | 3.19 | 23.94 |
| Example 4-2 | 223.7 | 3.75 | 27.87 |
| Example 4-3 | 148.7 | 2.88 | 19.56 |
| Example 4-4 | 100.9 | 1.97 | 11.33 |

According to the effect data of Examples 1-1 and 1-2, when the vinyl content is 0.18% and 0.23%, the elongation at break, tensile strength and tear strength of the vulcanized silicone tube are not significantly different, and all show good mechanical properties. This indicates that the vinyl content of both of the above is within a suitable range, and the crosslinked segments in the network structure formed after the addition reaction have appropriate length and the crosslinking points are distributed in an orderly manner. Therefore, the prepared silicone tube has good elasticity and certain tensile and tear resistance, which meets the requirements for preparing implants and clinical applications.
According to the effect data of Examples 2-1 to 2-4, when the amount of hydrophilic silica is in the range of 30-50 PHR, the mechanical properties of the silicone tubes after sulfidation treatment are good.
According to the effect data of Examples 2-1, 3-1 and 3-2, it can be seen that when the molar ratio of Si-H in hydrogen-containing silicone oil to vinyl in methyl vinyl polysiloxane increases from 1.2:1 to 1.5:1, the tensile strength and tear strength of silicone tubes are enhanced, when the molar ratio increases from 1.5 to 1.8, the elongation at break, tensile strength and tear strength of the sulfidation treatment silicone tubes are not significantly different, and the mechanical properties of the silicone tubes are all good. During the addition reaction, the hydrogen in the hydrogen-containing silicone oil and the vinyl group in the methyl vinyl polysiloxane theoretically react in a molar ratio of 1:1. However, Si-H can be lost due to side reactions in actual reactions, therefore, a slight excess of hydrogen-containing silicone oil is usually used to ensure that the addition reaction proceeds fully, thus the crosslinking becomes more and more complete, and the mechanical properties of the silicone tube reach their optimal state.
According to the effect data of Examples 4-1 to 4-4, as the second sulfidation temperature increases, the elongation at break, tensile strength, and tear strength of the sulfidation treatment silicone tube show a trend of first increasing and then decreasing; when the second sulfidation treatment temperature is 240°C, the values of each index reach the maximum, the second sulfidation treatment is mainly to allow the silicone tube to continue sulfidation and the cross-linking reaction to continue, a suitable temperature can make the cross-linking reaction complete and the mechanical properties better, however, if the temperature is too high, it will cause the silicone tube to be over-vulcanized, and the internal cross-linking bonds will undergo thermal decomposition, resulting in a decrease in performance.

II. In vitro release effect

1. In vitro release characterization of improved gestodene depot matrix implant

1.1 Test Method

(1) Chromatographic conditions

Chromatographic column: Diamonsil® C18 column (4.6 mm × 250 mm, 5 μm);
Mobile phase: methanol: water (80:20, v/v);
Column temperature: 30°C;
Detection wavelength: 239nm;
Flow rate: 1.0 mL•min$^{-1}$;
Injection volume: 20 μL

(2) In vitro release experimental method

The release experiment used the horizontal shaking method, one implant was taken and fixed at both ends to the bottom and wall of a 50 mL stoppered conical flask with silicone adhesive (to prevent the implant from floating on the liquid surface and causing inaccurate release results), after adhesion, it was left to stand for 12 hours to allow the silicone adhesive to cure completely. 50 mL of distilled water as the release medium was accurately measured and injected into the conical flask, the conical flask was placed in a constant temperature air shaker and shaken at a temperature of 37°C and an amplitude of 100 r•min$^{-1}$, the medium was replaced with an equal volume every 24 h. The release solution was filtered through a 0.22 μm microporous membrane and analyzed under chromatographic conditions with an injection volume of 20 μL.

1.2 Results Data

(1) In vitro release effect data over 30 days

Examples 8-1 to 8-3 involve implants with different silicone tube wall thicknesses, the in vitro release data of them are shown in Fig. 1. It can be seen that the thicker the silicone tube wall, the lower the daily release of the implant, the average daily release of the implant with a silicone tube wall thickness of 0.2 mm over 30 days is about 55 μg; the average daily release of the implant with a wall thickness of 0.3 mm over 30 days is about 48 μg; and the average daily release of the implant with a wall thickness of 0.4 mm over 30 days is about 42 μg. That is, for every 0.1 mm increase in the thickness of the silicone tube, the average daily drug release decreases by about 5-8 μg. The data above shows that when the outer diameter and length of the silicone tube, as well as the diameter and length of the drug core are fixed, the wall thickness of the silicone tube can slightly affect the in vitro release rate of the implant. In the actual preparation of implants, when the outer diameter of the silicone tube remains constant, the thicker the wall, the smaller the inner diameter, making it more difficult to assemble the drug core and the silicone tube, however, the inner diameter of a silicone tube with a wall thickness of 0.2 mm is relatively large, which allows for better matching with the drug core.

Examples 8-1, 9-1, and 9-2 involve different amounts of hydrophilic silica, the in vitro release data are shown in Fig. 2. It can be seen that the amount of hydrophilic silica slightly affects the release of the implant, with increasing hydrophilic silica amount, the average daily release of the implanting agent over 30 days slightly increases. When the hydrophilic silica amount is 40 PHR, the daily release is relatively high, approximately 55 μg, which is significantly higher than those of 30 PHR and 20 PHR in the 1-15 days period, as shown in the release curves in Fig. 2. The average daily release of the hydrophilic silica amounts of 30 PHR and 20 PHR are not significantly different, both around 48 μg, furthermore, the fluctuations in both release curves are smaller than that in the 40 PHR curve, indicating better release stability. Moreover, during silicone tube extrusion, the extrusion stability and smoothness are better when the fumed silica amount is 30 PHR.

Examples 8-1 and 10 involve implants with different vinyl contents, the in vitro release data of them are shown in Fig. 3. It can be seen that the vinyl content of the silicone tube has little effect on the release of the implant, the average daily release of both groups of implants within 30 days is between 47-50 μg, the methyl vinyl polysiloxane with a vinyl content of 0.18% is more in line with the requirements of mechanical properties and cost.

Examples 8-1, 11-1, and 11-2 involve implants with different amounts of hydrogen-containing silicone oil, the in vitro release data of them are shown in Fig. 4. It can be seen that when the molar ratio of hydrogen-containing silicone oil is 1.2 and 1.8, the release of the implant is relatively high in the first 3 days, with a sudden release phenomenon in the early stage, and the overall release curve fluctuates greatly. However, the release curve fluctuates relatively less when the molar ratio is 1.5, it can be seen that the release curve at the molar ratio of 1.5 is relatively stable, and previous studies have shown that the mechanical properties of the silicone tube are also better.

(3) In vitro release experiment

Long-term in vitro release experiments were conducted on the implants of Examples 8-1 and Comparative Example 1 for 150 days, and the results are shown in Tables 11 and 12, respectively.

Table 11

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 47.11 | | | | |
| 5 | 46.23 | 55 | 38.35 | 105 | 34.24 |
| 10 | 49.78 | 60 | 36.81 | 110 | 35.34 |
| 15 | 42.43 | 65 | 36.37 | 115 | 35.29 |
| 20 | 43.85 | 70 | 37.26 | 120 | 33.20 |
| 25 | 43.09 | 75 | 36.71 | 125 | 35.03 |
| 30 | 39.45 | 80 | 37.49 | 130 | 33.83 |
| 35 | 39.17 | 85 | 36.67 | 135 | 31.05 |
| 40 | 39.69 | 90 | 36.84 | 140 | 33.68 |
| 45 | 37.54 | 95 | 35.04 | 145 | 33.25 |
| 50 | 39.71 | 100 | 34.37 | 150 | 33.78 |

Table 12

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 33.72 | | | | |
| 5 | 32.05 | 55 | 19.30 | 105 | 15.52 |
| 10 | 28.87 | 60 | 17.26 | 110 | 15.45 |
| 15 | 27.19 | 65 | 17.41 | 115 | 15.88 |
| 20 | 25.82 | 70 | 17.87 | 120 | 12.19 |
| 25 | 25.44 | 75 | 17.82 | 125 | 15.56 |
| 30 | 24.06 | 80 | 16.78 | 130 | 17.04 |
| 35 | 22.83 | 85 | 18.57 | 135 | 18.31 |
| 40 | 21.58 | 90 | 18.43 | 140 | 11.83 |
| 45 | 21.32 | 95 | 17.83 | 145 | 15.71 |
| 50 | 19.48 | 100 | 17.85 | 150 | 13.90 |

The data in the tables above show that, under the premise of meeting the mechanical properties of the implant, hydrophilic silica increased the release of silicone tubes by 18-23 μg compared with hydrophobic silica, indicating that the drug release of the implant meets the requirements and has good drug release stability, can achieve stable drug release for at least 5 months. Furthermore, compared with the gestodene matrix depot implant, the in vitro release of the improved gestodene matrix depot implant can be increased by 192%.

2. In vitro release characterization of improved paliperidone depot implant

2.1 Test Method

(1) Chromatographic conditions

Column: InfinityLab Poroshell HPH-C8 (4.6 × 100 mm, 2.7 μm)
Mobile phase: methanol: acetonitrile: phosphate 1:4:15 isocratic elution
Flow rate: 1 mL/min
Column temperature: 35°C
Injection volume: 20 μL
Detection wavelength: 280nm

(2) In vitro release experimental method

One vial of the improved paliperidone depot implant was taken and fixed in a 20 mL sample bottle using silicone adhesive, an appropriate amount of distilled water was accurately measured into the sample bottle to keep the formulation below the surface of the release medium, and the bottle was placed in a constant temperature shaker with an amplitude of 100 r•min-1 and a temperature of 37 °C for release. Sampling was performed every 24 h, the release medium was replaced, and the solution was filtered through a 0.22 μm filter membrane and the sample was retained for testing.

2.2 Results Data

(1) In vitro release effect data over 30 days

Examples 12-1 to 12-3 involve implants prepared with different amounts of hydroxyapatite, the in vitro release effect data of them are shown in Fig. 5, the in vitro release of the silicone tube after sulfidation increases with the increase of the amount of hydroxyapatite.

Examples 12-1 and 13 investigated the effect of the vinyl content of methyl vinyl polysiloxane on the in vitro release of silicone tubes, and the results are shown in Fig. 6. It can be seen that the vinyl content in the silicone tube had little effect on the release of the implant, the daily release amounts of the two groups of implants within 30 days were both between 20-25 μg, with little difference between them. However, when the vinyl content of the raw rubber in the silicone tube was 0.18%, the release curve of the implant was more stable overall.

Examples 12-1, 14-1, and 14-2 investigated the effect of the molar ratio of hydrogen-containing silicone oil on the in vitro release of silicone tubes, the results are shown in Fig. 7. It can be seen that the release curves of the implants showed little difference at different molar ratios of hydrogen-containing silicone oil. This is because, during the addition reaction, the hydrogen in the hydrogen-containing silicone oil and the vinyl group in the methyl vinyl polysiloxane theoretically participate in the reaction at a molar ratio of 1:1. However, Si-H can be lost due to side reactions in actual reactions, therefore, a slight excess of hydrogen-containing silicone oil is usually used to ensure that the addition reaction proceeds fully and achieves a more complete crosslinking.

(2) Long-term in vitro release results data

Long-term in vitro release experiments were conducted on Examples 14-1 and Comparative Example 2 at a temperature of 37°C and a shaking speed of 100 r•min$^{-1}$, the long-term in vitro release results of Example X are shown in Table 13 below. The long-term in vitro release results of Comparative Example 2 are shown in Table 14.

Table 13

| Time (d) | Daily release amount (μg·d$^{-1}$) | Time (d) | Daily release amount (μg·d$^{-1}$) | Time (d) | Daily release amount (μg·d$^{-1}$) |
|---|---|---|---|---|---|
| 1 | 33.16 | | | | |
| 5 | 27.02 | 55 | 16.71 | 105 | 16.31 |
| 10 | 18.74 | 60 | 16.44 | 110 | 17.72 |
| 15 | 16.38 | 65 | 16.72 | 115 | 17.22 |
| 20 | 16.67 | 70 | 16.20 | 120 | 16.51 |
| 25 | 16.24 | 75 | 17.15 | 125 | 17.29 |
| 30 | 16.53 | 80 | 17.77 | 130 | 17.30 |

(continued)

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 35 | 17.28 | 85 | 16.84 | 135 | 17.75 |
| 40 | 16.74 | 90 | 17.17 | 140 | 16.39 |
| 45 | 16.82 | 95 | 17.76 | 145 | 16.45 |
| 50 | 17.79 | 100 | 17.10 | 150 | 17.65 |

Table 14

| Time (d) | Daily release amount ($\mu g \cdot d^{-1}$) | Time (d) | Daily release amount ($\mu g \cdot d^{-1}$) | Time (d) | Daily release amount ($\mu g \cdot d^{-1}$) |
|---|---|---|---|---|---|
| 1 | 14.28 | | | | |
| 5 | 10.57 | 55 | 6.66 | 105 | 6.89 |
| 10 | 6.80 | 60 | 6.58 | 110 | 6.44 |
| 15 | 6.28 | 65 | 6.61 | 115 | 6.21 |
| 20 | 6.75 | 70 | 6.17 | 120 | 6.79 |
| 25 | 6.97 | 75 | 6.63 | 125 | 7.02 |
| 30 | 6.84 | 80 | 7.23 | 130 | 6.03 |
| 35 | 7.43 | 85 | 6.71 | 135 | 7.18 |
| 40 | 6.54 | 90 | 5.77 | 140 | 5.87 |
| 45 | 6.70 | 95 | 7.24 | 145 | 5.99 |
| 50 | 5.86 | 100 | 5.94 | 150 | 6.30 |

According to the effect data in the table above, the in vitro release study of the improved paliperidone depot implant obtained by the silicone tube provided by the present disclosure shows that the drug release is stable within 150 days, compared with the paliperidone depot implant, the in vitro release of the improved paliperidone depot implant is increased by 260%.

3. Characterization of in vitro release of improved estradiol depot implant.

3.1 Test Method

(1) One improved estradiol depot implant was taken, the silicone tube was cut open, the drug core was sliced into several small fragments, and placed in a 100 mL volumetric flask, 10 mL of methanol was added for infiltration, and the mixture was allowed to swell for 3 h. The solution was diluted with anhydrous ethanol to the mark, shaken well and filtered. 0.2 mL of the filtrate was accurately measured and placed in a 50 mL volumetric flask, diluted with the mobile phase to the mark, and shaken well. 20 $\mu$L of the sample was injected into the HPLC system, and the drug content was calculated according to the standard curve. The chromatographic conditions are as follows:

Chromatographic conditions
Chromatographic column: Diamonsil® C18 column (250 mm × 4 mm, 5 $\mu$m)
Mobile phase: Acetonitrile-water (55:45, v/v)
Column temperature: 25°C
Detection wavelength: 202nm
Flow rate: 0.8 $mL \cdot min^{-1}$
Injection volume: 20 $\mu$L

(2) Method for determining release rate

Release rate determination was performed using the horizontal oscillation method: one improved estradiol depot implant was taken and fixed in a 100 mL stoppered conical flask with adhesive. 100 mL of distilled water was accurately measured as the release medium, the stoppered conical flask was placed in a constant temperature shaker with an amplitude of 100 r·min$^{-1}$ and a temperature of 37°C to ensure that the implant was always below the liquid surface. Samples were taken and the release medium was replaced in the same volume every 24 h, the sample solution was filtered through a 0.22 μm microporous membrane and injected according to the content determination method in (1), the drug release amount was calculated according to the standard curve.

3.2 Results Data

3.2.1 Effect of sulfidation conditions on in vitro release amount

Table 15

| Time (d) | Daily release amount (μg) | | | Time (d) | Daily release amount (μg) | | |
|---|---|---|---|---|---|---|---|
| | 220°C | 240°C | 260°C | | 220°C | 240°C | 260°C |
| 1 | 32.78 | 31.61 | 33.59 | 95 | 27.37 | 26.75 | 28.04 |
| 5 | 31.33 | 33.70 | 34.93 | 100 | 27.72 | 25.13 | 27.74 |
| 10 | 32.56 | 31.27 | 34.32 | 105 | 27.54 | 25.55 | 28.52 |
| 15 | 29.54 | 31.97 | 29.07 | 110 | 27.28 | 26.20 | 28.66 |
| 20 | 28.43 | 27.79 | 32.42 | 115 | 26.84 | 26.95 | 28.69 |
| 25 | 29.05 | 27.15 | 30.14 | 120 | 27.66 | 25.81 | 28.39 |
| 30 | 28.20 | 28.16 | 32.73 | 125 | 24.59 | 25.57 | 28.98 |
| 35 | 28.24 | 26.65 | 32.78 | 130 | 26.61 | 27.84 | 28.93 |
| 40 | 29.64 | 27.69 | 29.15 | 135 | 24.17 | 26.66 | 28.41 |
| 45 | 29.17 | 29.78 | 29.01 | 140 | 24.82 | 27.63 | 26.77 |
| 50 | 29.61 | 28.76 | 29.53 | 145 | 25.32 | 25.96 | 26.17 |
| 55 | 26.73 | 27.47 | 29.83 | 150 | 24.61 | 25.83 | 26.86 |
| 60 | 27.46 | 28.62 | 28.21 | 155 | 24.92 | 25.79 | 26.95 |
| 65 | 26.62 | 24.38 | 27.33 | 160 | 24.64 | 25.38 | 26.04 |
| 70 | 27.14 | 28.93 | 28.11 | 165 | 26.06 | 24.78 | 26.63 |
| 75 | 27.68 | 27.83 | 28.42 | 170 | 24.83 | 23.55 | 26.41 |
| 80 | 27.50 | 26.34 | 28.81 | 175 | 25.32 | 25.97 | 26.89 |
| 85 | 27.19 | 27.25 | 28.16 | 180 | 26.93 | 25.87 | 26.61 |
| 90 | 27.71 | 26.92 | 27.81 | | | | |

The in vitro release data of the improved estradiol depot implants prepared at different sulfidation temperatures in Examples 17-1, 17-2, and 17-3 were listed in Table 15, the data in the table show that the change in the sulfidation temperature of the silicone tube has little effect on the average daily release amount of the implant, and the fluctuation is small., therefore, silicone tubes with better mechanical properties were selected for the preparation of the implant, that is, the sulfidation conditions were 250°C for the first sulfidation treatment and 240°C for the second sulfidation treatment.

3.3.2 Silicone tube wall thickness

Table 16

| Time (d) | Daily release amount (μg) | | | Time (d) | Daily release amount (μg) | | |
|---|---|---|---|---|---|---|---|
| | 0.2mm | 0.4mm | 0.6mm | | 0.2mm | 0.4mm | 0.6mm |
| 1 | 31.61 | 28.98 | 28.31 | 95 | 26.75 | 25.90 | 20.78 |
| 5 | 33.63 | 30.36 | 30.35 | 100 | 25.13 | 24.36 | 23.79 |
| 10 | 31.27 | 28.21 | 27.78 | 105 | 25.55 | 24.27 | 21.09 |
| 15 | 31.97 | 28.05 | 26.66 | 110 | 26.20 | 24.57 | 20.26 |
| 20 | 27.79 | 24.41 | 22.76 | 115 | 26.95 | 24.44 | 22.86 |
| 25 | 27.15 | 24.15 | 21.97 | 120 | 25.81 | 22.47 | 21.39 |
| 30 | 28.16 | 25.16 | 20.32 | 125 | 25.70 | 23.56 | 23.88 |
| 35 | 26.65 | 25.65 | 22.59 | 130 | 27.84 | 24.92 | 22.91 |
| 40 | 27.69 | 24.69 | 21.64 | 135 | 26.66 | 24.75 | 20.59 |
| 45 | 29.78 | 24.04 | 20.55 | 140 | 27.45 | 24.29 | 20.81 |
| 50 | 28.76 | 25.96 | 20.97 | 145 | 25.96 | 23.33 | 20.23 |
| 55 | 27.47 | 23.28 | 22.81 | 150 | 25.83 | 24.32 | 22.48 |
| 60 | 28.62 | 24.85 | 20.35 | 155 | 25.88 | 24.89 | 21.91 |
| 65 | 24.38 | 23.14 | 20.57 | 160 | 25.36 | 22.80 | 20.45 |
| 70 | 28.93 | 25.23 | 20.30 | 165 | 24.78 | 24.34 | 23.57 |
| 75 | 27.83 | 24.64 | 20.63 | 170 | 23.55 | 23.55 | 23.74 |
| 80 | 26.34 | 23.93 | 20.65 | 175 | 25.97 | 23.54 | 21.42 |
| 85 | 27.25 | 24.38 | 22.87 | 180 | 25.87 | 23.66 | 21.88 |
| 90 | 26.92 | 25.45 | 20.43 | | | | |

The in vitro release data of the improved estradiol reservoir implants with different wall thicknesses prepared in Examples 18-1, 18-2 and 18-3 were listed in Table 16, the results show that, with the outer diameter and release area kept constant, the in vitro release amount of the improved estradiol depot implants prepared from silicone tubes with three different wall thicknesses gradually decreased with the increase of wall thickness. To ensure drug release amount, the wall thickness should be reduced as much as possible, however, in actual operation, a silicone tube with a wall thickness of 0.2 mm is already the limit, further reducing the wall thickness would make it difficult to ensure the smooth extrusion molding of the silicone tube, therefore, it is recommended to use a silicone tube with a wall thickness of 0.2 mm.

3.3.3 Investigation of the drug release area of the silicone tube

The effect of the rod core length in the silicone tube on in vitro release is shown in the table below.

Table 17

| Drug release area($cm^2$) | Average daily release amount ($\mu g \cdot d^{-1}$) |
|---|---|
| 0.69 | 6.63+0.42 |
| 1.38 | 12.67+1.18 |
| 2.07 | 19.37±2.08 |
| 2.76 | 25.68+1.79 |
| 3.45 | 32.02±2.78 |

The results showed that the daily release amount of the improved estradiol depot implant increased with the increase of the release area. In practical applications, due to significant individual differences among patients, different specifications of improved estradiol depot implants can be produced during manufacturing to achieve personalized drug

delivery, reduce side effects, and maximize benefits according to different needs.

3.4 Long-term in vitro release experiment of estradiol depot implants

The estradiol depot implants prepared in Examples 17-2 and Comparative Example 3 were subjected to long-term in vitro release experiments at a temperature of 37°C and a shaking speed of 100 r·min$^{-1}$, the results are shown in the table and figure below.

Table 18

| Time (d) | Daily release amount (μg·d$^{-1}$) | Time (d) | Daily release amount (μg·d$^{-1}$) | Time (d) | Daily release amount (μg·d$^{-1}$) |
|---|---|---|---|---|---|
| 1 | 31.61 | | | | |
| 5 | 33.63 | 65 | 24.38 | 125 | 25.75 |
| 10 | 31.27 | 70 | 28.93 | 130 | 27.84 |
| 15 | 31.97 | 75 | 27.83 | 135 | 26.46 |
| 20 | 27.79 | 80 | 26.34 | 140 | 27.67 |
| 25 | 27.15 | 85 | 27.25 | 145 | 25.91 |
| 30 | 28.16 | 90 | 26.92 | 150 | 25.83 |
| 35 | 26.65 | 95 | 26.75 | 155 | 25.96 |
| 40 | 27.69 | 100 | 25.13 | 160 | 25.38 |
| 45 | 29.78 | 105 | 25.55 | 165 | 24.78 |
| 50 | 28.76 | 110 | 26.20 | 170 | 23.55 |
| 55 | 27.47 | 115 | 26.95 | 175 | 25.90 |
| 60 | 28.62 | 120 | 25.81 | 180 | 25.87 |

The results in the table above show that the implant prepared using hydrophilic silica has a stable daily in vitro drug release amount within the range of 20-35 μg, indicating good stability.

Table 19

| Time (d) | Daily release amount (μg·d$^{-1}$) | Time (d) | Daily release amount (μg·d$^{-1}$) | Time (d) | Daily release amount (μg·d$^{-1}$) |
|---|---|---|---|---|---|
| 1 | 26.23 | | | | |
| 5 | 18.34 | 65 | 11.08 | 125 | 10.72 |
| 10 | 14.27 | 70 | 12.77 | 130 | 10.86 |
| 15 | 13.97 | 75 | 12.86 | 135 | 10.38 |
| 20 | 12.79 | 80 | 11.37 | 140 | 10.62 |
| 25 | 12.17 | 85 | 12.23 | 145 | 10.96 |
| 30 | 13.13 | 90 | 11.98 | 150 | 10.89 |
| 35 | 11.65 | 95 | 11.74 | 155 | 10.95 |
| 40 | 12.67 | 100 | 11.12 | 160 | 10.36 |
| 45 | 11.75 | 105 | 11.52 | 165 | 9.75 |
| 50 | 12.44 | 110 | 10.93 | 170 | 10.55 |
| 55 | 12.46 | 115 | 11.36 | 175 | 10.92 |
| 60 | 11.83 | 120 | 10.97 | 180 | 10.82 |

The results in the table above indicate that the average daily release amount of estradiol depot implants is about 12 μg, with a burst release in the early stage and a stable release in the later stage.

The in vitro release of the improved estradiol depot implant was compared with that of the original estradiol depot implant, the results showed that, under the premise of meeting the mechanical properties of the implant, the in vitro release amount of the improved estradiol depot implant was increased by 245%, and the problem of burst release of the original estradiol depot implant was improved.

The preparation process of the silicone tube in the following examples is shown in Fig. 8.

The formulations of the silicone tubes with drug release holes for the perforated implants of Examples 20-38 and Comparative Examples 4-5 are as follows:

| Raw and auxiliary material names | Amount or content |
|---|---|
| Methyl vinyl silicone rubber | 100 parts |
| Vinyl content in methyl vinyl silicone rubber | 0.18% |
| Content of Si-H group in hydrogen-containing silicone oil | 0.75% |
| Molar ratio of Si-H groups in hydrogen-containing silicone oil to vinyl groups in methyl vinyl silicone rubber | 1.2:1 |
| Silica (water contact angle > 90°) | 40 parts |
| Platinum catalyst | 0.00001 parts |
| 2-Methyl-3-butyn-2-ol | 0.7 parts |
| Extrusion speed | 7.0r·min$^{-1}$ |
| First sulfidation temperature and time | 300°C, 5s |
| Second sulfidation temperature and time | 240°C, 2min |

Wherein, the water contact angle in the present disclosure is the static water contact angle of the material, and its measurement is conducted based on the principle of optical appearance projection. During the process, the water droplet volume was set using the testing software, and the syringe needle dropped the water droplet of the set volume onto the surface of the sample to be tested. Against a background of LED light source, the image of the droplet was projected into the automatic image analysis system by a screen micrometer for measurement.

The preparation methods of the silicone tubes with drug release holes for the perforated implants of Examples 20-38 and Comparative Examples 4-5 are as follows:

S1. the methyl vinyl silicone rubber was dried in an oven at 40 °C for 12h, and the silica was dried in an oven at 110 °C for 12h. Methyl vinyl silicone rubber and silica were kneaded in a vacuum kneader to obtain a rubber compound, which was then refined in an open mill to obtain mixture A;
S2. the mixture A was divided into a component A1 and a component A2, the component A1 was refined in an open mill, and hydrogen-containing silicone oil and 2-methyl-3-butyn-2-ol were added to obtain a component B1; the component A2 was dropwise added into a platinum catalyst, the roll distance was continuously shortened, and thin-pass processing was performed to obtain the component B2; the mass ratio of component B1 to component B2 was 1: 1;
S3. the component B1 and the component B2 were mixed and extruded in an open mill to obtain a mixture B, and first sulfidation treatment and second sulfidation treatment on the mixture B were sequentially carried out to obtain a silicone tube;
S4. silicone tubes with drug release holes can be obtained by laser drilling. The obtained silicone tube had a length of 4cm and an outer diameter of 2.4 mm.

The preparation methods of the rod core in Examples 20-38 and Comparative Examples 4-5 are as follows:

The drug composition can be pressed into a drug-containing layer by solid column molding at 0.3 MPa;
When a booster layer is contained, it can be prepared by subjecting the raw material of the booster layer to solid column molding and pressing at 0.3 MPa, and the booster layer can then be composited onto the drug-containing layer;
the prepared rod core had a length of 7 mm and a diameter of 2 mm, the plane where the rod core was located was perpendicular to the radial plane of the silicone tube.

(I) Process investigation of drug release holes for perforated implants

I. Investigation on the diameter of the drug release holes of perforated implants

Fig. 10 shows a schematic diagram of the perforated implants used in Examples 20-23.

Example 20 Insulin perforated Implant

The formulation composition of the rod core:

| | Example 20-1 | Example 20-2 | Example 20-3 | Example 20-4 | Example 20-5 |
|---|---|---|---|---|---|
| Drug release hole diameter μm | 150 | 250 | 350 | 450 | 550 |
| Drug-containing layer: Active drug (insulin): 15mg, sustained and controlled release excipient (hydrophilic gel material, HPMC K4M): 15mg, drug stabilizer (trehalose): 50mg, drug-to-excipient ratio (the ratio of the mass of the active drug to the total mass of the sustained and controlled release excipient and drug stabilizer) was 1:4.33 | | | | | |
| Booster layer: 32mg of expandable material (PEO N-80), 8mg of penetration enhancer (NaCl) | | | | | |
| The mass ratio of drug-containing layer to booster layer was 2: 1 | | | | | |
| Silicone tube with drug release holes: the number of drug release holes is one, and the position of drug release holes: the distance from the center point of drug release holes to the first end surface and the second end surface of silicone tube with drug release holes is 0.5 times of the length of silicone tube | | | | | |

Example 21 Semaglutide perforated Implant

The formulation composition of the rod core:

| | Example 21-1 | Example 21-2 | Example 21-3 | Example 21-4 | Example 21-5 |
|---|---|---|---|---|---|
| Drug release hole diameter μm | 150 | 250 | 350 | 450 | 550 |
| Drug-containing layer: active drug (semaglutide): 15mg, sustained and controlled release excipient (hydrophilic gel material, HPMC K4M): 15mg, filler (MCC): 50mg, drug-to-excipient ratio (the ratio of the mass of the active drug to the total mass of the sustained and controlled release excipient and filler) was 1:4.33 | | | | | |
| Booster layer: 32mg of expandable material (PEO N-80), 8mg of penetration enhancer (NaCl) | | | | | |
| Silicone tube with drug release holes: the number of drug release holes is one, and the position of drug release holes: the distance from the center point of drug release holes to the first end surface and the second end surface of silicone tube with drug release holes was 0.5 times of the length of silicone tube | | | | | |

Example 22 Metformin perforated Implant

The formulation composition of the rod core:

| | Example 22-1 | Example 22-2 | Example 22-3 | Example 22-4 | Example 22-5 |
|---|---|---|---|---|---|
| Drug release hole diameter μm | 150 | 250 | 350 | 450 | 550 |
| Drug-containing layer: active drug (metformin): 15mg, sustained and controlled release excipient (hydrophilic gel material, HPMC K4M): 15mg, filler (MCC): 50mg, drug-to-excipient ratio (the ratio of the mass of the active drug to the total mass of the sustained and controlled release excipient and filler) was 1:4.33 | | | | | |
| Booster layer: 32mg of expandable material (PEO N-80), 8mg of penetration enhancer (NaCl) | | | | | |
| Silicone tube with drug release holes: the number of drug release holes is one, and the position of drug release holes: the distance from the center point of drug release holes to the first end surface and the second end surface of silicone tube with drug release holes was 0.5 times of the length of silicone tube | | | | | |

Example 23 Paliperidone perforated Implant

The formulation composition of the rod core:

| | Example 23-1 | Example 23-2 | Example 23-3 | Example 23-4 | Example 23-5 |
|---|---|---|---|---|---|
| Drug release hole diameter μm | 150 | 250 | 350 | 450 | 550 |
| Drug-containing layer: active drug (paliperidone): 15mg, sustained and controlled release excipient (hydrophilic gel material, HPMC K4M): 15mg, filler (MCC): 50mg, drug-to-excipient ratio (the ratio of the mass of the active drug to the total mass of the sustained and controlled release excipient and filler)was 1:4.33 | | | | | |
| Booster layer: 32mg of expandable material (PEO N-80), 8mg of penetration enhancer (NaCl) | | | | | |
| Silicone tube with drug release holes: the number of drug release holes is one, and the position of release holes: the distance from the center point of drug release holes to the first end surface and the second end surface of silicone tube with drug release holes was 0.5 times of the length of silicone tube | | | | | |

II. Investigation on the number of drug release holes in perforated implants

Schematic diagrams of the perforated implants in Examples 24-25 are shown in Figures 12-14.

Example 24, Comparative Example 4: insulin perforated implant

The formulation composition of the rod core:

| | Comparative example 4 | Example 24-1 | Example 24-2 | Example 24-3 |
|---|---|---|---|---|
| Number of drug release holes | nonporous | Single hole | two holes | three holes |
| Drug-containing layer: Active drug (insulin) 15mg, sustained and controlled release excipient (hydrophilic gel material, HPMC K4M): 15mg, drug stabilizer (trehalose): 50mg, drug-to-excipient ratio (the ratio of the mass of the active drug to the total mass of the sustained and controlled release excipient and drug stabilizer) was 1:4.33 | | | | |
| Booster layer: 32mg of expandable material (PEO N-80), 8mg of penetration enhancer (NaCl) | | | | |
| The mass ratio of drug-containing layer to booster layer was 2: 1 | | | | |
| Silicone tube with drug release hole: the diameter of the drug release hole was 350μm,<br>For single hole, the position of the drug release hole: the distance from the center point of the drug release hole to the first end surface and the second end surface of the silicone tube with the drug release hole was 0.5 times the length of the silicone tube.<br>For two holes, there were first and second drug release holes in turn, with the first drug release hole near the first end surface and the second drug release hole near the second end surface;<br>The first and second release holes were not on the same radial plane of the silicone tube, in the axial direction of the silicone tube, the distance from the center point of the first release hole to the first end surface of the silicone tube was 0.5 times the length of the silicone tube, the distance between the center points of the second and first release holes was 0.3 cm, and the distance from the second release holes to the second end surface was 1.7 cm<br>For three holes, there were the first drug release hole, the second drug release hole and the third drug release hole in turn, with the first drug release hole close to the first end surface and the third drug release close to the second end surface; the second drug release hole was arranged between the first drug release hole and the second drug release hole; hole | | | | |
| The first drug release hole, the second drug release hole and the third drug release hole were not on same radial section of the silicone tube, in the axial section direction of the silicone tube, the distance the center point of the first drug release hole to the first end surface of the silicone tube was 0.5 times length of the silicone tube, the distance between the center points of the second drug release hole and first drug release hole was 0.3cm, and the distance between the center points of the third drug release and the second drug release hole was 0.3cm, the distance from the third drug release hole to the second surface was 1.4 cm. fromholeendthethethe | | | | |

Example 25, Comparative Example 5: Semaglutide perforated implant

The formulation composition of the rod core:

| | Comparative example 5 | Example 25-1 | Example 25-2 | Example 25-3 |
|---|---|---|---|---|
| Number of drug release holes | nonporous | single hole | two holes | three holes |
| Drug-containing layer: active drug (somalurutide): 15mg, sustained and controlled release excipient (hydrophilic gel material, HPMC K4m): 15mg, filler (MCC): 50mg, and the ratio of drug-to-excipient (the ratio of the mass of active drug to the total mass of sustained and controlled release excipient and filler) was 1: 4.33 | | | | |
| Booster layer: 32mg of expandable material (PEO N-80), 8mg of penetration enhancer (NaCl) | | | | |
| Silicone tube with drug release holes: the diameter of the drug release hole is 350μm,<br>For single hole, the position of the drug release hole: the distance from the center point of the drug release hole to the first end surface and the second end surface of the silicone tube with the drug release hole was 0.5 times the length of the silicone tube.<br>For two holes, there were first and second drug release holes in turn, with the first drug release hole near the first end surface and the second drug release hole near the second end surface;<br>The first and second release holes were not on the same radial plane of the silicone tube, in the axial direction of the silicone tube, the distance from the center point of the first release hole to the first end surface of the silicone tube was 0.5 times the length of the silicone tube, the distance between the center points of the second and first release holes was 0.3 cm, and the distance from the second release holes to the second end surface was 1.7 cm<br>For three holes, there were the first drug release hole, the second drug release hole and the third drug release hole in turn, with the first drug release hole close to the first end surface and the third drug release hole close to the second end surface; the second drug release hole is arranged between the first drug release hole and the second drug release hole; | | | | |
| The first drug release hole, the second drug release hole and the third drug release hole were not on same radial section of the silicone tube, in the axial section direction of the silicone tube, the distance the center point of the first drug release hole to the first end surface of the silicone tube was 0.5 times length of the silicone tube, the distance between the center points of the second drug release hole and first drug release hole was 0.3cm, and the distance between the center points of the third drug release and the second drug release hole was 0.3cm, the distance from the third drug release hole to the second surface was 1.4 cm. | | | | |

III. Investigation of the location of the drug release hole in perforated implants

Schematic diagrams of the perforated implants in Examples 26-29 are shown in Fig 11 and 15, respectively.

Example 26 Insulin Perforated Implant

The formulation composition of the rod core:

| | Example 26-1 | Example 26-2 |
|---|---|---|
| Position of drug release hole | the distance from the center point of the drug release hole to the first end surface and the second end surface of the silicone tube containing the drug release hole was 0.5 times the length of the silicone tube | the distance from the center point of the drug release hole to the first end surface of the silicone tube containing the drug release hole was 0.3 cm |
| Drug-containing layer: active drug (insulin): 15mg, sustained and controlled release excipient (hydrophilic gel material, HPMC K4m): 15mg, drug stabilizer (trehalose): 50mg, and the drug-to-excipient ratio (the ratio of the mass of active drug to the total mass of sustained and controlled release excipient and drug stabilizer) was 1: 4.33 | | |
| Booster layer: 32mg of expandable material (PEO N-80), 8mg of penetration enhancer (NaCl) | | |
| Silicone tube with drug release hole: the diameter of the drug release hole was 350μm, and the number of drug release holes was one | | |

Example 27 Somaruritide Perforated Implant

The formulation composition of the rod core:

| | Example 27-1 | Example 27-2 |
|---|---|---|
| Position of drug release hole | the distance from the center point of the drug release hole to the first end surface and the second end surface of the silicone tube containing the drug release hole was 0.5 times the length of the silicone tube | the distance from the center point of the drug release hole to the first end surface of the silicone tube containing the drug release hole was 0.3 cm |
| Drug-containing layer: active drug (somalurutide): 15mg, sustained and controlled release excipient (hydrophilic gel material, HPMC K4m): 15mg, filler (MCC): 50mg, and the ratio of drug-to-excipient (the ratio of the mass of active drug to the total mass of sustained and controlled release excipient and filler) was 1: 4.33 | | |
| Booster layer: 32mg of expandable material (PEO N-80), 8mg of penetration enhancer (NaCl) | | |
| Silicone tube with drug release hole: the diameter of the drug release hole was 350μm, and the number of drug release holes was one | | |

Example 28 Metformin perforated Implant

The formulation composition of the rod core:

| | Example 28-1 | Example 28-2 |
|---|---|---|
| Position of drug release hole | the distance from the center point of the drug release hole to the first end surface and the second end surface of the silicone tube containing the drug release hole was 0.5 times the length of the silicone tube | the distance from the center point of the drug release hole to the first end surface of the silicone tube containing the drug release hole was 0.3 cm |
| Drug-containing layer: active drug (metformin): 15mg, sustained and controlled release excipient (hydrophilic gel material, HPMC K4M): 15mg, filler (MCC): 50mg, drug-to-excipient ratio (the ratio of the mass of the active drug to the total mass of the sustained and controlled release excipient and filler) was 1:4.33 | | |
| Booster layer: 32mg of expandable material (PEO N-80), 8mg of penetration enhancer (NaCl) | | |
| Silicone tube with drug release hole: the diameter of the drug release hole was 250μm, and the number of drug release holes was one | | |

Example 29 Paliperidone perforated Implant

The formulation composition of the rod core:

| | Example 29-1 | Example 29-2 |
|---|---|---|
| Position of drug release hole | the distance from the center point of the drug release hole to the first end surface and the second end surface of the silicone tube containing the drug release hole was 0.5 times the length of the silicone tube | the distance from the center point of the drug release hole to the first end surface of the silicone tube containing the drug release hole was 0.3 cm |
| Drug-containing layer: active drug (paliperidone): 15mg, sustained and controlled release excipient (hydrophilic gel material, HPMC K4M): 15mg, filler (MCC): 50mg, drug-to-excipient ratio (the ratio of the mass of the active drug to the total mass of the sustained and controlled release excipient and filler) was 1:4.33 | | |
| Booster layer: 32mg of expandable material (PEO N-80), 8mg of penetration enhancer (NaCl) | | |
| Silicone tube with drug release hole: the diameter of the drug release hole was 350μm, and the number of drug release holes was one | | |

(II) Investigation of the rod core formulation of perforated implants

I. Single-layer and single-gradient perforated implant

The pore parameters of silicone tubes with drug release holes of the perforated implants of Examples 30, 32 and 34-38 are as follows:

| | |
|---|---|
| Drug release hole diameter | 350μm |
| Number of drug release holes | 1 |
| Position of drug release hole | the distance from the center point of the drug release hole to the first end surface and the second end surface of the silicone tube with drug release hole was 0.5 times the length of the silicone tube |

The pore parameters of silicone tubes with drug release holes of the perforated implants of Examples 31 and 33 are as follows:

| | |
|---|---|
| Drug release hole diameter | 250μm |
| Number of drug release holes | 1 |
| Position of drug release hole | the distance from the center point of the drug release hole to the first end surface and the second end surface of the silicone tube with drug release hole was 0.5 times the length of the silicone tube |

(i) Investigation on the types of excipients for drug-containing layer of the rod core of single-layer and single-gradient perforated implant

Fig. 9 shows a schematic diagram of the perforated implants used in Examples 30-33.

Example 30 Exenatide Perforated Implant

The formulation composition of the rod core:

| | Example 30-1 | Example 30-2 | Example 30-3 | Example 30-4 | Example 30-5 | Example 30-6 |
|---|---|---|---|---|---|---|
| Types of sustained and controlled release excipients | UTEC S100 | Calcium alginate | Ethyl cellulose | PLGA | HPMC K 15M | PEO N80 |
| Drug-containing layer: active drug (exenatide) 40mg, drug-to-excipient ratio (mass ratio of active drug to sustained and controlled release excipients) 1: 3 | | | | | | |

Example 31 Metformin perforated Implant

The formulation composition of the rod core:

| | Example 31-1 | Example 31-2 | Example 31-3 |
|---|---|---|---|
| Types of hydrophilic gel materials | HPMCK 15M | HPMCK 4M | PEO N80 |
| Drug-containing layer: active drug (metformin) 40mg, drug-to-excipient ratio (mass ratio of active drug to sustained and controlled release excipients) 1: 7, the insoluble matrix material in sustained and controlled release excipients was ethyl cellulose, and the mass ratio of insoluble matrix material to hydrophilic gel material was 1:1 | | | |

(ii) Investigation on the drug-to-excipient ratio of the drug-containing layer of the rod core of a single-layer and single-gradient perforated implant

Example 32 Exenatide Perforated Implant

The formulation composition of the rod core:

| | Example 32-1 | Example 32-2 | Example 32-3 |
|---|---|---|---|
| Drug-to -excipient ratio (mass ratio of active drug to sustained and controlled release excipient) | 1:3 | 1:5 | 1:7 |
| Drug-containing layer: active drug (exenatide) 40mg, sustained and controlled release excipient: ethyl cellulose | | | |

(iii) Investigation on the ratio of insoluble matrix material to hydrophilic gel material in the sustained and controlled release excipients of drug-containing layer of the rod core of single-layer and single-gradient perforated implant

Example 33 Metformin perforated Implant

The formulation composition of the rod core:

| | Example 33-1 | Example 33-2 | Example 33-3 |
|---|---|---|---|
| The ratio of insoluble matrix material to hydrophilic gel material | 1:1 | 2:1 | 1:2 |
| Drug-containing layer: active drug (metformin) 40mg, drug-to-excipient ratio (mass ratio of active drug to sustained and controlled release excipients) 1: 7, the sustained and controlled release excipients were ethyl cellulose and HPMC K15M | | | |

II. Single-layer and multi-gradient perforated implant

(i) Investigation of active drug concentration in each gradient release module of the rod core of single-layer and multi-gradient perforated implant.

Fig. 11 shows a schematic diagram of the perforated implants used in Example 34.

Example 34 Somaruritide Perforated Implant

| | Example 34-1 | Example 34-2 | Example 34-3 | Example 34-4 |
|---|---|---|---|---|
| Concentration gradient of active drugs | 2%/10%/20% | 2%/20%/30% | 10%/20%/30% | 5%/20%/30% |
| Drug-containing layer: active drug (somalurutide), sustained and controlled release excipient: ethyl cellulose EC | | | | |
| Mass of single drug release module: 50mg, total mass of drug-containing layer: 150mg | | | | |

III. Double-layer perforated implant

(i) Investigation on the types of excipients for the drug-containing layer of the rod core of the double-layer perforated implant

Example 35 Levothyroxine Sodium Perforated Implant

The formulation composition of the rod core:

| | Example 35-1 | Example 35-2 | Example 35-3 | Example 35-4 |
|---|---|---|---|---|
| Types of sustained and controlled release excipients | PEO WSR N-750 | PEO WSR 303 | PVP K30 | HPMC K4M |
| Drug-containing layer: active drug (levothyroxine sodium) 40mg, sustained and controlled release excipients 60mg, and drug-to-excipients ratio (mass ratio of active drug to sustained and controlled release excipients) 1: 1.5 | | | | |
| Booster layer: expandable material (PEO WSR N-60k): 40mg, penetration enhancer (NaCl): 10mg | | | | |

(ii) Investigation on the mass ratio of sustained and controlled release excipients and fillers in the drug-containing layer of the rod core of the double-layer perforated implant

Example 36 Levothyroxine Sodium Perforated Implant

The formulation composition of the rod core:

| | Example 36-1 | Example 36-2 | Example 36-3 |
|---|---|---|---|
| Mass ratio of sustained and controlled release excipients to fillers | 1:2 | 2:1 | No fillers |
| Drug-containing layer: active drug (levothyroxine sodium) 40mg, sustained and controlled release excipient (PEO WSR N-750), filler (MCC), and drug-to-excipient ratio (the ratio of the mass of active drug to the total mass of sustained and controlled release excipient and filler) is 1: 1.5 | | | |
| Booster layer: expandable material (PEO WSR N60k): 40mg, penetration enhancer (NaCl): 10mg | | | |

(iii) Investigation of the types of expandable materials in the booster layer of the rod core of the double-layer perforated implant

Example 37 Levothyroxine Sodium Perforated Implant

The formulation composition of the rod core:

| | Example 37-1 | Example 37-2 | Example 37-3 |
|---|---|---|---|
| Expandable material in the booster layer | PEO WSR 1105 | PEO WSR N-12K | PEO WSR N-60K |
| Drug-containing layer: active drug (levothyroxine sodium) 40mg, sustained and controlled release excipient (HPMC K4M) 40mg, filler (MCC) 20mg, drug-to-excipient ratio (the ratio of the mass of the active drug to the total mass of the sustained and controlled release excipient and filler) was 1: 1.5 | | | |
| Booster layer: expandable material: 40mg, penetration enhancer (NaCl): 10mg | | | |

(iv) Investigation on the amount of expandable material in the booster layer of the rod core of double-layer perforated implant

Example 38 Levothyroxine Sodium Perforated Implant

Formulation of the drug rod

| | Example 38-1 | Example 38-2 | Example 38-3 |
|---|---|---|---|
| Expandable material in the booster layer mg | 20 | 40 | 60 |

(continued)

| | Example 38-1 | Example 38-2 | Example 38-3 |
|---|---|---|---|
| Drug-containing layer: active drug (levothyroxine sodium) 40mg, sustained and controlled release excipient (HPMC K4M) 40mg, filler (MCC) 20mg, drug-to-excipient ratio (the ratio of the mass of the active drug to the total mass of the sustained and controlled release excipient and filler) was 1: 1.5 | | | |
| Booster layer: expandable material (PEO WSRN60K), penetration enhancer (NaCl) 10 mg | | | |

Effect Example Establishment of Release Determination Method

1. Testing conditions

(1) Chromatographic conditions of exenatide

Chromatographic column: Diamonsil® C18 column (250mm × 4.60mm, 5μm);
Mobile phase: Phase A: 0.08% trifluoroacetic acid acetonitrile solution;
Mobile phase: Phase B: 0.08% aqueous trifluoroacetic acid solution;
The ratio of mobile phase A changes with time from 28% to 55% (0 ~ 25 min).
Flow rate: 1.0 $mL\cdot min^{-1}$;
Detection wavelength: 214nm;
Column temperature: 30°C;
Injection volume: 20 μL;

(2) Chromatographic conditions of estradiol

Column: Diamonsil® C18 column (250mm×4mm, 5μm);
Mobile phase: Acetonitrile-water (55:45, v/v);
Column temperature: 25°C;
Detection wavelength: 202nm;
Flow rate: 0.8 $mL\cdot min^{-1}$;

(3) Chromatographic conditions of semaglutide

Chromatographic column: Diamonsil® C18 column (250mm × 4.60mm, 5μm);
Mobile phase: 0.2 $mol\cdot L^{-1}$ sulfate buffer-acetonitrile (60:40, v/v);
Column temperature: 30°C;
Detection wavelength: 192nm;
Flow rate: 1 $mL\cdot min^{-1}$;
Injection volume: 20 μL;

(4) Chromatographic conditions of levothyroxine sodium

Chromatographic column: SinoDetectHCL-C18 column (4.6×250mm, 5μm);
Mobile phase: acetonitrile -0.1% TFA (50:50, v/v/v);
Column temperature: 30°C;
Detection wavelength: 225nm;
Flow rate: 1 $mL\cdot min^{-1}$;
Injection volume: 20 μL;

(5) Chromatographic conditions for metformin

Chromatographic column: Agilent® C18 column (4.6 mm × 150 mm, 5 μm);
Mobile phase: methanol: water (10:90, v/v) (adjusted to pH 3.5 with phosphoric acid and triethylamine);
Column temperature: room temperature;
Detection wavelength: 233nm;
Flow rate: 1.0 mL·min$^{-1}$;
Injection volume: 20 μL;

(6) Chromatographic conditions for insulin

Chromatographic column: Diamonsil® C18 column (250mm × 4.60mm, 5μm);
Mobile phase: Methanol: water (65:35, v/v, with 0.1% TFA added by total volume);
Column temperature: 40°C;
Detection wavelength: 214nm;
Flow rate: 1.0 mL·min$^{-1}$;
Injection volume: 20 μL;

(7) chromatographic conditions of paliperidone

Column: Agilent® C8 column (4.6 × 100 mm, 2.7 μm);
Mobile phase: methanol: acetonitrile: water (5:25:70, v/v) (phosphoric acid and triethylamine adjust pH to 3);
Column temperature: 35°C;
Detection wavelength: 270nm;
Flow rate: 0.8 mL·min$^{-1}$;
Injection volume: 20 μL;

2. Release Results

(I) Process investigation of the drug release hole of perforated implants

I. Investigation on the diameter of the drug release hole for perforated implants

(1) The release results (daily release dose μg) of insulin single-layer and single-gradient perforated implant model are shown in the following table.

| Time (d) | Example 20-1 | Example 20-2 | Example 20-3 | Example 20-4 | Example 20-5 |
|---|---|---|---|---|---|
| 1 | 16.39 | 88.06 | 127.41 | 206.48 | 304.13 |
| 5 | 37.30 | 102.61 | 181.88 | 308.61 | 542.01 |
| 10 | 80.32 | 64.71 | 95.19 | 204.94 | 244.78 |
| 15 | 19.74 | 46.95 | 88.11 | 113.42 | 254.86 |
| 20 | 27.15 | 41.17 | 89.64 | 234.13 | 197.21 |
| 25 | 19.09 | 34.78 | 52.12 | 97.58 | 105.48 |
| 30 | 15.99 | 27.47 | 61.85 | 152.74 | 173.38 |
| 35 | 29.58 | 54.69 | 80.33 | 105.25 | 142.01 |
| 40 | 13.19 | 53.43 | 92.78 | 188.53 | 244.76 |

(continued)

| Time (d) | Example 20-1 | Example 20-2 | Example 20-3 | Example 20-4 | Example 20-5 |
|---|---|---|---|---|---|
| 45 | 19.24 | 28.22 | 65.37 | 101.09 | 109.86 |
| 50 | 22.38 | 25.59 | 64.79 | 110.26 | 132.49 |
| 55 | 16.52 | 30.93 | 78.22 | 95.09 | 105.66 |
| 60 | 12.24 | 17.72 | 65.71 | 112.84 | 132.41 |

(2) The release results (daily release dose μg) of the semaglutide single-layer and single-gradient perforated implant model are shown in the table below.

| Time (d) | Example 21-1 | Example 21-2 | Example 21-3 | Example 21-4 | Example 21-5 |
|---|---|---|---|---|---|
| 1 | 114.16 | 125.68 | 155.75 | 421.09 | 308.38 |
| 5 | 272.47 | 347.75 | 400.93 | 733.43 | 766.56 |
| 10 | 141.12 | 188.92 | 260.52 | 559.65 | 528.48 |
| 15 | 102.32 | 252.14 | 204.12 | 467.07 | 422.89 |
| 20 | 83.52 | 154.76 | 209.81 | 255.25 | 305.27 |
| 25 | 83.13 | 90.32 | 127.82 | 310.17 | 298.73 |
| 30 | 87.29 | 86.43 | 220.71 | 325.54 | 206.81 |
| 35 | 60.92 | 130.62 | 221.57 | 220.29 | 254.26 |
| 40 | 58.97 | 96.16 | 169.64 | 214.48 | 217.53 |
| 45 | 91.28 | 93.65 | 236.51 | 276.81 | 262.22 |
| 50 | 59.85 | 115.51 | 227.13 | 212.82 | 268.62 |
| 55 | 79.15 | 68.06 | 263.09 | 285.68 | 233.35 |
| 60 | 66.03 | 52.91 | 188.56 | 212.39 | 148.45 |

(3) The release results (daily release dose μg) of metformin single-layer and single-gradient perforated implant model are shown in the following table.

| Time (d) | Example 22-1 | Example 22-2 | Example 22-3 | Example 22-4 | Example 22-5 |
|---|---|---|---|---|---|
| 1 | 242.55 | 249.74 | 221.16 | 394.99 | 573.94 |
| 5 | 404.68 | 364.32 | 479.23 | 876.11 | 1218.81 |
| 10 | 360.44 | 185.24 | 330.33 | 732.05 | 1312.67 |
| 15 | 289.73 | 226.15 | 449.85 | 438.72 | 823.73 |
| 20 | 173.87 | 257.83 | 329.21 | 462.53 | 395.88 |
| 25 | 117.74 | 243.31 | 212.97 | 241.19 | 377.12 |
| 30 | 108.58 | 182.03 | 163.31 | 131.78 | 485.39 |
| 35 | 108.89 | 154.12 | 181.07 | 110.05 | 316.77 |
| 40 | 99.35 | 214.81 | 191.48 | 132.91 | 385.43 |
| 45 | 144.47 | 135.04 | 178.40 | 199.97 | 289.07 |
| 50 | 127.74 | 124.09 | 211.37 | 229.07 | 201.41 |
| 55 | 86.13 | 99.04 | 128.83 | 114.53 | 322.11 |
| 60 | 107.29 | 149.33 | 92.46 | 141.72 | 98.71 |

(4) The release results (daily release dose μg) of paliperidone single-layer and single-gradient perforated implant model are shown in the following table.

| Time (d) | Example 23-1 | Example 23-2 | Example 23-3 | Example 23-4 | Example 23-5 |
|---|---|---|---|---|---|
| 1 | 77.91 | 185.29 | 213.12 | 255.24 | 408.35 |
| 5 | 209.14 | 267.77 | 364.51 | 537.97 | 766.56 |
| 10 | 147.95 | 303.09 | 389.41 | 478.91 | 528.42 |
| 15 | 71.79 | 241.47 | 334.67 | 230.66 | 423.62 |
| 20 | 106.74 | 226.16 | 211.21 | 298.06 | 343.54 |
| 25 | 146.74 | 129.39 | 213.15 | 229.36 | 200.01 |
| 30 | 98.54 | 130.81 | 208.48 | 203.08 | 240.22 |
| 35 | 101.35 | 165.45 | 180.61 | 206.62 | 250.03 |
| 40 | 143.21 | 152.17 | 170.19 | 230.75 | 257.68 |
| 45 | 88.79 | 174.47 | 192.94 | 224.33 | 294.95 |
| 50 | 94.83 | 147.96 | 233.78 | 175.13 | 254.71 |
| 55 | 114.05 | 114.38 | 152.96 | 232.46 | 253.41 |
| 60 | 102.76 | 114.47 | 140.34 | 188.99 | 188.88 |

According to Example 20-4, the implant can be applied to soluble macromolecular drugs, insoluble macromolecular drugs, insoluble macromolecular drugs and soluble small molecule drugs.

Further analysis of Example 20-4 shows that in the initial stage of drug release, burst release occurred when the pore diameter was greater than 450μm, characterized by rapid drug release followed by a sharp decrease in the release amount, resulting in insufficient power in the later stage and difficulty to realize long-term drug release. When the pore diameter is less than 250μm (except for metformin), the release amount is relatively stable throughout the release period, but the daily release amount of the formulation is lower than that of the larger pore diameter (for example, more than 250μm).

II. Investigation on the number of drug release holes in perforated implants

(1) The release results (daily release dose μg) of insulin single-layer and single-gradient perforated implant model are shown in the following table.

| Time (d) | Comparative example 4 | Example 24-1 | Example 24-2 | Example 24-3 |
|---|---|---|---|---|
| 1 | 0 | 127.41 | 138.31 | 199.43 |
| 5 | 0 | 181.88 | 292.65 | 373.12 |
| 10 | 0 | 95.11 | 133.37 | 165.90 |
| 15 | 0 | 88.16 | 88.52 | 110.27 |
| 20 | 0 | 89.64 | 93.06 | 92.03 |
| 25 | 0 | 52.12 | 79.67 | 66.81 |
| 30 | 0 | 61.85 | 83.37 | 122.28 |
| 35 | 0 | 80.33 | 53.45 | 72.76 |
| 40 | 0 | 92.78 | 86.96 | 121.85 |
| 45 | 0 | 65.37 | 68.52 | 98.21 |
| 50 | 0 | 64.79 | 96.12 | 57.87 |
| 55 | 0 | 78.22 | 98.02 | 53.43 |

(continued)

| Time (d) | Comparative example 4 | Example 24-1 | Example 24-2 | Example 24-3 |
|---|---|---|---|---|
| 60 | 0 | 65.71 | 74.28 | 84.67 |

(2) The release results (daily release dose μg) of the semaglutide single-layer and single-gradient perforated implant model are shown in the table below.

| Time (d) | Comparative example 5 | Example 25-1 | Example 25-2 | Example 25-3 |
|---|---|---|---|---|
| 1 | 0 | 155.75 | 438.35 | 635.34 |
| 5 | 0 | 400.32 | 773.42 | 731.11 |
| 10 | 0 | 260.52 | 516.69 | 697.42 |
| 15 | 0 | 204.12 | 381.37 | 388.69 |
| 20 | 0 | 209.81 | 236.82 | 273.49 |
| 25 | 0 | 127.82 | 263.68 | 175.47 |
| 30 | 0 | 220.71 | 223.65 | 263.41 |
| 35 | 0 | 221.57 | 261.55 | 177.15 |
| 40 | 0 | 169.64 | 132.69 | 186.17 |
| 45 | 0 | 236.51 | 337.99 | 256.53 |
| 50 | 0 | 227.13 | 165.82 | 221.28 |
| 55 | 0 | 263.09 | 216.42 | 165.55 |
| 60 | 0 | 188.56 | 186.66 | 186.25 |

At the initial stage of drug release, the daily release amount of the formulation will increase with the increase of the number of drug release holes, among which the implants with two or three drug release holes have burst release phenomenon.

III. Investigation of the location of the drug release hole in perforated implants

(1) The release results (daily release dose μg) of insulin single-layer and single-gradient perforated implant model are shown in the following table.

| Time (d) | Example 26-1 | Example 26-2 | Time (d) | Example 26-1 | Example 26-2 |
|---|---|---|---|---|---|
| 1 | 127.41 | 134.19 | 35 | 80.33 | 61.78 |
| 5 | 181.88 | 151.16 | 40 | 92.78 | 69.45 |
| 10 | 95.11 | 120.63 | 45 | 65.37 | 103.48 |
| 15 | 88.33 | 125.43 | 50 | 64.79 | 112.69 |
| 20 | 89.64 | 109.94 | 55 | 78.22 | 91.39 |
| 25 | 52.12 | 96.01 | 60 | 65.71 | 91.67 |
| 30 | 61.85 | 114.27 | | | |

(2) The release results (daily release dose μg) of the semaglutide single-layer and single-gradient perforated implant model are shown in the table below.

| Time (d) | Example 27-1 | Example 27- 2 | Time (d) | Example 27- 1 | Example 27- 2 |
|---|---|---|---|---|---|
| 1 | 155.75 | 161.11 | 35 | 221.57 | 167.18 |
| 5 | 400.93 | 428.42 | 40 | 169.64 | 163.57 |
| 10 | 260.52 | 328.66 | 45 | 236.51 | 182.89 |
| 15 | 204.12 | 214.34 | 50 | 227.13 | 151.44 |
| 20 | 209.81 | 193.46 | 55 | 263.09 | 104.47 |
| 25 | 127.82 | 218.69 | 60 | 188.56 | 144.83 |
| 30 | 220.71 | 155.92 | | | |

(3) The release results (daily release dose μg) of metformin single-layer and single-gradient perforated implant model are shown in the following table.

| Time (d) | Example 28-1 | Example 28-2 | Time (d) | Example 28-1 | Example 28-2 |
|---|---|---|---|---|---|
| 1 | 249.72 | 310.95 | 35 | 154.12 | 155.20 |
| 5 | 364.32 | 451.08 | 40 | 214.81 | 156.59 |
| 10 | 185.21 | 294.16 | 45 | 135.04 | 150.63 |
| 15 | 226.15 | 273.99 | 50 | 124.09 | 118.99 |
| 20 | 257.70 | 192.67 | 55 | 99.04 | 205.77 |
| 25 | 243.31 | 182.01 | 60 | 149.33 | 157.41 |
| 30 | 182.03 | 233.67 | | | |

(4) The release results (daily release dose μg) of paliperidone single-layer and single-gradient perforated implant model are shown in the following table.

| Time (d) | Example 29-1 | Example 29-2 | Time (d) | Example 29-1 | Example 29-2 |
|---|---|---|---|---|---|
| 1 | 213.12 | 233.10 | 35 | 180.61 | 198.26 |
| 5 | 364.51 | 400.52 | 40 | 170.19 | 217.62 |
| 10 | 389.41 | 319.83 | 45 | 192.94 | 201.98 |
| 15 | 334.67 | 261.55 | 50 | 233.78 | 231.28 |
| 20 | 211.21 | 176.18 | 55 | 152.96 | 170.84 |
| 25 | 213.15 | 201.23 | 60 | 140.30 | 127.11 |
| 30 | 208.48 | 208.70 | | | |

According to the results of drug release curves, the change of the position of drug release hole in single channel has no great influence on drug release, and the release curves are close, with no significant difference.

(II) Investigation of the formulation of rod core of perforated implants

I. Single-layer and single-gradient perforated implant

(i) The investigation results (daily release dose μg) of the expandable materials of the drug-containing layer of the rod core of the single-layer and single-gradient perforated implant are shown in the following table.

| Time (d) | Example 30-1 | Example 30-2 | Example 30-3 | Example 30-4 | Example 30-5 | Example 30-6 |
|---|---|---|---|---|---|---|
| 1 | 2.16 | 2.46 | 39.66 | 200.09 | 150.98 | 0.00 |
| 5 | 18.98 | 12.29 | 76.54 | 275.29 | 130.20 | 0.00 |
| 10 | 42.98 | 11.37 | 73.77 | 200.22 | 121.19 | 0.00 |
| 15 | 36.28 | 21.17 | 62.62 | 29.72 | 32.68 | 379.45 |
| 20 | 42.77 | 28.63 | 74.66 | 33.78 | 22.74 | 100.13 |
| 25 | 39.82 | 12.83 | 62.07 | 42.54 | 37.42 | 87.27 |
| 30 | 48.12 | 19.78 | 62.44 | 37.12 | 73.01 | 92.87 |
| 35 | 46.77 | 22.96 | 54.36 | 29.34 | 52.99 | 91.68 |
| 40 | 39.99 | 7.12 | 60.99 | 55.87 | 47.11 | 10.09 |
| 45 | 16.13 | 0.00 | 40.57 | 28.67 | 47.99 | 3.48 |
| 50 | 4.21 | 0.00 | 37.04 | 23.88 | 69.02 | 18.02 |
| 55 | 0.00 | 0.00 | 31.51 | 41.83 | 50.01 | 3.23 |
| 60 | 0.00 | 0.00 | 34.43 | 37.43 | 20.88 | 2.12 |

According to Example 30, when the sustained and controlled release excipients are insoluble matrix materials (Utrecht S100, calcium alginate, ethyl cellulose), the release curve is relatively stable. In contrast, the excessively strong controlled release ability of Utrecht S100 and calcium alginate, possibly due to their poor water solubility, resulted in a release stagnation in the early stage of drug release. Comparatively, ethyl cellulose showed a good drug release trend.

In the drug release curve with hydrophilic gel material as sustained and controlled release excipient material, PEO N80 exerted a stronger sustained release effect on drugs owing to its higher viscosity and greater hydration degree, leading to an extremely low drug release amount in the early stage. Subsequently, the continuous erosion of the hydration layer on the surface of the drug rod caused an increase in the local drug release at the surface of the formulation. However, most of the drug was still wrapped in the hydration layer formed by PEO, and the release amount gradually decreased again. However, PLGA and HPMC K15M showed no obvious drug release stagnation phenomenon. Obvious swelling phenomenon of the formulation, also referred to as tube swelling phenomenon, can be clearly observed in the process of in vitro release. Considering that the silicone tube requires certain mechanical properties to maintain long-term stable drug release after being implanted in human body, hydrophilic matrix material used alone as sustained and controlled release materials exhibited inferior effects compared with insoluble matrix materials.

| Time (d) | Example 31-1 | Example 31-2 | Example 31-3 | Time (d) | Example 31-1 | Example 31-2 | Example 31-3 |
|---|---|---|---|---|---|---|---|
| 1 | 41.93 | 90.85 | 50.26 | | | | |
| 5 | 48.55 | 110.24 | 48.35 | 35 | 51.64 | 73.99 | 37.47 |
| 10 | 73.64 | 51.32 | 150.05 | 40 | 40.42 | 78.32 | 39.46 |
| 15 | 49.33 | 70.41 | 168.95 | 45 | 50.73 | 73.53 | 35.93 |
| 20 | 47.01 | 69.53 | 58.34 | 50 | 48.52 | 55.25 | 33.11 |
| 25 | 40.34 | 64.71 | 36.68 | 55 | 43.88 | 18.33 | 39.53 |
| 30 | 53.16 | 61.36 | 37.07 | 60 | 25.53 | 2.05 | 36.24 |

From the release curve in the figure below, we can conclude that when the hydrophilic gel matrix material is HPMC K15M, it can interact with ethyl cellulose to achieve an ideal sustained release effect.

(2) The release results (daily release dose μg) of the drug-containing layer of the rod core of single-layer and single-gradient perforated implant are shown in the following table.

| Time (d) | Example 32-1 | Example 32-2 | Example 32-3 | Time (d) | Example 32-1 | Example 32-2 | Example 32-3 |
|---|---|---|---|---|---|---|---|
| 1 | 39.66 | 79.30 | 21.61 | | | | |
| 5 | 76.54 | 95.67 | 31.18 | 35 | 54.36 | 49.52 | 20.51 |
| 10 | 73.77 | 48.71 | 40.35 | 40 | 60.99 | 54.21 | 24.47 |
| 15 | 62.62 | 44.98 | 33.84 | 45 | 40.57 | 47.62 | 31.57 |
| 20 | 74.66 | 45.64 | 30.13 | 50 | 37.04 | 43.28 | 22.38 |
| 25 | 62.07 | 47.98 | 31.82 | 55 | 31.51 | 43.73 | 30.72 |
| 30 | 62.44 | 49.16 | 25.78 | 60 | 34.43 | 30.46 | 23.09 |

As can be seen from the above, when the drug-to-excipient ratio is 1:5, the drug release is stable, with no obvious burst release or stagnation of drug release.

(3) The ratio of insoluble matrix material to hydrophilic gel material in the drug-containing layer of the rod core of single-layer and single-gradient perforated implant was investigated. The release results (daily release dose μg) are shown in the following table.

| Time (d) | Example 33-1 | Example 33-2 | Example 33-3 | Time (d) | Example 33-1 | Example 33-2 | Example 33-3 |
|---|---|---|---|---|---|---|---|
| 1 | 45.86 | 39.45 | 81.35 | | | | |
| 5 | 44.24 | 30.56 | 83.63 | 35 | 46.83 | 32.92 | 60.43 |
| 10 | 41.56 | 35.62 | 87.46 | 40 | 44.84 | 38.44 | 60.05 |
| 15 | 49.90 | 32.09 | 64.95 | 45 | 42.33 | 34.15 | 68.45 |
| 20 | 47.26 | 30.83 | 65.82 | 50 | 49.39 | 30.09 | 68.23 |
| 25 | 47.77 | 33.69 | 64.72 | 55 | 41.28 | 32.63 | 62.81 |
| 30 | 42.03 | 31.81 | 61.19 | 60 | 43.67 | 39.60 | 67.72 |

When EC: HPMC K15M = 2: 1, the drug release curve is relatively stable, when the content of HPMC K15M increases, the drug burst release increases in the early stage.

II. Single-layer and multi-gradient perforated implant

(1) The release results (daily release dose μg) of single-layer and multi-gradient perforated implants in different stages are shown in the following table.

| Time (d) | Example 34-1 | Example 34-2 | Example 34-3 | Example 34-4 |
|---|---|---|---|---|
| 1 | 40.35 | 39.02 | 89.18 | 121.63 |
| 5 | 47.26 | 92.03 | 150.07 | 197.36 |
| 10 | 90.42 | 109.79 | 170.19 | 220.91 |
| 15 | 62.15 | 86.81 | 185.90 | 202.24 |
| 20 | 67.04 | 88.96 | 151.22 | 189.35 |
| 25 | 67.11 | 94.95 | 152.62 | 187.37 |
| 30 | 68.97 | 75.15 | 151.51 | 123.06 |
| 35 | 67.99 | 81.95 | 135.76 | 116.25 |
| 40 | 68.09 | 67.42 | 91.94 | 94.89 |
| 45 | 58.28 | 58.04 | 97.85 | 85.82 |

(continued)

| Time (d) | Example 34-1 | Example 34-2 | Example 34-3 | Example 34-4 |
|---|---|---|---|---|
| 50 | 58.44 | 64.65 | 68.19 | 49.09 |
| 55 | 52.80 | 63.59 | 71.34 | 45.54 |
| 60 | 56.71 | 47.57 | 63.98 | 41.49 |

III. Double-layer implant

(1) The release results (daily release dose μg) of the drug-containing layer of the rod core of the double-layer perforated implant are shown in the following table.

| Time (d) | Example 35-1 | Example 35-2 | Example 35-3 | Example 35-4 |
|---|---|---|---|---|
| 1 | 10.22 | 10.35 | 12.03 | 20.67 |
| 5 | 50.83 | 19.79 | 7.13 | 45.26 |
| 10 | 5.79 | 7.22 | 13.57 | 42.09 |
| 15 | 17.41 | 14.93 | 13.32 | 52.79 |
| 20 | 17.85 | 0.00 | 9.72 | 52.40 |
| 25 | 6.44 | 0.00 | 17.35 | 59.65 |
| 30 | 0.00 | 0.00 | 18.01 | 50.94 |
| 35 | 0.00 | 0.00 | 16.13 | 42.91 |
| 40 | 0.00 | 0.00 | 16.62 | 47.69 |
| 45 | 0.00 | 0.00 | 8.18 | 18.90 |
| 50 | 0.00 | 0.00 | 5.10 | 20.33 |
| 55 | 0.00 | 0.00 | 0.00 | 29.60 |
| 60 | 0.00 | 0.00 | 0.00 | 9.02 |

Both PEO WSR-303 and PEO WSRN-750 have a good stable release, but the high viscosity of PEO WSR303 in the drug-containing layer may affect the appearance of the whole implant.

(2) The release results (daily release dose μg) of the sustained and controlled release excipients and fillers in the drug-containing layer of the rod core of the double-layer perforated implant are shown in the following table.

| Time (d) | Example 36-1 | Example 36-2 | Example 36-3 | Time (d) | Example 36-1 | Example 36-2 | Example 36-3 |
|---|---|---|---|---|---|---|---|
| 1 | 50.03 | 39.67 | 20.67 | | | | |
| 5 | 65.27 | 40.26 | 45.26 | 35 | 17.25 | 32.91 | 42.91 |
| 10 | 68.02 | 40.09 | 42.09 | 40 | 23.30 | 31.34 | 47.69 |
| 15 | 16.33 | 43.74 | 52.79 | 45 | 18.14 | 40.00 | 18.90 |
| 20 | 24.44 | 44.95 | 52.40 | 50 | 16.59 | 33.01 | 20.33 |
| 25 | 23.80 | 41.63 | 59.65 | 55 | 10.73 | 27.83 | 29.60 |
| 30 | 20.27 | 33.19 | 50.94 | 60 | 1.02 | 20.93 | 9.02 |

The results showed that when the amount of PEOWSN-750 was 20mg, the drug could not be fully suspended, and the drug release power was insufficient in the later period, however, with the increase of the amount of PEOWSN-750, the drug release rate was slow, and when the amount of PEOWSN-750 was 60mg, the drug release rate decreased.

(3) The release results (daily release dose μg) of the expandable materials in the booster layer of the rod core of the double-layer perforated implant are shown in the following table.

| Time (d) | Example 37-1 | Example 37-2 | Example 37-3 | Time (d) | Example 37-1 | Example 37-2 | Example 37-3 |
|---|---|---|---|---|---|---|---|
| 1 | 36.89 | 50.55 | 39.67 | | | | |
| 5 | 37.44 | 54.35 | 40.26 | 35 | 42.13 | 34.43 | 32.91 |
| 10 | 37.28 | 54.12 | 40.09 | 40 | 45.42 | 42.31 | 31.34 |
| 15 | 48.12 | 59.05 | 43.74 | 45 | 45.43 | 54.07 | 40.08 |
| 20 | 49.26 | 60.68 | 44.95 | 50 | 49.24 | 44.56 | 33.01 |
| 30 | 47.39 | 44.81 | 33.19 | 60 | 28.02 | 30.26 | 20.93 |

The results show that the change of molecular weight of PEO in the selected range has no significant effect on drug release, in addition, with the increase of molecular weight of PEO, the greater the viscosity, the better the formability of excipient powder.

(4) The release results (daily release dose μg) of the expandable material in the booster layer of the rod core of the double-layer perforated implant are shown in the following table.

| Time (d) | Example 38-1 | Example 38-2 | Example 38-3 | Time (d) | Example 38-1 | Example 38-2 | Example 38-3 |
|---|---|---|---|---|---|---|---|
| 1 | 10.63 | 39.67 | 20.74 | | | | |
| 5 | 20.83 | 40.26 | 45.02 | 35 | 16.93 | 32.91 | 29.34 |
| 10 | 20.72 | 40.09 | 40.21 | 40 | 18.53 | 31.34 | 31.24 |
| 15 | 16.77 | 43.74 | 30.94 | 45 | 14.55 | 40.08 | 22.66 |
| 20 | 16.31 | 44.95 | 33.60 | 50 | 6.01 | 33.01 | 20.21 |
| 25 | 13.41 | 41.63 | 26.97 | 55 | 7.10 | 27.83 | 23.95 |
| 30 | 14.81 | 33.19 | 33.62 | 60 | 4.22 | 20.93 | 20.61 |

The amount of PEO WSR N60K in the selected range has no significant effect on drug release.

While specific embodiments of the present invention have been described above, those skilled in the art should understand that these are merely illustrative examples, and various changes or modifications can be made to these embodiments without departing from the principles and essence of the present disclosure. Therefore, the scope of protection of in the present disclosure is defined by the appended claims.

## Claims

**1.** A raw material composition of silicone material, comprising the following components in parts by weight:

R-vinyl polysiloxane: 100 parts;
a reinforcing agent: 10-80 parts;
a catalyst: 0.000002-1 part;
wherein, R in R-vinyl polysiloxane is a substituted or unsubstituted $C_1$-$C_5$ straight-chain alkane or branched-chain alkane, a substituted or unsubstituted $C_6$-$C_{20}$ aromatic hydrocarbon;
the vinyl content in the R-vinyl polysiloxane is 0.1 mol%-0.5 mol%;
the components in the reinforcing agent contains hydrophilic groups;
the Si-H group content in the hydrogen-containing silicone oil is 0.18-1.6 mol%;
the molar ratio of Si-H groups in the hydrogen-containing silicone oil to the vinyl groups in R-vinyl polysiloxane is (0.5-10):1;
optionally, further comprising an inhibitor, which is a substance that can inhibit the addition reaction between the R- vinyl polysiloxane and the hydrogen-containing silicone oil.

**2.** The raw material composition of silicone material according to claim 1, wherein, the raw material composition of silicone material satisfies one or more of the following conditions a-n:

a. R in R-vinyl polysiloxane is a substituted or unsubstituted $C_1$-$C_5$ straight-chain alkane, for example, methyl;
b. the vinyl content in the R-vinyl polysiloxane is 0.15 mol%-0.3 mol%, for example, 0.18 mol% or 0.23 mol%;
c. the reinforcing agent is 20-70 parts by weight, for example, 30 parts, 40 parts, 45 parts or 50 parts;
d. the water contact angle of the reinforcing agent is ≤90°, for example, 14-90°, 16-35° or ≤25°;
e. the hydrophilic groups are one or more selected from the group consisting of hydroxyl group, mercapto group, aldehyde group, ketone group, ether bond, block polyether group, carboxyl group, carboxyl ester group, sulfate group, sulfone group, sulfinate group, sulfonate group, sulfonamide group, sulfonate ester group, thioether group, phosphate group, phosphoramide group, phosphinimide group, phosphate ester group, phosphite ester group, phosphino group, amino group, imino group, tertiary amino group, quaternary ammonium group, cyano group, cyanate ester group, thiocyanate group, amide group, and other oxygen-containing groups capable of forming hydrogen bonds with water molecules;
f. the reinforcing agent is one or more selected from the group consisting of silica, hydroxyapatite, diatomaceous earth, quartz powder, silica micropowder, aluminum hydroxide, magnesium hydroxide, aluminum oxide, magnesium oxide, titanium dioxide, magnesium silicate, carbon black, zinc oxide, titanium dioxide, clay, lignin, carbonates, and kaolin; the silica is preferably hydrophilic silica or a mixture of "hydrophilic silica and hydrophobic silica"; the hydrophilic silica is preferably hydrophilic fumed silica, for example, one or more selected from the group consisting of hydrophilic fumed silica N20, hydrophilic silica TH-a200,, hydrophilic fumed silica A380, hydrophilic fumed silica A300, T30 hydrophilic fumed silica, T40 hydrophilic fumed silica, and V15 hydrophilic fumed silica;
g. the amount of the hydrogen-containing silicone oil is 0.3-5 parts, preferably 0.3-3 parts, for example, 1.01 parts, 1.07 parts, 1.33 parts, 1.60 parts or 1.70 parts;
h. the molar ratio of Si-H groups in the hydrogen-containing silicone oil to the vinyl groups in R-vinyl polysiloxane is (0.5-8):1, preferably (0.8-3):1, for example, 0.8:1, 1:1, 1.2:1, 1.5:1, 1.8:1, 2:1 or 3:1;
i. the Si-H group content in the hydrogen-containing silicone oil is 0.15-1.6 mol%, preferably 0.15-1.2 mol%, for example, 0.75 mol%;
j. the catalyst is a platinum catalyst; wherein, the concentration of platinum in the platinum catalyst is preferably $1\times10^3$-$1\times10^5$ ppm, for example, $1\times10^3$ ppm, $1\times10^4$ ppm, where ppm means that the mass concentration of platinum in the platinum catalyst is one part per million;
k. the amount of the catalyst used is 0.00001-0.5 parts, for example, 0.00001 parts, 0.0098 parts or 0.21 parts;
l. the inhibitor is an alkyne alcohol compound, a nitrogen-containing compound, or an organic peroxide, for example, methylbutynol, and for another example, 2-methyl-3-butyn-2-ol;
m. the inhibitor is 0.03-2.0 parts by weight, for example, 0.69 parts;
n. the raw material composition of the silicone material may comprise the following components: methyl vinyl polysiloxane: 100 parts; the vinyl content in methyl vinyl polysiloxane is 0.18 mol%; hydrophilic silica: 30 parts; hydrogen-containing silicone oil: 1.33 parts; the molar ratio of Si-H groups in the hydrogen-containing silicone oil to vinyl groups in methyl vinyl polysiloxane is 1.5:1; platinum catalyst (concentration of 3000 ppm): 0.0098 parts; 2-methyl-3-butyn-2-ol: 0.69 parts;

or, the raw material composition of the silicone material may comprise the following components: methyl vinyl polysiloxane: 100 parts; the vinyl content in methyl vinyl polysiloxane is 0.18 mol%; hydrophilic silica: 30 parts; hydrogen-containing silicone oil: 1.33 parts; the molar ratio of Si-H groups in the hydrogen-containing silicone oil to vinyl groups in methyl vinyl polysiloxane is 1.5:1; platinum catalyst (concentration of 3000 ppm): 0.01 parts; 2-methyl-3-butyn-2-ol: 0.69 parts;
or, the raw material composition of the silicone material may comprise the following components: methyl vinyl polysiloxane: 100 parts; the vinyl content in methyl vinyl polysiloxane is 0.18 mol%; hydroxyapatite: 20 parts; HB-615 hydrophobic fumed silica: 20 parts; hydrogen-containing silicone oil: 1.33 parts; the molar ratio of Si-H groups in the hydrogen-containing silicone oil to vinyl groups in methyl vinyl polysiloxane is 1.5:1; platinum catalyst (concentration of 3000 ppm): 0.21 parts; 2-methyl-3-butyn-2-ol: 0.98 parts;
or, the raw material composition of the silicone material may comprise the following components: methyl vinyl polysiloxane: 100 parts; the vinyl content in methyl vinyl polysiloxane is 0.17 mol%; hydrophilic fumed silica N20: 20 parts; hydrophobic fumed silica R106: 20 parts; Si-H group content of hydrogen-containing silicone oil is 0.75%; the molar ratio of Si-H groups in the hydrogen-containing silicone oil to vinyl groups in methyl vinyl polysiloxane is 1.2:1; platinum catalyst: 0.00001 parts; 2-methyl-3-butyn-2-ol: 0.7 parts.

**3.** A method for preparing a silicone material, comprising the following steps:

S1. mixing R-vinyl polysiloxane and a reinforcing agent to obtain mixture A;
S2. dividing the mixture A into component A1 and component A2, mixing the component A1 and hydrogen-containing silicone oil to obtain component B1, and mixing the component A2 and a catalyst to obtain component B2; optionally, the component B1 further comprises an inhibitor;
S3. mixing the component B1 and the component B2 to obtain mixture B, and carrying out catalytic addition to obtain the silicone material;
wherein, preferably, the method for preparing the silicone material satisfies one or more of the following conditions:

(i) the reinforcing agent is pretreated by drying at 90-220°C for 1-24 hours; for example, drying at 130°C for 24 hours;
(ii) the R-vinyl polysiloxane is pretreated by drying at 20-90°C for 1-24 hours; for example, drying at 40°C for 12 hours;
(iii) in step S1, the mixing step involves adding the reinforcing agent in small portions multiple times to the R-vinyl polysiloxane for kneading, then subjecting to extrusion and thin-pass processing;
(iv) in step S2, the preparation method of component B1 involves refining component A1 in an open mill with a roller distance parameter of 1 unit, and adding the hydrogen-containing silicone oil and the inhibitor dropwise, then setting different roller distances after addition, performing triangular packing, and thin-pass processing, and then taking out and allowing to stand for 24 hours;
(v) in step S2, the preparation method of component B2 involves refining component A2 in an open mill with a roller distance parameter of 1 unit, adding the catalyst dropwise, continuously shortening the roller distance, performing thin-pass processing, and then taking out and allowing to stand for 24 hours;
(vi) in step S3, the mass ratio of the component B1 to the component B2 in mixture B is 1:1;
(vii) in step S3, the preparation method of the mixture B involves placing component B1 and component B2 in an open mill for mixing and extrusion, and performing thin-pass processing for 6 times to obtain the mixture.
(viii) in step S3, the catalytic addition step involves sequentially subjecting the mixture B to a first sulfidation treatment and a second sulfidation treatment, preferably further comprising a third sulfidation treatment; wherein, the temperature of the first sulfidation treatment is preferably 200°C-340°C, for example, 250°C; the time of the first sulfidation treatment is preferably 2s-30s; wherein, the first sulfidation treatment is preferably carried out in a vertical hot air sulfidation channel; the temperature of the second sulfidation treatment is preferably 180°C-280°C, for example, 220°C, 240°C, 260°C or 280°C; the time of the second sulfidation treatment is preferably 0.1min-5min; the second sulfidation treatment is preferably carried out in a horizontal hot air sulfidation channel; the temperature of the third sulfidation treatment is preferably 150°C-240°C, for example, 180°C.

**4.** A silicone material, which is prepared by the preparation method according to claim 3.

**5.** A method for preparing a silicone tube, comprising forming the mixture B according to claim 3 into a tube by extrusion and then carrying out catalytic addition to obtain the silicone tube;
or, carrying out the catalytic addition process described above in a tubular mold to obtain the silicone tube.

**6.** A silicone tube, which is prepared by the method according to claim 5.

**7.** An implant, comprising a drug core and the silicone tube according to claim 6, the drug core contains the pharmaceutically active ingredient.

**8.** A use of the silica material according to claim 4 or the silicone tube according to claim 6 as a release rate regulating medium in a sustained and controlled release formulation.

**9.** A rod core for a perforated implant, comprising a drug-containing layer, the drug-containing layer contains an active drug and excipients;

wherein, the excipients comprise sustained and controlled release excipients; the molecular weight of the sustained and controlled release excipients is 1000-10,000,000;
the drug-to-excipient ratio is 1:(0.01-100); the drug-to-excipient ratio represents the mass ratio of the active drug to the excipients in the drug-containing layer;
optionally, the rod core of the perforated implant further comprises a booster layer composited on the drug-containing layer.

**10.** The rod core for a perforated implant according to claim 9, wherein, the active drug comprises an active drug with a drug solubility > 10 mg/mL and a drug molecular weight greater than 800 Da, preferably comprises an active drug with a drug solubility > 30 mg/mL and a drug molecular weight greater than 800 Da, for example, one or more selected from the group consisting of exenatide, semaglutide, total asiaticoside and human growth hormone;

and/or, the active drug comprises an active drug with a drug solubility > 10 mg/mL and a drug molecular weight less than or equal to 800 Da, preferably comprises an active drug with a drug solubility > 30 mg/mL and a drug molecular weight less than or equal to 800 Da, for example, metformin and/or doxorubicin hydrochloride;
and/or, the active drug comprises an active drug with a drug solubility ≤10 mg/mL and a drug molecular weight greater than 800 Da, preferably comprises an active drug with a drug solubility ≤5 mg/mL and a drug molecular weight greater than 800 Da, for example, one or more selected from the group consisting of bovine insulin, paclitaxel and levothyroxine sodium;
and/or, the active drug comprises an active drug with a drug solubility ≤10 mg/mL and a drug molecular weight ≤800 Da, preferably comprises an active drug with a drug solubility ≤5 mg/mL and a drug molecular weight ≤800 Da, for example, one or more selected from the group consisting of estradiol, pregnardene, levonorgestrel, meloxicam, ganciclovir, puerarin and paliperidone;
and/or, the weight-average molecular weight of the sustained and controlled release excipients is 1,100 - 8,000,000, for example, 1,171, 10,000, 44,000, 200,000, 300,000, 400,000 or 7,000,000;
the sustained and controlled release excipients preferably comprise an insoluble matrix material and/or a hydrophilic gel material; the insoluble matrix material preferably has a weight-average molecular weight of 2,000-8,000,000, more preferably 3,000-7,000,000; more preferably comprise one or more selected from the group consisting of calcium alginate, methylcellulose, polyethylene, polyvinyl chloride, methacrylic acid-methyl methacrylate copolymer, and ethyl cellulose, for example, ethyl cellulose; the methacrylic acid-methyl methacrylate copolymer is preferably one or more selected from the group consisting of Eutec S100, Eutec L100, RL, RS, NE, and FS, for example, Eutec S100; the hydrophilic gel material preferably has a weight-average molecular weight of 1,000-8,000,000, more preferably comprise one or more selected from the group consisting of hydroxypropyl methylcellulose, polyoxyethylene, sodium alginate, chitosan, agar, xanthan gum, carbomer, polyvinyl alcohol, N-vinylamide polymers, polylactic acid-glycolic acid copolymer (PLGA), and polyethylene oxide; the hydroxypropyl methylcellulose is preferably one or more selected from the group consisting of HPMC·K100M, HPMC K15M, and HPMC K4M; the N-vinylamide polymer is preferably PVP K30 and/or PVPK90; the polyethylene oxide is preferably one or more selected from the group consisting of PEO N80, PEO N-750, PEO WSR 303, PEO WSR N-12K, PEO WSR N-60K, and PEO WSR 1105;
and/or, the excipients further comprise one or more selected from the group consisting of drug stabilizers, fillers and suspending agents;
and/or, the drug-to-excipient ratio is 1:(0.01-99), for example, 1:0.01, 1:0.05, 1:0.1, 1:0.2, 1:0.5, 1:1, 1:1.5, 1:3, 1:4.33, 1:5, 1:7, 1:10, 1:20, 1:40, 1:60 or 1:80;
and/or, the mass ratio of the sustained and controlled release excipients to the excipients is 1:(0.01-100), for example, 1:0.01, 1:0.05, 1:0.1, 1:0.2, 1:0.5, 1:1, 1:1.5, 1:3, 1:4.33, 1:5, 1:7, 1:10, 1:20, 1:40, 1:60, 1:80 or 1:100;
and/or, the concentration of the active drug in the drug-containing layer is set as a gradient concentration; the gradient is preferably 1-50 segments, for example, 2 segments, 3 segments, 5 segments, 10 segments, 20 segments, or 40 segments; the gradient concentration is generally expressed such that the concentration of each gradient is independent;
and/or, the raw materials of the booster layer comprise expandable materials and penetration enhancer;
and/or, when the rod core further comprises a booster layer, the mass ratio of the drug-containing layer to the booster layer is (0.01-100):1, for example, 0.01:1, 0.02:1, 0.05:1, 0.1:1, 0.2:1, 0.5:1, 1:1, 2:1, 3:1, 5:1, 10:1, 20:1, 40:1, 60:1, 80:1 or 100:1;
and/or, when the rod core further comprises a booster layer, the number of booster layers is an integer, preferably an even number, for example, 2; when the number of booster layers is an even number, the booster layers are preferably respectively disposed at both ends of the drug-containing layer;
and/or, the single pressing length of the rod core is 1-50mm, for example, 1mm or 2mm or 3mm or 4mm or 5mm or 7mm or 10mm or 20mm or 30mm or 40mm or 50mm; the total length of the rod core can be selected according to the length of the silicone tube;
and/or, the diameter of the rod core is 1-10 mm, more preferably 1-5 mm, for example, 2 mm or 3 mm or 4 mm.

**11.** The rod core for a perforated implant according to claim 9 or 10, wherein, when the gradient is 3 segments, the gradient is sequentially configured as a first gradient drug release module, a second gradient drug release module, and a third gradient drug release module; the concentration of the active drug in the first gradient drug release module is 0.1%-50%, for example, 2%, 5%, or 10%; the concentration of the active drug in the second gradient drug release

module is 1%-80%, for example, 10% or 20%; the concentration of the active drug in the third gradient drug release module is 1%-100%, for example, 20% or 30%; the concentration of the active drug represents the percentage of the mass of the active drug relative to the mass of the gradient drug release module;

and/or, the expandable material in the booster layer is polyethylene oxide, preferably polyethylene oxide with a weight-average molecular of 10-7,000,000, for example, one or more selected from the group consisting of PEO N-80, PEO N-750, PEO WSR 1105, PEO WSR 303, PEO N-12K and PEO N-60K;
and/or, the permeation enhancer in the booster layer is one or more selected from the group consisting of mannitol, lactose, fructose, glucose, and NaCl, for example, NaCl;
wherein, the mass ratio of the expandable material and the permeation enhancer in the booster layer is preferably (0.01-100):1, for example, 0.01:1, 0.02:1, 0.05:1, 0.1:1, 0.2:1, 0.5:1, 1:1, 2:1, 3:1, 4:1, 5:1, 10:1, 20:1, 40:1, 60:1, 80:1 or 100:1.

**12.** A method for preparing the rod core for a perforated implant according to any one of claims 9-11, comprising the following steps: pressing the active drug and the excipients to obtain the drug-containing layer;

optionally, compositing the booster layer onto the drug-containing layer;
preferably, the pressing pressure is 0.01-10 MPa, for example, 0.1, 0.2, 0.3, 0.5, 1, 2, 5 or 10 MPa.

**13.** A perforated implant, comprising the rod core for a perforated implant according to any one of claims 9-11 and a silicone tube with drug release holes covering the outside of the rod core;

wherein, preferably, in the silicone tube with drug release holes, the ratio of the diameter of the drug release holes to the outer diameter of the silicone tube is (0.0001-0.5):1, preferably (0.002-0.4):1, for example, 0.06:1, 0.14:1 or 0.23:1, more preferably (0.005-0.2):1;
and/or, in the silicone tube with drug release holes, the ratio of the total area of the drug release holes to the outer surface area of the silicone tube is (0.00001-0.4):1, preferably (0.0001-0.35):1, more preferably (0.0005-0.3):1, for example, 0.0005:1, 0.001:1, 0.005:1, 0.01:1, 0.05:1, 0.1:1 or 0.2:1;
and/or, the diameter of the drug release holes is 1μm-1000μm, preferably 10-600μm, for example, 10, 20, 50, 100, 150, 250, 350, 450 or 550μm, more preferably 250-450μm;
and/or, the number of the drug release holes is >1, preferably 1-1,000,000, for example, 1, 2, 5, 10, 20, 50, 100, 200, 500, 1,000, 5,000, 10,000, 50,000, 100,000, 500,000 or 1,000,000;
and/or, the silicone tube with drug release holes is obtained by drilling holes in the silicone tube using laser drilling or mechanical drilling;
and/or, the position of the drug release holes satisfies the following conditions: the silicone tube includes a first end surface and a second end surface disposed opposite to each other, the distance between the first end surface and the second end surface is the total length of the silicone tube, and a plurality of drug release holes are provided in the silicone tube;
wherein, when the number of the drug release holes is one, the distance from the center point of the drug release hole to the first end surface of the silicone tube with drug release holes is preferably 0-100% of the length of the silicone tube, but not 0 and 100%, more preferably the distance from the center point of any of the drug release holes to the first end surface of the silicone tube with drug release holes is 0.1%-99.9% of the length of the silicone tube, more preferably 1%-99%, for example, 1%, 2%, 3%, 5%, 7%, 7.5%, 10%, 20%, 30%, 40%, 50%, 60%, 70%, 80%, 90%, 95%, 98% or 99%;
wherein, when the number of drug release holes is ≥2, the distance between the center points of the drug release holes preferably satisfies the following conditions:

when the planes of any two of the drug release holes are not on the same radial cross-section of the silicone tube, in the axial cross-section direction of the silicone tube, the distance from the center point of any of the drug release holes to the first end surface of the silicone tube with the drug release holes is preferably 0-100% of the length of the silicone tube, but not 0 and 100%, preferably the distance from the center point of any of the drug release holes to the first end surface of the silicone tube with the drug release holes is 0.1%-99.9% of the length of the silicone tube, more preferably 1%-99%, for example, 1%, 2%, 3%, 5%, 7%, 7.5%, 10%, 20%, 30%, 40%, 50%, 60%, 70%, 80%, 90%, 95%, 98% or 99%; the distance between the center points of adjacent drug release holes is preferably 0.001-80 mm;
wherein, when the number of drug release holes is ≥2, the distance between the center points of the drug release holes can also satisfy the following conditions:

when the planes of any two of the drug release holes are on the same radial cross-section of the silicone tube, the distance from the center point of any of the drug release holes to the first end surface of the silicone tube with drug release holes is preferably 0-100% of the length of the silicone tube, but not 0 and 100%, preferably the distance from the center point of any of the drug release holes to the first end surface of the silicone tube with drug release holes is 0.1 %-99.9% of the length of the silicone tube, more preferably 1%-99%, for example, 1%, 2%, 3%, 5%, 7%, 7.5%, 10%, 20%, 30%, 40%, 50%, 60%, 70%, 80%, 90%, 95%, 98% or 99%; the circumferential distance between the center points of adjacent drug release holes is preferably (2-10)$\pi$ mm.

**14.** A method for preparing the perforated implant according to claim 13, comprising the following steps: filling the rod core of the perforated implant according to any one of claims 9-11 into a silicone tube with drug release holes.

**15.** A use of the rod core of a perforated implant according to any one of claims 9-11 or the perforated implant according to claim 13 as a release rate regulating medium in a sustained and controlled release formulation.

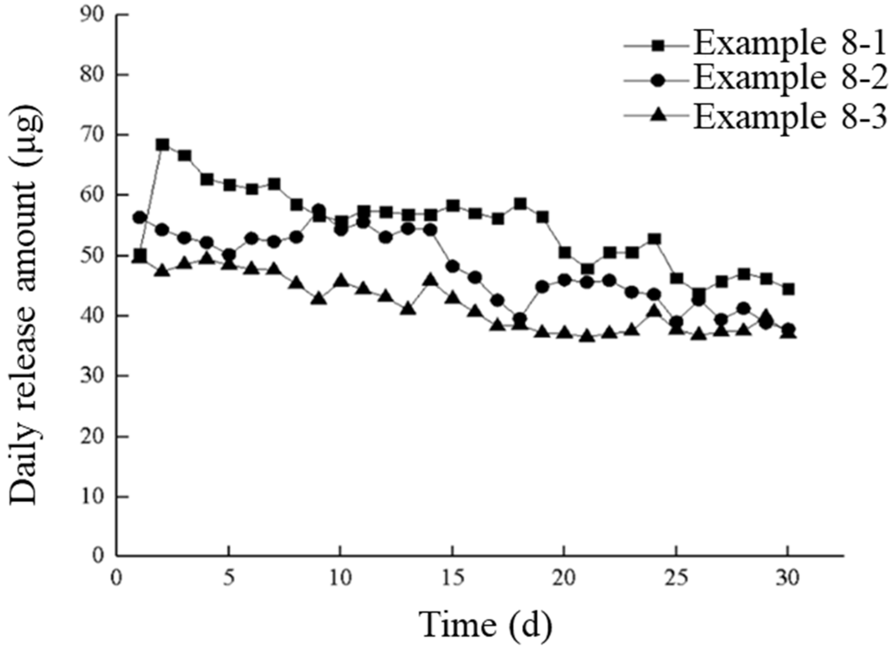
Example 8-1
Example 8-2
Example 8-3
Daily release amount (μg)
Time (d)
0
10
20
30
40
50
60
70
80
90
0
5
10
15
20
25
30

FIG. 1

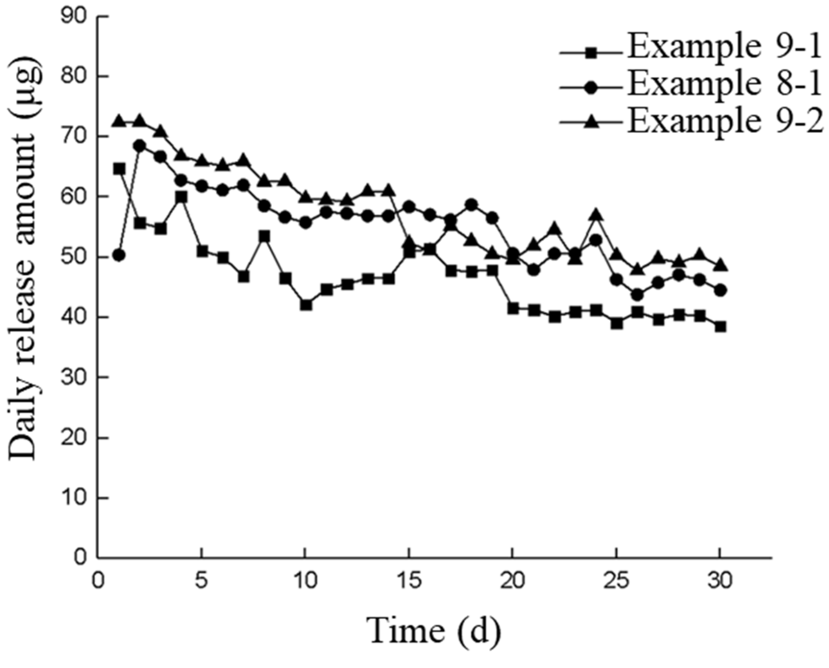
Example 9-1
Example 8-1
Example 9-2
Daily release amount (μg)
Time (d)
0
10
20
30
40
50
60
70
80
90
0
5
10
15
20
25
30

FIG. 2

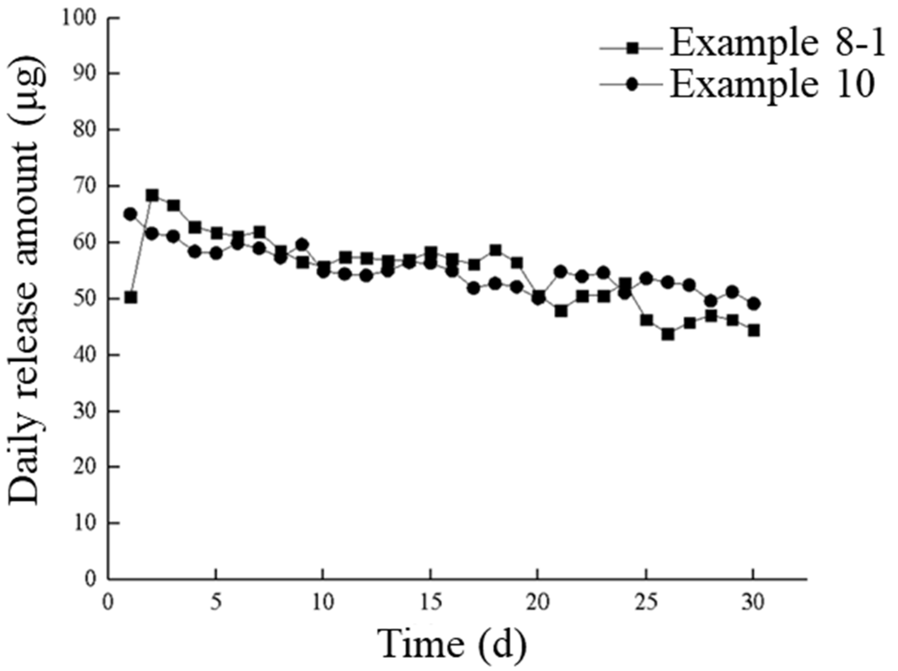
Daily release amount (μg)
Time (d)
Example 8-1
Example 10
0
10
20
30
40
50
60
70
80
90
100
0
5
10
15
20
25
30

FIG. 3

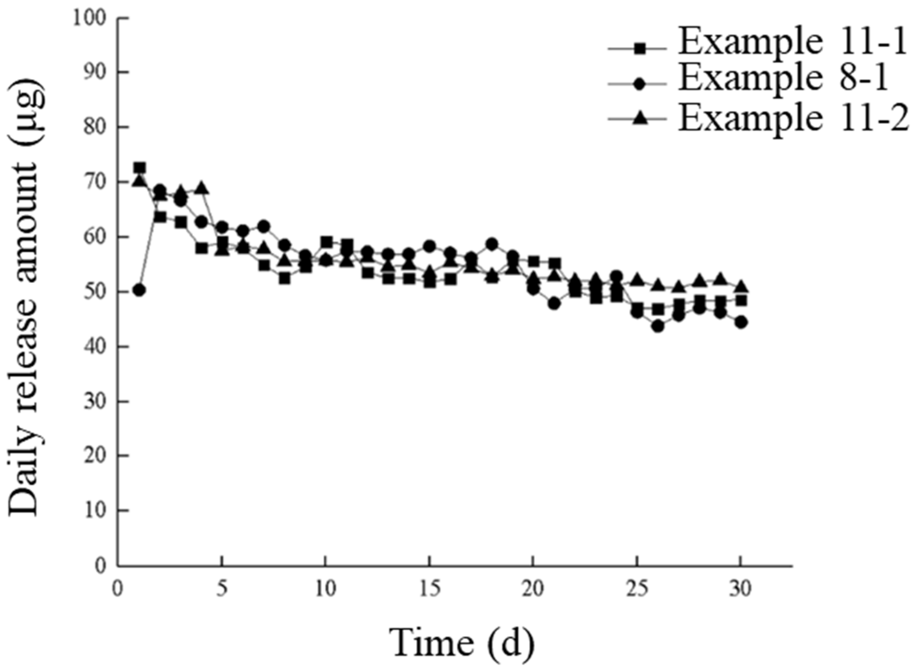
Daily release amount (μg)
Time (d)
Example 11-1
Example 8-1
Example 11-2
0
10
20
30
40
50
60
70
80
90
100
0
5
10
15
20
25
30

FIG. 4

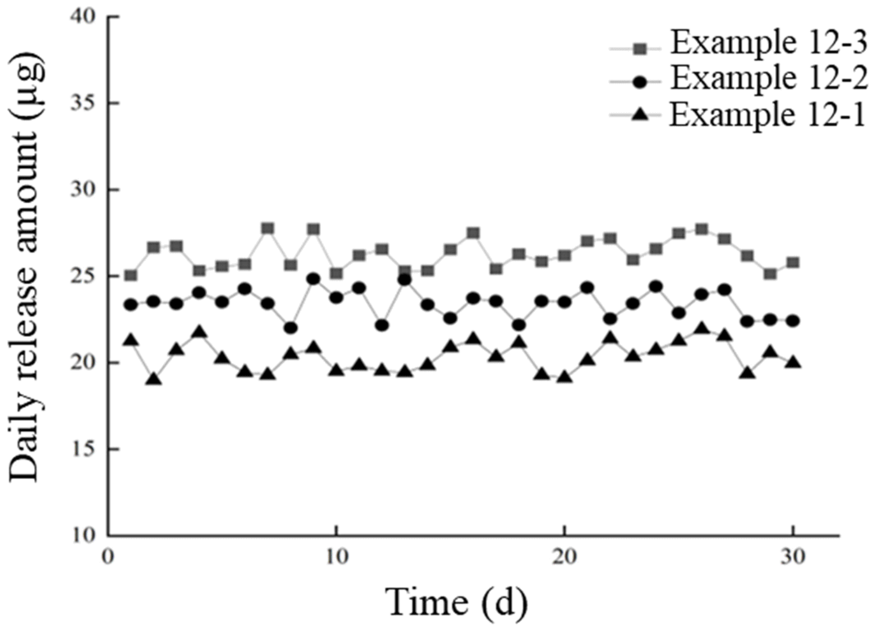
Example 12-3
Example 12-2
Example 12-1
Daily release amount (μg)
Time (d)
40
35
30
25
20
15
10
0
10
20
30

FIG. 5

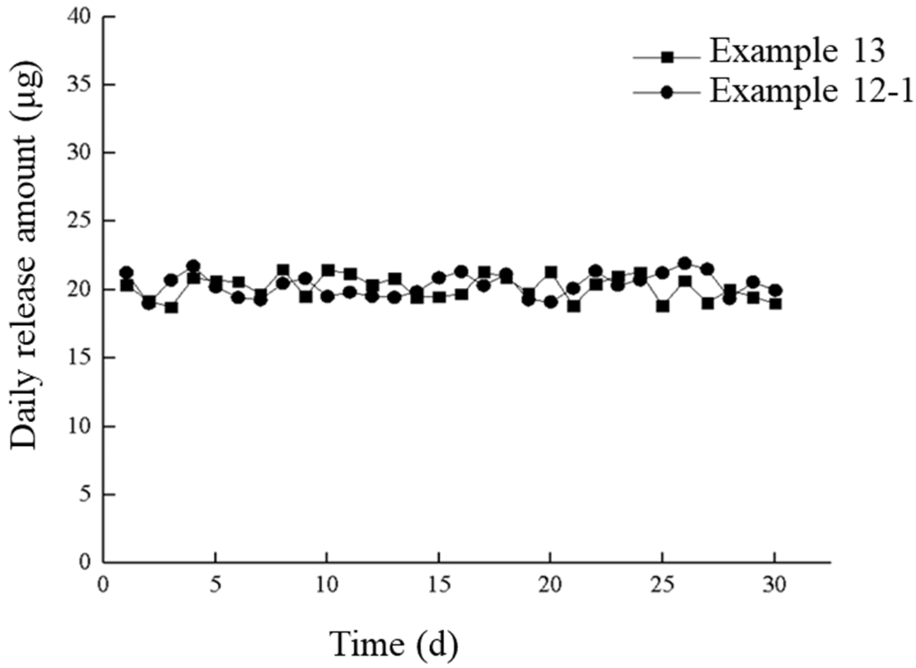
Example 13
Example 12-1
Daily release amount (μg)
Time (d)
40
35
30
25
20
15
10
5
0
0
5
10
15
20
25
30

FIG. 6

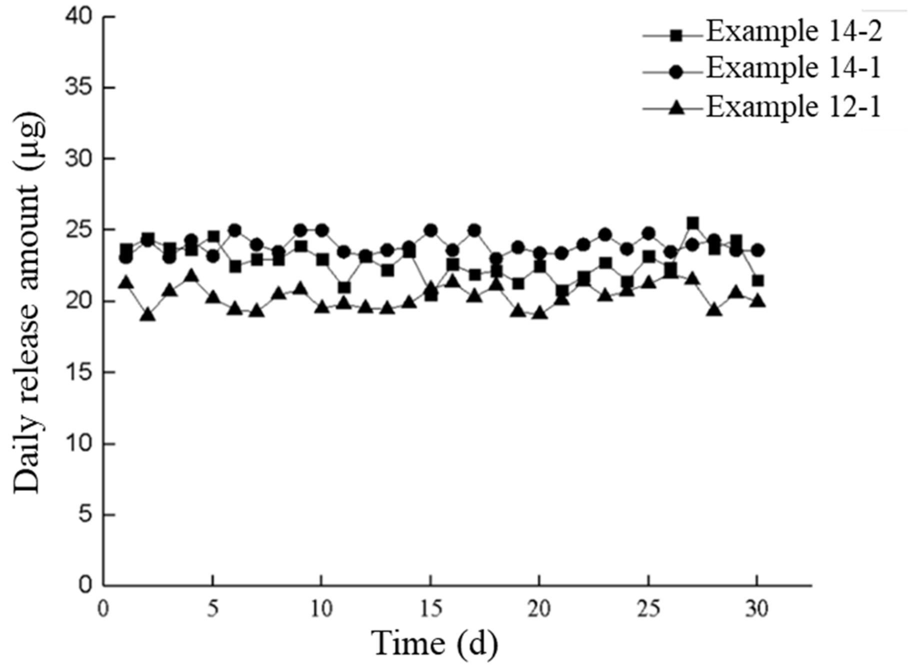


FIG. 7

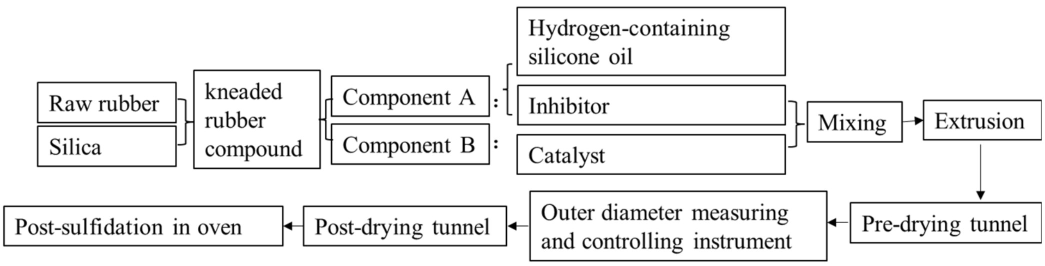


FIG. 8

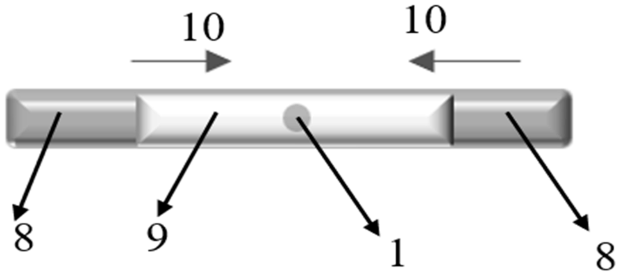


FIG. 9

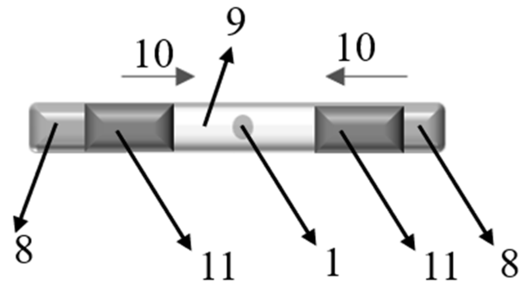


FIG. 10

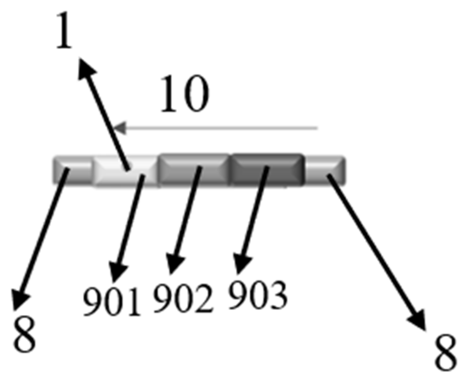


FIG. 11

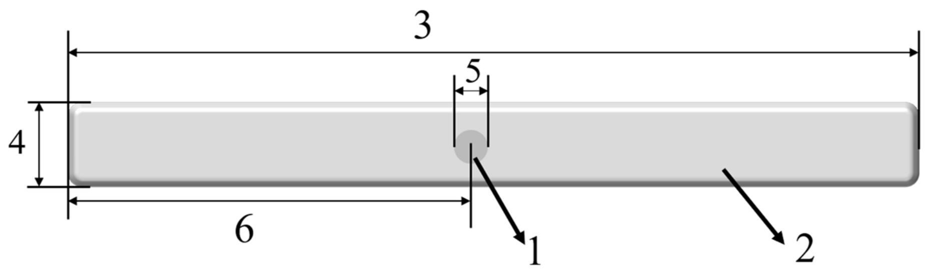


FIG. 12

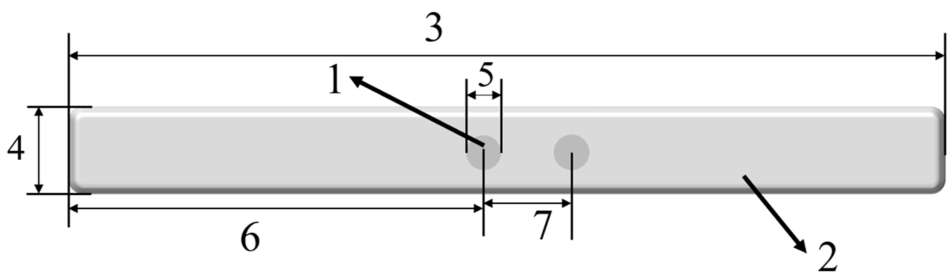


FIG. 13

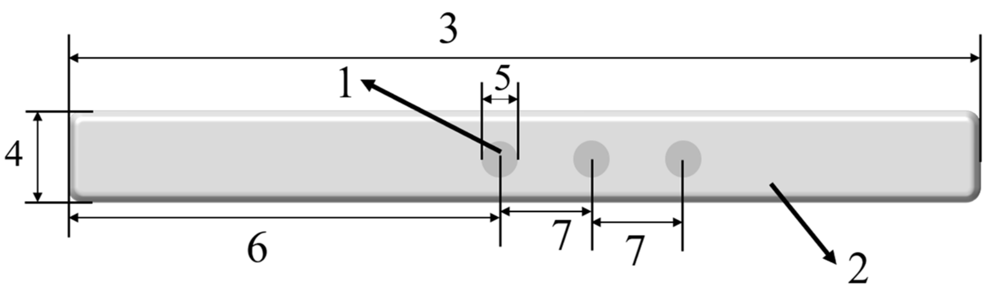


FIG. 14

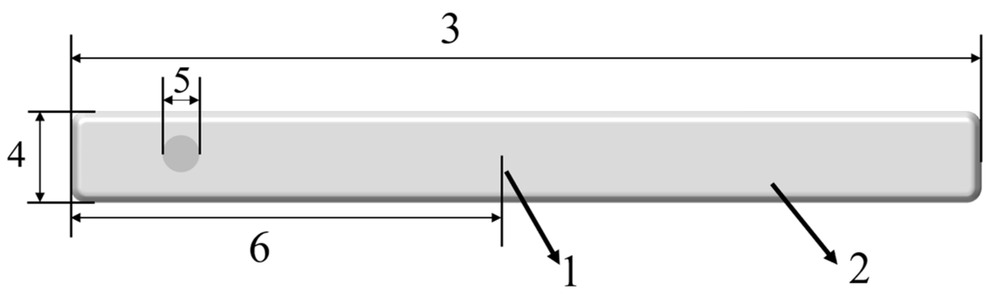


FIG. 15

## INTERNATIONAL SEARCH REPORT

International application No.
**PCT/CN2024/118882**

**A. CLASSIFICATION OF SUBJECT MATTER**

C08L83/07(2006.01)i; C08L83/05(2006.01)i; A61L31/06(2006.01)i; A61L31/16(2006.01)i; A61K47/34(2017.01)i; A61K9/00(2006.01)i; A61K31/00(2006.01)i; A61J3/00(2006.01)i

According to International Patent Classification (IPC) or to both national classification and IPC

**B. FIELDS SEARCHED**

Minimum documentation searched (classification system followed by classification symbols)

IPC:C08L,A61L,A61K,A61J

Documentation searched other than minimum documentation to the extent that such documents are included in the fields searched

Electronic data base consulted during the international search (name of data base and, where practicable, search terms used)

CNTXT, ENTXTC, DWPI, ISI Web of Science, 万方, WANFANG, CNKI: 乙烯基硅橡胶, 硅生胶, 生胶, 含氢硅油, SiH, 亲水, 填料, 补强, 白炭黑, 羟基磷灰石, 艾塞那肽, 棒芯, 雌二醇, 二甲双胍, 葛根素, 更昔洛韦, 积雪草总苷, 埋植剂, 美洛昔康, 帕利哌酮, 片芯, 人生长激素, 索玛鲁肽, 盐酸阿霉素, 药芯, 孕二烯酮, 植入剂, 左炔诺孕酮, silicone, implant, vinyl, Si-H, hydrophilic filler, white carbon black, hydroxyapatite, drug core, rod core, tablet core

**C. DOCUMENTS CONSIDERED TO BE RELEVANT**

| Category* | Citation of document, with indication, where appropriate, of the relevant passages | Relevant to claim No. |
|---|---|---|
| PX | WO 2023174450 A2 (SHENYANG XINGHUA PHARMACEUTICAL TECHNOLOGY CO., LTD.) 21 September 2023 (2023-09-21)<br>description, page 2, paragraph 6 to page 31, last paragraph | 1-15 |
| X | CN 1727409 A (SHANGHAI RUBBER PRODUCTS RESEARCH INSTITUTE) 01 February 2006 (2006-02-01)<br>description, page 3, paragraph 1 to page 5, paragraph 2, and embodiments 1-4, and figure 1 | 1-8 |
| Y | CN 1727409 A (SHANGHAI RUBBER PRODUCTS RESEARCH INSTITUTE) 01 February 2006 (2006-02-01)<br>description, page 3, paragraph 1 to page 5, paragraph 2, and embodiments 1-4, and figure 1 | 13-15 |
| X | CN 101161242 A (BEIJING HONGLIN PHARMACEUTICAL CO., LTD.) 16 April 2008 (2008-04-16)<br>description, page 3, paragraph 4 to page 11, paragraph 7 | 9-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 December 2024** | **31 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/118882

C. DOCUMENTS CONSIDERED TO BE RELEVANT

| Category* | Citation of document, with indication, where appropriate, of the relevant passages | Relevant to claim No. |
|---|---|---|
| Y | CN 101161242 A (BEIJING HONGLIN PHARMACEUTICAL CO., LTD.) 16 April 2008 (2008-04-16) description, page 3, paragraph 4 to page 11, paragraph 7 | 13-15 |
| X | CN 110623933 A (DEZHOU DEYAO PHARMACEUTICAL CO., LTD.) 31 December 2019 (2019-12-31) claims 1-7 | 9-12 |
| Y | CN 110623933 A (DEZHOU DEYAO PHARMACEUTICAL CO., LTD.) 31 December 2019 (2019-12-31) claims 1-7 | 13-15 |
| X | CN 103271889 A (SHENYANG PHARMACEUTICAL UNIVERSITY) 04 September 2013 (2013-09-04) description, paragraphs 10-29 | 9-12 |
| Y | CN 103271889 A (SHENYANG PHARMACEUTICAL UNIVERSITY) 04 September 2013 (2013-09-04) description, paragraphs 10-29 | 13-15 |
| X | CN 107693502 A (TIANHENG ACADEMY OF PHARMACEUTICAL SCIENCES CO., LTD.) 16 February 2018 (2018-02-16) description, page 2, paragraph 2 to page 3, last paragraph | 9-12 |
| Y | CN 107693502 A (TIANHENG ACADEMY OF PHARMACEUTICAL SCIENCES CO., LTD.) 16 February 2018 (2018-02-16) description, page 2, paragraph 2 to page 3, last paragraph | 13-15 |
| A | CN 101049278 A (SHANGHAI RUBBER PRODUCTS RESEARCH INSTITUTE) 10 October 2007 (2007-10-10) entire document | 1-15 |

Form PCT/ISA/210 (second sheet) (July 2022)

**INTERNATIONAL SEARCH REPORT**
**Information on patent family members**

International application No.
**PCT/CN2024/118882**

| Patent document cited in search report | | | Publication date (day/month/year) | Patent family member(s) | | | Publication date (day/month/year) |
|---|---|---|---|---|---|---|---|
| WO | 2023174450 | A2 | 21 September 2023 | AU | 2023236675 | A1 | 31 October 2024 |
| | | | | WO | 2023174450 | A3 | 09 November 2023 |
| | | | | CN | 116808226 | A | 29 September 2023 |
| | | | | CN | 116808314 | A | 29 September 2023 |
| | | | | CN | 117017897 | A | 10 November 2023 |
| | | | | CN | 117138051 | A | 01 December 2023 |
| CN | 1727409 | A | 01 February 2006 | CN | 100494282 | C | 03 June 2009 |
| CN | 101161242 | A | 16 April 2008 | US | 2008089937 | A1 | 17 April 2008 |
| | | | | CN | 100563637 | C | 02 December 2009 |
| CN | 110623933 | A | 31 December 2019 | CN | 110623933 | B | 01 July 2022 |
| CN | 103271889 | A | 04 September 2013 | CN | 103271889 | B | 28 October 2015 |
| CN | 107693502 | A | 16 February 2018 | | None | | |
| CN | 101049278 | A | 10 October 2007 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023111805573
- CN 2023111805569